# EUROPEAN PATENT SPECIFICATION

(11) **EP 2 800 581 B1**
(45) Date of publication and mention of the grant of the patent: **22.05.2024**
(21) Application number: 13733621.0
(22) Date of filing: 03.01.2013
(51) Int. Cl.: A61K 38/46, A61P 1/18, A61P 25/00, A61K 31/122, A61K 38/48, A61K 38/54

(54) **METHODS OF TREATING BEHAVIORAL SYMPTOMS OF NEUROLOGICAL AND MENTAL DISORDERS**
VERFAHREN ZUR BEHANDLUNG VON VERHALTENSSYMPTOMEN FÜR NEUROLOGISCHE UND PSYCHISCHE ERKRANKUNGEN
PROCÉDÉS DE TRAITEMENT DES SYMPTÔMES COMPORTEMENTAUX DE TROUBLES NEUROLOGIQUES ET MENTAUX

(30) Priority: 03.01.2012 US 201261582813 P; 17.02.2012 US 201261600110 P
(43) Date of publication of application: 12.11.2014
(73) Proprietor: Curemark, LLC, Rye Brook, NY 10573 (US)
(72) Inventor: FALLON, Joan M., Rye Brook, NY 10573 (US); HEIL, Matthew F., Rye Brook, NY 10573 (US); SZIGETHY, James, Montgomery, NY 12549 (US); NANUS, Kenneth, Long Island City, NY 11101 (US); FALLON, James J., Rye Brook, NY 10573 (US)
(74) Representative: HGF
(86) International application number: PCT/US2013/020183
(87) International publication number: WO 2013/103746

(56) References cited:
- WO-A1-2010/002972
- WO-A1-2010/120781
- WO-A2-03/051345
- US-A1- 2002 001 575
- US-A1- 2008 112 944
- US-B1- 6 534 063
- JASON BRINKLEY ET AL: "Factor Analysis of the Aberrant Behavior Checklist in Individuals with Autism Spectrum Disorders", JOURNAL OF AUTISM AND DEVELOPMENTAL DISORDERS, KLUWER ACADEMIC PUBLISHERS-PLENUM PUBLISHERS, NE, vol. 37, no. 10, 21 December 2006 (2006-12-21), pages 1949-1959, XP019552461, ISSN: 1573-3432
- GLADYS BLOCK ET AL: "A rapid food screener to assess fat and fruit and vegetable intake", AMERICAN JOURNAL OF PREVENTIVE MEDICINE, vol. 18, no. 4, 1 May 2000 (2000-05-01), pages 284-288, XP055196074, ISSN: 0749-3797, DOI: 10.1016/S0749-3797(00)00119-7
- Marsha Mailick Seltzer ET AL: "0162-3257/03/1200-0565/0 2003 Plenum Publishing Corporation The Symptoms of Autism Spectrum Disorders in Adolescence and Adulthood", Journal of Autism and Developmental Disorders, 1 December 2003 (2003-12-01), XP055196077, Retrieved from the Internet: URL:http://www.waisman.wisc.edu/family/pub s/Autism/2003-The_Symptoms_of_Autism_Spect rum_Disorders.pdf [retrieved on 2015-06-16]
- HORVATH KAROLY ET AL: "Autistic disorder and gastrointestinal disease", CURRENT OPINION IN PEDIATRICS, CURRENT SCIENCE, PHILADELPHIA, PA, US, vol. 14, no. 5, 1 October 2002 (2002-10-01), pages 583-587, XP009099977, ISSN: 1040-8703, DOI: 10.1097/00008480-200210000-00004
- FERRONE, M ET AL.: 'Pancreatic Enzyme Pharmacotherapy.' PHARMACOTHERAPY. vol. 27, 2007, pages 910 - 920, XP055090867
- OLIVAR-PARRA, JS ET AL.: 'Training referential communicative skills to individuals with autism spectrum disorder: a pilot study.' PSYCHOLOGICAL REPORTS. vol. 109, 2011, pages 921 - 939, XP008174115
- TAMARO, C.: 'Vitamin K Deficiency as a Cause of Autistic Symptoms' VITAMIN K DEFICIENCY AS A CAUSE OF AUTISTIC SYMPTOMS 2006, XP055156033 Retrieved from the Internet: <URL:http://web.archive.org/web/20090612022 246/http://www.gutresearch.com/VitaminK.pd f> [retrieved on 2013-02-08]

## Description

### BACKGROUND OF THE INVENTION

Autism is a lifelong disorder of unknown origin. The disorder is characterized by behavioral, developmental, neuropathological, and sensory abnormalities (American Psychiatric Association 1994) and is usually diagnosed between the ages of 3 and 10 with peak prevalence rates observed in children aged 5-8.

Although there is debate as to whether autism has a pre- or post-natal origin, it is generally accepted that the symptoms and pathology persist throughout the life of the subj ect.

Nothing presented in the background section should be construed as an admission of prior art.

WO2010002972 discloses a therapeutic composition for the treatment of the symptoms of neurological and mental health disorders. The therapeutic agent is a stable pharmaceutical preparation containing, but not limited to, digestive/pancreatic enzymes.

WO2010120781 discloses coated digestive enzyme preparations and enzyme delivery systems and pharmaceutical compositions comprising the preparations to treat persons having ADD, ADHD, autism, cystic fibrosis and other behavioral and neurological disorders.

### SUMMARY OF THE INVENTION

According to a first aspect of the invention there is provided, a pharmaceutical composition comprising digestive enzymes and one or more excipients, for use in the treatment of vitamin K deficiency or bone fragility in a subject exhibiting Autism Spectrum Disorder (ASD), wherein the digestive enzymes comprise a protease, an amylase, and a lipase.

In certain embodiments, the subject having an ASD has malnutrition as a result of dietary restriction self-imposed by the subject.

In certain embodiments, the composition is administered to the subject two or more times a day with food for at least 12 weeks.

In certain embodiments, the subject has an abnormal fecal chymotrypsin level before administration of the pharmaceutical composition.

In certain embodiments, the subject has a fecal chymotrypsin level of less than 13 U/g or less than 12.6 U/g as measured in a frozen stool sample before administration of the pharmaceutical composition; or wherein the subject has a fecal chymotrypsin level of less than 10 U/g or less than 9 U/g as measured in a fresh stool sample before administration of the pharmaceutical composition.

In certain embodiments, the subject has increased fecal chymotrypsin levels after administration of the pharmaceutical composition.

In certain embodiments, the subject exhibits a decrease in the number or severity of infections or a decrease in the number or severity of allergic incidents after administration of the pharmaceutical composition in comparison to before the subject was administered the pharmaceutical composition.

In certain embodiments, the ASD is autism.

In certain embodiments, the subject has increases in calcium after administration or after treatment with the pharmaceutical composition.

Disclosed herein but not claimed is a method for treating a subject with an Autistic Spectrum Disorder (ASD), which includes Pervasive Developmental Disorders (PDDs), such as autism or an autistic disorder, in need thereof with a pharmaceutical composition comprising a therapeutic composition comprising proteases, amylases and/or lipases and an excipient: administering said pharmaceutical composition comprising one or more pancreatic digestive enzymes to said subject, wherein said subject has an abnormal fecal chymotrypsin level or stool pH, and wherein said subject shows a reduction in the severity or frequency of one or more symptoms associated with an Autistic Spectrum Disorder after administration of said pharmaceutical composition.

Disclosed herein but not claimed is a method for treating a subject with an Attention Deficit Disorder (ADD) and/or Attention Deficit Hyperactivity Disorder (ADHD) in need thereof with a pharmaceutical composition comprising a therapeutic composition comprising proteases, amylases and/or lipases and an excipient: administering said pharmaceutical composition comprising one or more pancreatic digestive enzymes to said subject, wherein said subject has an abnormal fecal chymotrypsin level or stool pH, and wherein said subject shows a reduction in the severity or frequency of one or more symptoms associated with an Autistic Spectrum Disorder after administration of said pharmaceutical composition.

In another embodiment, one or more symptoms associated with an Autistic Spectrum Disorder and/or ADD or ADHD comprise irritability, agitation, social withdrawal, lethargy, inattention, hyperactivity, non-compliance, stereotypy or inappropriate speech. In another embodiment, the one or more symptoms comprise lethargy and hyperactivity, and/or the reciprocity of symptoms associated with each. In another embodiment, the symptoms are measured on the Aberrant Behavior Checklist (ABC) scale. In another embodiment, other measures are used to assess changes in autistic symptomotology as seen utilizing the behavioral and cognitive measures such as the Conners test, Expressive Vocabulary Test, second edition (EVT-2), Peabody Picture Vocabulary Test, fourth edition (PPVT-4), Social Communication Questionnaire (SCQ), The Autism Diagnostic Interview-Revised (ADI-R test), the American Psychiatric Association's Diagnostic and Statistical Manual-IV (DSM-IV) test, any other test described herein or conventionally recognized in the art. In another embodiment, a pharmaceutical composition described herein does not produce a sedating effect, an increase in neurological symptoms such as dizziness, dystonia, akathisia, somnolence, fatigue, extrapyramidal disorders, tremor, drooling, or other symptoms seen in medications treating behavioral disorders such as weight gain, *etc.* In another embodiment a pharmaceutical composition described herein does not produce a sedating any side effects in accordance with FDA reporting standards (such as at a rate greater than about 5%).

In another embodiment, the subject is administered a pharmaceutical composition described herein for at least 12 weeks. In one non-limiting example, the subject is administered a pharmaceutical composition described herein three times a day for at least 12 weeks. In another embodiment, the subject showed said reduction in the severity or frequency of one or more symptoms after administration of a pharmaceutical composition described herein for at least 12 weeks.

A subject to be treated with the methods described herein may be between the ages of 1-18 yrs of age. In another embodiment, the subject is between the ages of 2-16 yrs of age. In another embodiment, the subject is between the ages of 1-10 yrs of age. In another embodiment, the subject is between the ages of 3-8 yrs of age. In another embodiment the subject is from any geographical location on the planet earth.

In another embodiment, the subject has mild and or moderate levels of lethargy, hyperactivity, social withdrawal or irritability prior to administration. In another embodiment the subject has high levels of lethargy, hyperactivity, social withdrawal or irritability prior to administration.

In another embodiment, the subject showed an improvement in receptive and/or expressive language after administration of a pharmaceutical composition described herein. In another embodiment, the improvement is measured by the Expressive Vocabulary Test (EVT) and/or the Peabody Picture Vocabulary Test. In another embodiment, the subject has autism and aphasia or another lack of expressive or receptive language.

In another embodiment, the subject manifests one or more speech improvements, age appropriate grammatical structure and/or vocabulary after administration of a pharmaceutical composition described herein. In another embodiment, the subject showed said manifestation without a learning curve. In another embodiment, the subject showed improvement in overall growth scales, increased question ceiling levels or reduction in error rates. In another embodiment, the subject demonstrated improved working memory and/or fluid reasoning after administration of a pharmaceutical composition described herein. In another embodiment, the subject had reduced hyperactivity after administration of a pharmaceutical composition described herein. In another embodiment, the changes in hyperactivity were observed in the Conners-3TR test. In another embodiment, the one or more symptoms comprises inattention, learning problems, executive functioning, aggression, hyperactivity impulsivity, conduct disorder, or oppositional defiance. In another embodiment, the subject had improved peer relations. In another embodiment the symptoms were measured on the Conners DSM-IV Scale.

In another embodiment, the subject has increases in balanced food consumption, protein intake, vegetable intake, meat intake, cholesterol, vitamin K, calcium, or improvements in glycemic load after administration of after treatment with a pharmaceutical composition described herein compared to subject of the same age without an ASD or ADHD compared to a subject treated with a placebo. In another embodiment, the subject has lower overall caloric intake compared to subject of the same age without an ASD or ADHD or compared to a subject treated with a placebo. In another embodiment, the subject has the same overall caloric intake after treatment with a pharmaceutical composition described herein, but increased protein and fat intake compared to a subject treated with a placebo. In another embodiment, the subject of the same age without an ASD, ADD or ADHD, or treated with a placebo, had a sedentary life style or an active life style. In another embodiment, a subject has fewer fractures after administration of a pharmaceutical composition described herein compared to subject of the same age without an ASD or ADHD compared to a subject treated with a placebo.

In another embodiment, a subject exhibits improved overall health after administration of a pharmaceutical composition described herein compared to subject of the same age without an ASD or ADHD compared to a subject treated with a placebo. In another embodiment, a subject exhibits improved gastrointestinal health (e.g., improvement in constipation and/or diarrhea) after administration of a pharmaceutical composition described herein compared to subject of the same age without an ASD or ADHD compared to a subject treated with a placebo. In another embodiment, a subject exhibits a reduction in the number and/or severity of seizures (e.g., "Grand Mal", absence, myoclonic, tonic, clonic or atonic) after administration of a pharmaceutical composition described herein compared to subject of the same age without an ASD or ADHD compared to a subject treated with a placebo.

In another embodiment, an abnormal fecal chymotrypsin level indicates that said subject has physiological malnutrition. In another embodiment, the subject having a behavioral, neurological or mental disorder consumes fewer calories fat, protein, or carbohydrates than a subject with a normal fecal chymotrypsin level. In another embodiment, the administration of a pharmaceutical composition described herein reverses the loss of protein and fat caloric intake; in some cases, such intake occurs where the total caloric intake does not differ from a subject treated with a placebo. In another embodiment, the subject is male. In another embodiment the subject is female. In another embodiment, the stool pH of a subject treated with a pharmaceutical composition described becomes more alkaline.

The subject may have a reduction in intake of whole grains after administration of a pharmaceutical composition described herein. The subject may also have a reduction in overall carbohydrate intake after administration of a pharmaceutical composition described herein. In another embodiment, the reduction is at least 5%. In another embodiment, the reduction is at least 10%. In another embodiment, the reduction is at least 15%. In another embodiment, the reduction is at least 20%. In another embodiment, the reduction is at least 25%. In another embodiment, the reduction is at least 30%. In another embodiment, the reduction is at least 35%. In another embodiment, the reduction is at least 40%. In another embodiment, the reduction is at least 50%. In another embodiment, the reduction is at least 60%. In another embodiment, the reduction is at least 70%. In another embodiment the reduction is at least 80%. In another embodiment, the reduction is at least 90%. In another embodiment, the reduction is at least 95%.

In another embodiment, the administration of a pharmaceutical composition described herein is over a 12 week period. In another embodiment, the administration of a pharmaceutical composition described herein is over a 16 week period. In another embodiment, the administration of a pharmaceutical composition described herein is over a 20 week period. In another embodiment, the administration of a pharmaceutical composition described herein is over a 6 month period. In another embodiment, the said administration of a pharmaceutical composition described herein is over a 1 year period. In another embodiment, the said administration of a pharmaceutical composition described herein occurs on a regular, re-occurring basis. A pharmaceutical composition described herein can be administered one, two or three times a day for the treatment period. A pharmaceutical composition described herein can be administered with meals.

In another embodiment, the subject increases overall protein intake after administration of a pharmaceutical composition described herein. In another embodiment, the increase is at least 5%. In another embodiment, the increase is at least 10%. In another embodiment, the increase is at least 15%. In another embodiment, the increase is at least 20%. In another embodiment, the increase is at least 25%. In another embodiment, the increase is at least 30%. In another embodiment, the increase is at least 35%. In another embodiment, the increase is at least 40%. In another embodiment, the increase is at least 50%. In another embodiment, the increase is at least 60%. In another embodiment, the increase is at least 70%. In another embodiment, the increase is at least 80%. In another embodiment, the increase is at least 90%. In another embodiment, the increase is at least 100%. In another embodiment, the increase is at least 150%. In another embodiment, the increase is at least 200%.

In another embodiment, the subject increases overall fat intake after administration of a pharmaceutical composition described herein. In another embodiment, the increase is at least 5%. In another embodiment, the increase is at least 10%. In another embodiment the increase is at least 15%. In another embodiment, the increase is at least 20%. In another embodiment, the increase is at least 25%. In another embodiment, the increase is at least 30%. In another embodiment, the increase is at least 35%. In another embodiment, the increase is at least 40%. In another embodiment, the increase is at least 50%. In another embodiment, the increase is at least 60%. In another embodiment, the increase is at least 70%. In another embodiment, the increase is at least 80%. In another embodiment, the increase is at least 90%. In another embodiment, the increase is at least 100%. In another embodiment, the increase is at least 150%. In another embodiment, the increase is at least 200%. In another embodiment, the subject had a history of one or more allergies.

In another embodiment, the subject with said history of one or more allergies had a larger intake of fiber, calories, fat, protein, and carbohydrates after administration of a pharmaceutical composition described herein than a subject that did not have allergies. In another embodiment, the subject had an increase in B6, B12, A, C, E, K, Copper, Iron, Cholesterol, Niacin, Riboflavin, Thiamin, or Zinc consumption after administration of a pharmaceutical composition described herein. In another embodiment, the subject had an increase in B6, B 12, A, C, E, K, Copper, Iron, Cholesterol, Niacin, Riboflavin, Thiamin, or Zinc blood levels after administration of a pharmaceutical composition described herein. In another embodiment, the subject further had a vitamin K deficiency. In another embodiment, the vitamin K deficiency is measured by detecting levels of gamma carboxylated proteins in the blood.

Disclosed herein but not claimed is a method for treating a subject with an ASD, ADD, or ADHD in need thereof with a pharmaceutical composition described herein comprising: administering said pharmaceutical composition comprising one or more pancreatic digestive enzymes to said subject, wherein said subject has a vitamin K deficiency, and wherein said subject shows a reduction in the severity or frequency of one or more symptoms associated with an ASD, ADD, or ADHD after administration of said pharmaceutical composition. In one example the ASD is autism. In another example the vitamin K deficiency is measured by detecting levels of gamma carboxylated proteins in the blood.

Disclosed herein but not claimed is a method of treating a subject with an ASD, ADD, or ADHD in need thereof with a pharmaceutical composition described herein comprising: administering said pharmaceutical composition comprising: 1) one or more pancreatic digestive enzymes; 2) glutamate enhancing therapy; wherein said subject has a vitamin K deficiency or an abnormal fecal chymotrypsin level, and wherein said subject shows a reduction in the severity or frequency of one or more symptoms associated with an ASD, ADD, or ADHD after administration of said pharmaceutical composition. In one embodiment the ASD is autism. In another example the vitamin K deficiency is measured by detecting levels of gamma carboxylated proteins in the blood. In another example a pharmaceutical composition described herein reduces the frequency or severity of diarrhea, gas, bloating, cramping, flatulence, nausea or abdominal pain in said subject. In another example the subject has stunting, protein energy malnutrition, wasting, vitamin or mineral deficiency, abnormal albumin levels, abnormal prealbumin levels, abnormal cholesterol levels, abnormal elastase levels, or abnormal trypsin levels.

Disclosed herein but not claimed is a method of treating a subject with an ASD, ADD or ADHD in need thereof with a pharmaceutical composition described herein comprising: administering said pharmaceutical composition comprising one or more digestive enzymes to said subject, wherein said subject has an abnormal fecal enzyme level, and wherein said subject shows a reduction in the severity or frequency of one or more symptoms associated with an ASD, ADD, or ADHD after administration of said pharmaceutical composition. The ASD may be autism.

One or more symptoms that may be improve include, but are not limited to, irritability, agitation, social withdrawal, lethargy, hyperactivity, stereotypy and/or inappropriate speech. In one example, the one or more symptoms include lethargy and hyperactivity.

Symptoms may be measured, for example, using the Aberrant Behavior Checklist (ABC) scale, any other test described herein and/or any other art-recognized test known in the art.

In such methods, a pharmaceutical composition described herein does not produce a sedating effect, an increase in neurological symptoms such as dizziness, Parkinson's disease, dystonia, akathisia, somnolence, fatigue, extrapyramidal disorders, tremor, drooling, weight gain, or a combination thereof. In one embodiment, a pharmaceutical composition described herein does not produce a sedating any side effects in accordance with FDA reporting standards (such as at a rate greater than 5%).

In another example, a subject is administered a pharmaceutical composition described herein for at least 4 weeks. In one non-limiting example, a subject shows a reduction in the severity or frequency of one or more symptoms after administration of said pharmaceutical composition for at least 4 weeks.

Subjects to be treated with such methods include children. In one embodiment, the child is male. In another embodiment, the child is female.

A subject to be treated with such methods may be from any geographical location on the planet earth. In one embodiment, the subject to be treated is of Asian descent, of North American descent, of South American descent, of Eurasian descent, of Australian descent, of European descent, of African descent, or a combination thereof.

In one embodiment, a subject to be treated exhibits mild and or moderate levels of lethargy, hyperactivity, hypersensitivity, social withdrawal and/or irritability prior to administration of a pharmaceutical composition described herein.

In another embodiment, a subject to be treated exhibits subject has high levels of lethargy, hyperactivity, hypersensitivity, social withdrawal or irritability prior to administration of a pharmaceutical composition described herein.

In one embodiment, the subject shows an improvement in receptive and/or expressive language after administration of a pharmaceutical composition described herein. Such improvements may be measured, for example, by the Expressive Vocabulary Test (EVT) and/or the Peabody Picture Vocabulary Test. Subjects may be assessed prior to, during, and after treatment with any of the tests described herein.

In one embodiment, a subject to be treated with such methods has autism and aphasia or another lack of expressive language.

A subject treated with a pharmaceutical composition described herein may manifest one or more speech improvements, age appropriate grammatical structure and/or vocabulary after administration of the pharmaceutical composition. In one embodiment, the subject may show the manifestation without a learning curve. In another embodiment, the subject show improvement in overall growth scales, increased question ceiling levels or reduction in error rates. In another embodiment, a subject demonstrates improved working memory and/or fluid reasoning after administration of a pharmaceutical composition described herein. Such improvements may be measured using a Stanford Binet Test. In another embodiment, a subject has reduced hyperactivity after administration of a pharmaceutical composition described herein. Such changes in hyperactivity may be observed in the Conners-3TR test.

Symptoms to be treated by such methods include, but are not limited to, inattention, learning problems, executive functioning, aggression, hyperactivity impulsivity, conduct disorder, oppositional defiance, or a combination thereof. In one embodiment, a subject may have improved peer relations. Such symptoms may be measured, for example, on the Conners DSM-IV Scale.

A subject may exhibit increases in balanced food consumption, protein intake, vegetable intake, meat intake, cholesterol, vitamin K, calcium, and/or improvements in glycemic load after administration of a pharmaceutical composition described herein. In one embodiment, a subject has a lower overall caloric intake compared to a subject of the same age without an ASD or ADHD; where, in some cases, a subject of the same age without an ASD, ADD, or ADHD had a sedentary life style or an active life style. In another embodiment, a subject has fewer fractures after administration of a pharmaceutical composition described herein.

An abnormal fecal enzyme level may indicate that the subject has physiological malnutrition. In some cases, the abnormal fecal enzyme level is an abnormal fecal chymotrypsin level (FCT). Fecal enzyme levels may be monitored over time.

The amount of digestive enzymes administered to a subject may be based on one or more criteria including, but not limited to: said subject's weight, said subject's baseline fecal chymotrypsin level, said subject's instantaneous fecal chymotrypsin level, said subject's time averaged fecal chymotrypsin level, change in said subject's chymotrypsin level, change in chymotrypsin level per unit time, rate of change of fecal chymotrypsin level per unit time (2nd derivative), cumulative dose of digestive enzymes to said subject to date, time averaged dosing over a given time period, rate of change of dosing against rate of change in fecal chymotrypsin level, or derivative of rate of change of dosing against rate of change in fecal chymotrypsin level.

In one embodiment, the fecal chymotrypsin level is used to titrate the amount of digestive enzymes administered to the subject.

A change in fecal chymotrypsin levels after administration of a pharmaceutical composition described herein, comprising one or more digestive enzymes may be used to determine when administration of the pharmaceutical composition comprising one or more digestive enzymes may be reduced, increased, or terminated. In one embodiment, a subject to be treated has a low pre-treatment fecal chymotrypsin level. In one embodiment, a low fecal chymotrypsin level correlates to an increased severity in at least one symptom of autism. In another embodiment, decreasing levels of fecal chymotrypsin directly correlate with increasing severity of at least one symptom of autism. In yet another embodiment, improvements in the chymotrypsin level from baseline directly correlate with improvement of at least one symptom in the subject.

A subject to be treated with such methods may be diagnosed with autism neurological or mental disorder described herein. In one embodiment, the subject has a greater improvement in at least one symptom than a similar subject with a higher pretreatment fecal chymotrypsin level. In another embodiment, a subject has a greater improvement in post-treatment fecal chymotrypsin levels than a similar subject with a higher pretreatment fecal chymotrypsin level. The level of fecal chymotrypsin may be measured and used to titrate the amount of digestive enzymes administered to the subject.

The pH of the subject's stool may return to normal, or closer to normal, following treatment with a pharmaceutical composition described herein. The pH of the subject's stool and the fecal chymotrypsin level of the subject may return to normal, or closer to normal, following treatment with a composition described herein.

A subject may exhibit an improvement in overall health following treatment with a pharmaceutical composition described herein. In one embodiment the improvement is a decrease in the rate or severity of infection. In another embodiment the improvement is a decrease in the number or severity of allergic incidents, which can include respiratory, dermatological, and/or gastrointestinal allergies.

A therapeutic composition disclosed herein may comprises an amylase, a protease, or a lipase. A therapeutic composition disclosed herein may comprise two of: an amylase, a protease, and a lipase. A therapeutic composition disclosed herein comprises an amylase, a protease, and a lipase.

The subject may be determined or diagnosed as having at least one symptom of an ASD by using a screen comprising a DSM-IV, SCQ or ADI-R test. In another embodiment, a subject is determined to have an ASD by using a screen comprising a DSM-IV, SCQ, or ADI-R test. In one embodiment the subject is treated with a pharmaceutical composition described herein. In one embodiment the ASD is autism. In one embodiment the subject is a child. In one embodiment the child is 1, 2, 3, 4, 5, 6, 7, 8, 9,10, 11, 12, 13, 14, 15, 16, 17, or 18 years old.

In another embodiment, a subject is screened utilizing the ADI-R and determined to be able to be randomized for a clinical trial utilizing at least one or more of the following tests: ADI-R, DSM-IV and SCQ.

In another embodiment, a subject with at least one symptom of an ASD and a level of chymotrypsin in a frozen stool sample <12.6 U/g is eligible for treatment with a pharmaceutical composition disclosed herein.

In another embodiment, a subject with at least one symptom of an ASD and a level of chymotrypsin in a fresh stool sample < 9 U/g is eligible for treatment with a pharmaceutical composition disclosed herein.

In another embodiment, a clinical trial with those with autism has a subset with those with abnormal chymotrypsin levels.

In another embodiment, a subject administered a pharmaceutical composition disclosed herein demonstrated higher fecal chymotrypsin levels over the course of 12 weeks of administration.

In yet another embodiment, a pharmaceutical composition delivered chymotrypsin to the subject who was administered the pharmaceutical composition.

In another embodiment, there was a statistically significant difference between subjects who were administered a pharmaceutical composition of (Formulation 1) and subjects who were administered a placebo.

In one embodiment, a pharmaceutical composition comprises a therapeutic composition that comprises proteases, amylases and lipases, and/or an excipient. In one embodiment, the therapeutic composition comprises proteases, amylases and lipases. In one embodiment, the therapeutic composition is pancreatin. In one embodiment, the therapeutic composition is coated with an excipient. In one embodiment, the coating is a lipid or polymer coating. In one embodiment, the coating is a soy lipid coating, such as a soybean oil coating. The coating may optionally comprise an emulsifier. In one embodiment, the pharmaceutical composition is provided as a tablet, capsule or granules. In one embodiment, the pharmaceutical composition has a protease activity of not less than 156 U.S.P. units/mg. In one embodiment, the pharmaceutical composition is provided as granules. In one embodiment, about 1.1 to 0.8 mg of the pharmaceutical composition is provided per dose. In another embodiment, the dose is provided in a pouch/sachet. In one embodiment, the pharmaceutical composition is Formulation 1.

In another embodiment, a stool pH measurement is taken after treatment with a pharmaceutical composition disclosed herein of a subject with a symptom of an ASD, such as autism. In one embodiment, the subject was diagnosed with autism before administration of the pharmaceutical composition disclosed herein. In one embodiment, the stool pH becomes more alkaline after treatment of the subject with a pharmaceutical composition disclosed herein.

In another embodiment, the stool pH is measured to determine the gastrointestinal health of a subject with a symptom of an ASD, such as autism. In another embodiment, the stool pH is measured to determine the ability of a subject wit with a symptom of an ASD, such as autism to digest protein.

In another embodiment, a subject administered a pharmaceutical composition described herein exhibited a significantly greater change in stool pH than a subject administered a placebo. In another embodiment, a subject administered a pharmaceutical composition described herein exhibited a positive change in stool pH demonstrating greater alkalinization of their stool, while a subject administered a placebo had an acidic change of their stool. In another embodiment, a subject administered a pharmaceutical composition described herein exhibited an alkalinization of their stool commencing at approximately week 4 after administration of the pharmaceutical composition began.

In one embodiment, an ABC test is used to measure changes in one or more symptoms of an ASD in a subject administered a pharmaceutical composition described herein versus a subject administered a placebo. In another embodiment, a total ABC test is used to measure the outcomes of treatment of a subject with a pharmaceutical composition described herein. In one embodiment the subject is a child. In one embodiment a total ABC test produces a total ABC scale. In another embodiment, the total ABC test can be used when a compound is non-sedating. In another embodiment, the total ABC scale can be used when the hyperactivity non-compliance scale and the lethargy social withdrawal scale are not reciprocal. In another embodiment, the total ABC scale or individual ABC subscales can be used to determine the outcomes of experimental observations in children with autism. In another embodiment, the ABC scale can be used when coupled with the ADI-R to determine the level of autism and the ability to change based on the level of autism. In one embodiment, the one or more symptoms comprise irritability, agitation, social withdrawal, lethargy, hyperactivity, non-compliance, stereotypy or inappropriate speech. In another embodiment, the one or more symptoms comprise lethargy and hyperactivity or the reciprocity of symptoms associated with each.

In another embodiment, the total ABC scale is used to measure change in autistic symptomotology. In another embodiment, the symptoms are measured on the Aberrant Behavior Checklist (ABC) scale. In another embodiment, other measures are used to assess changes in autistic symptomotology as seen using the behavioral and cognitive measures such as Conners test, EVT-2 test, PPVT-4 test, Social Communication Questionnaire, ADI-R test, the DSM-IV test, any other test described herein or conventionally recognized in the art.

In another embodiment, a pharmaceutical composition described herein does not produce a sedating effect, an increase in neurological symptoms such as dizziness, Parkinson's disease, dystonia, akathisia, somnolence, fatigue, extrapyramidal disorders, tremor, drooling, or other symptoms seen in medications treating behavioral disorders.

In another embodiment, a pharmaceutical composition described herein does not produce any sedating any side effects in accordance with FDA reporting standards.

In another embodiment, the subject is administered a pharmaceutical composition described herein for at least 12 weeks. In another embodiment, the subject showed said reduction in the severity or frequency of one or more symptoms after administration of said pharmaceutical composition for at least 12 weeks. In another embodiment, the subject is administered said pharmaceutical composition for 24, 48, or additional weeks.

In another embodiment, the placebo adjusted 12 week scores demonstrated a significant difference between a subject administered a pharmaceutical composition described herein and a subject administered a placebo as demonstrated by a greater percentage (%) change from baseline (i.e., before a pharmaceutical composition described herein was administrated). In one embodiment the subject is a child.

In another embodiment, that change as demonstrated by a continued increase in % change from baseline at 24 and 48 weeks shows a continued improvement over time in a subject administered a pharmaceutical composition described herein. In one embodiment the subject is a child.

In yet another embodiment, the changes observed in a subject administered a pharmaceutical composition described herein, as measured by the ABC are in both positive and negative symptoms of the autism as measured by the ABC: positive symptoms (additive symptoms) hyperactivity, irritability, agitation, stereopathy, and/or inappropriate speech. And the negative symptoms (symptoms that take away behavior): social withdrawal, and lethargy.

In one embodiment, a subject treated with a pharmaceutical composition described herein is between 1 and 18 years of age. In another embodiment, the subject is between the age of 2 and 16 years of age. In another embodiment, the subjects are between 3 and 8 years of age, in yet another embodiment the subjects are between 9 and 12 years of age. In another embodiment, the subjects are between the ages of 1 and 10 years of age. In one embodiment, the subject is a child. In another embodiment, the subject is from any geographical location on the planet earth.

In another embodiment, a subject has mild and or moderate levels of lethargy, hyperactivity, social withdrawal and/or irritability prior to administration. In another embodiment, a subject has high levels of lethargy, hyperactivity, social withdrawal and/or irritability prior to administration.

In another embodiment, a subject has mild and or moderate levels of hyperactivity and/or non-compliance prior to administration. In another embodiment, a subject has high levels of hyperactivity and/or non-compliance prior to administration.

In another embodiment, a subject has mild and or moderate levels of inappropriate speech prior to administration. In another embodiment, a subject has high levels of inappropriate speech prior to administration.

In another embodiment, the subject has mild and or moderate levels of stereopathy prior to administration. In another embodiment, the subject has high levels of stereopathy prior to administration.

In one embodiment, a PPVT and an EVT (A version or B version) are used to measure vocabulary a method of treatment of a subject (e.g., a child) with a pharmaceutical composition described herein. In one embodiment, the subject has autism. In another embodiment, both a PPVT and an EVT are used to reduce pre-test post-test bias out of the trial.

In another embodiment, the use of the PPVT coupled with the EVT is used to measure change in a subject for expressive and receptive language. In another embodiment, the change is measured before the subject is administered a pharmaceutical composition described herein. In another embodiment, the change is measured after the subject is administered a pharmaceutical composition described herein.

In another embodiment, the number of errors seen in the PPVT test in a subject is administered a pharmaceutical composition described herein is less than a subject administered a placebo.

In another embodiment, the growth scores seen in the PPVT test in a subject administered a pharmaceutical composition described herein is greater than a subject administered a placebo.

In another embodiment, the ceiling to error scores seen in the PPVT test (ceiling minus (-) errors) is greater for a subject administered a pharmaceutical composition described herein.

In another embodiment, there is a greater change for a subject administered a pharmaceutical composition described herein in the EVT growth score versus the PPVT score, demonstrating larger gains in expressive language.

In another embodiment, the number of errors seen in the EVT test in a subject administered a pharmaceutical composition described herein is less than a subject administered a placebo.

In another embodiment, the growth scores seen in the EVT tests in a subject administered a pharmaceutical composition described herein is greater than a subject administered a placebo.

In another embodiment, the ceiling to error scores seen in the EVT test (ceiling minus (-) errors) is greater in a subject administered Formulation 1, demonstrating improved performance.

In one embodiment, the block food screener is used to examine food intake by nutrient of a subject administered a pharmaceutical composition described herein or of a subject administered a placebo. In another embodiment, the block food screener measures the qualitative and quantitative measure of food intake on a daily basis. In another embodiment, the block food screener measures of the qualitative and quantitative food intake can be compared to the changes in behavior, cognitive, or other physiological measures in subjects with an ASD, such as autism, or other neurological disorders. In one embodiment, the changes as measured on the block food screener can be used to determine the nutritional status of a subject before, after or during administration of a pharmaceutical composition described herein. In yet another embodiment, as measured on the block food screener, as the final amount of calories consumed per day at week 12 increased there are a greater improvement in scores on the total ABC. In yet another embodiment, as the total amount of calories consumed per day by a subject reached 800+ calories, as measured on the block food screener, the total ABC scores for a subject administered a placebo worsened while the total ABC score for a subject administered a pharmaceutical composition described herein, improved significantly.

In another embodiment, there is improvement on the total ABC scores as seen in a subject administered a pharmaceutical composition described herein versus a subject administered a placebo at all levels of protein intake.

In another embodiment, at approximately 2+ grams of increase in protein intake per day as measured on the block food screener, a subject administered a pharmaceutical composition described herein has a greater improvement in at least one symptom of ASD, while a subject administered a placebo has a decline in at least one symptom of ASD.

In another embodiment, the worsening of symptoms on the total ABC in a subject administered a placebo is indicative of a lack of enzyme for the digestion of protein.

In yet another embodiment, there is improvement seen in subjects in their total ABC scores who are administered a pharmaceutical composition described herein versus subjects administered a placebo, at all levels of protein intake, as measured at 12 weeks after the pharmaceutical composition or placebo is first administered.

In yet another embodiment, at approximately 35+ grams of total protein intake per day as measured on the block food screener at week 12 , subjects administered a pharmaceutical composition described herein began to have increased improvement, and subjects administered a placebo began to worsen in at least one symptom of ASD.

In yet another embodiment, the worsening of symptoms on the total ABC administered a placebo is indicative of a lack of an enzyme for digestion of protein.

In one embodiment, there is improvement on total ABC scores in a subject administered a pharmaceutical composition described herein versus a subject administered a placebo at all levels of carbohydrate intake change as measured by the block food screener.

In yet another embodiment, at a level of 5+ grams change in carbohydrate intake as measured by the block food screener a subject administered a placebo began to worsen and a subject administered a pharmaceutical composition began to improve at a greater rate. In another embodiment, the worsening of symptoms as measured on the total ABC in a subject administered a placebo is due to the lack of enzyme to digest the protein portion of carbohydrates (such as gliadin protein).

Disclosed herein but not claimed is a method of treating a subject with one or more symptoms of an ASD, comprising: administering to the subject a pharmaceutical composition comprising one or more excipients and a therapeutic composition, wherein the therapeutic composition comprises protease, amylase and/or lipase, wherein the subject exhibits improvement in one or more symptoms of an ASD comprising: (a) protein intake, fat intake, carbohydrate intake, vitamin intake, diarrhea, constipation, seizures, and/or bone fragility; and/or (b) hyperactivity, irritability, agitation, obsessive compulsive behavior, eye contact, speech, lethargy, hypersensitivity, stereotypy, toilet training, non-compliance, inattention, and/or social withdrawal and wherein the subject has a greater improvement in the one or more symptoms of an ASD after administration of the pharmaceutical composition than a subject a subject with one or more symptoms of an ASD administered a placebo.

Improvement in one or more symptoms of an ASD may be at least 1-fold greater improvement than in a subject with one or more symptoms of an ASD subject administered a placebo.

Improvement in one or more symptoms of an ASD may also be at least 2, 3, 4, or 5-fold greater than in a subject with one or more symptoms of an ASD administered a placebo.

A subject may exhibit about a 10% or greater improvement in one or more symptoms of an ASD after administration of the pharmaceutical composition in comparison to before the subject was administered the pharmaceutical composition.

The subject may exhibit greater than about a 20%, 30%, 40%, 50%, 60%, 70%, 80%, 90% or 100% improvement in the one or more symptoms of an ASD after administration of the pharmaceutical composition.

Total daily protein, fat, carbohydrate and/or vitamin intake (by weight) by a subject may increase following administration of the pharmaceutical composition in comparison to protein, fat, carbohydrate and/or vitamin intake by the subject before administration of the pharmaceutical composition.

Total daily carbohydrate intake (by weight) by a subject may decrease following administration of the pharmaceutical composition in comparison to carbohydrate intake by the subject before administration of the pharmaceutical composition.

The subject may exhibit a greater increase in daily protein, fat, carbohydrate and/or vitamin intake (by weight) following administration of the pharmaceutical composition in comparison to a subject with one or more symptoms of an ASD following administration of a placebo.

The greater the amount of daily protein, fat, carbohydrate and/or vitamin intake (by weight) consumed by a subject after administration of the pharmaceutical composition, the greater the subject's improvement in one or more symptoms of an ASD. In one embodiment, the daily protein, fat, carbohydrate and/or vitamin intake (by weight) consumed by a subject may be measured before the subject was administered the pharmaceutical composition and at about 2, 4, 6, 8, 10, 12, 14, 16, 18, 20, 24, 36, 48, or 52 weeks after administration of the pharmaceutical composition begins.

A subject may exhibit a greater increase in daily vitamin intake (by weight) following administration of the pharmaceutical composition in comparison to a subject with one or more symptoms of an ASD following administration of a placebo.

A subject may exhibit a greater increase in daily carbohydrate intake (by weight) following administration of the pharmaceutical composition in comparison to a subject with one or more symptoms of an ASD following administration of a placebo.

The total daily calories consumed by a subject may increase after administration of the pharmaceutical composition in comparison to total daily calories consumed by a subject before administration of the pharmaceutical composition. The more daily calories the subject consumed after administration of the pharmaceutical composition, the greater a subject's improvement in one or more symptoms of an ASD.

The total amount of daily calories consumed by a subject may be measured before the subject is administered the pharmaceutical composition and at about 2, 4, 6, 8, 10, 12, 14, 16, 18, 20, 24, 36, 48, or 52 weeks after administration of the pharmaceutical composition begins.

In one embodiment, a subject may consume at least 800 kcalories per day after about 12 weeks of administration of the pharmaceutical composition.

A subject may further exhibit an improvement of a symptom comprising diarrhea, constipation, seizures, bone fragility, hyperactivity, irritability, agitation, obsessive compulsive behavior, eye contact, speech, lethargy, hypersensitivity, stereotypy, toilet training, non-compliance, aggression, impulsivity, conduct disorder, or oppositional defiance and/or social withdrawal.

A subject's improvement in one or more symptoms of an ASD may be measured on an Aberrant Behavior Checklist (ABC) scale.

A subject's improvement in one or more symptoms of an ASD may be measured as a total Aberrant Behavior Checklist (ABC) score.

The daily protein intake of a subject may be higher after administration of the pharmaceutical composition begins compared to protein intake by the subject before administration of the pharmaceutical composition.

The daily protein intake of a subject may be higher after administration of the pharmaceutical composition begins compared to protein intake by the subject administered the placebo.

The daily protein intake of a subject may be higher by 12 weeks after administration of the pharmaceutical composition begins compared to protein intake by the subject before administration of the pharmaceutical composition.

The daily protein intake of a subject may be higher by 12 weeks after administration of the pharmaceutical composition begins compared to protein intake by a subject before administration of the pharmaceutical composition.

The daily protein intake of a subject may be about 2 grams higher by 12 weeks after administration of the pharmaceutical composition begins compared to daily protein intake by the subject before administration of the pharmaceutical composition.

The daily protein intake of a subject may be about 2 grams higher by 12 weeks after administration of the pharmaceutical composition begins compared to daily protein intake by the subject before administration of the pharmaceutical composition.

A subject may consume from about 30 to about 50 grams of protein per day by 12 weeks after administration of the pharmaceutical composition begins.

A subject may consume about 35 grams or more of protein per day by 12 weeks after administration of the pharmaceutical composition begins.

A subject may further exhibit an improvement of a symptom comprising diarrhea, constipation, seizures, bone fragility, hyperactivity, irritability, agitation, obsessive compulsive behavior, eye contact, speech, lethargy, hypersensitivity, stereotypy, toilet training, non-compliance, aggression, impulsivity, conduct disorder, or oppositional defiance and/or social withdrawal.

A subject's improvement in one or more symptoms of an ASD may be measured on an Aberrant Behavior Checklist (ABC) scale. A subject's improvement in one or more symptoms of an ASD may be measured as a total Aberrant Behavior Checklist (ABC) score.

The total daily calories consumed by a subject administered the pharmaceutical composition may be about the same as the subject consumed prior to administration of the pharmaceutical composition.

The total daily calories consumed by a subject administered the pharmaceutical composition may be about the same as a subject administered a placebo for the same length of time.

A subject may exhibit an improvement in carbohydrate intake of at least 3-10 grams per day.

A subject may exhibit an improvement in carbohydrate intake of at least 5 grams per day.

A Block Food Screener may be used to measure food intake and/or to measure nutrient intake.

A Block Food Screener may be used to measure the quantity of food intake and/or to measure quality of food intake.

A subject may exhibit improvement in hyperactivity by 12 weeks after administration of the pharmaceutical composition in comparison to a subject administered a placebo. Hyperactivity may be measured by a Conners test.

A subject administered the pharmaceutical composition may show increased carbohydrate intake at week 12 in comparison to before the pharmaceutical composition was administered.

A subject may exhibit improvement in attention by 12 weeks after administration of the pharmaceutical composition in comparison to a subject administered a placebo. Attention may be measured by a Conners test.

A subject administered the pharmaceutical composition may show increased carbohydrate intake at week 12 in comparison to before the pharmaceutical composition was administered.

A subject may have a more alkaline stool pH after administration of the pharmaceutical composition than prior to administration of the pharmaceutical composition. A subject may have a more alkaline stool pH after at least four weeks of administration of the pharmaceutical composition than prior to administration of the pharmaceutical composition. A subject may have a more alkaline stool pH after administration of the pharmaceutical composition than the subject administered the placebo.

A subject may have an abnormal fecal chymotrypsin level before administration of the pharmaceutical composition. A subject may have a fecal chymotrypsin level of less than 13 U/g as measured in a frozen stool sample before administration of the pharmaceutical composition. A subject may have a fecal chymotrypsin level of less than 12.6 U/g as measured in a frozen stool sample before administration of the pharmaceutical composition. A subject may have a fecal chymotrypsin level of less than 10 U/g as measured in a fresh stool sample before administration of the pharmaceutical composition. A subject may have fecal chymotrypsin levels less than 9 U/g as measured in a fresh stool sample before administration of the pharmaceutical composition.

A subject may have increased fecal chymotrypsin levels after administration of the pharmaceutical composition. A subject may have increased fecal chymotrypsin levels after at least 12 weeks of administration of the pharmaceutical composition.

Improvement in one or more symptoms of an ASD may comprise an increase in protein, intake, fat intake, carbohydrate intake, and/or vitamin intake in the subject. Improvement in one or more symptoms of an ASD may comprise a decrease in carbohydrate intake.

Improvement in one or more symptoms of an ASD may comprise a decrease in the number of incidents or the severity of diarrhea, constipation, seizures, and/or bone fragility in the subject. Improvement in one or more symptoms of an ASD may comprise a decrease in the number of incidents or the severity of hyperactivity, irritability, agitation, obsessive compulsive behavior, lethargy, hypersensitivity, stereotypy, non-compliance, aggression, impulsivity, conduct disorder, or oppositional defiance and/or social withdrawal in the subject. Improvement in one or more symptoms of an ASD may comprise an increase in the number of incidents or duration of eye contact. Improvement in one or more symptoms of an ASD may comprise an increase in the number of incidents, articulation or vocabulary of the subject's speech. Improvement in one or more symptoms of an ASD may comprise an improvement in toilet training. Improvement may be exhibited by the subject administered the pharmaceutical composition and may be greater than the subject administered the placebo. A subject administered the pharmaceutical composition may further have an improvement in overall health.

A subject may have a decrease in the number or severity of infections or a decrease in the number or severity of allergic incidents after administration of the pharmaceutical composition in comparison to before the subject was administered the pharmaceutical composition. A subject may have a decrease in the number or severity of infections or a decrease in the number or severity of allergic incidents after administration of the pharmaceutical composition in comparison to the subject administered the placebo.

A subject may be diagnosed as having an ASD before administration of the pharmaceutical composition. A subject may be diagnosed as having an ASD by a DSM-IV, SCQ, or ADI-R screen. A subject may be diagnosed as having an ASD by a DSM-IV, SCQ, or ADI-R screen, prior to administration of the pharmaceutical composition. A subject may be diagnosed as having a PDD, ADD, or ADHD. A subject may be diagnosed as having autism. One or more symptoms of an ASD may be measured for the subject using an Aberrant Behavior Checklist (ABC) scale, Conners test, Expressive Vocabulary Test (EVT) test, Peabody Picture Vocabulary Test (PPVT), Social Communications Questionnaire (SCQ), ADI-R test, or DSM-IV test. In one embodiment, the EVT may be an EVT-2 test. In another embodiment, the PPVT may be a PPVT-4 test. Hyperactivity may be measured by a Conners test. In one embodiment, the Conners test may be a Conners-3 test. Hyperactivity may be measured by comparing the Conners 3 test results with hyperactivity results measured on the ABC scale.

One or more symptoms of an ASD may be measured prior to administration of the pharmaceutical composition. One or more symptoms of an ASD may be measured after or during administration of the pharmaceutical composition. One or more symptoms of an ASD may be measured one or more times after administration of the pharmaceutical composition for about 1, 2, 3, 4, 8, 12, 16, 20, 24, 28, 32, 36, 40, 44, 48, 52, 56, 60, 64, 70, 74, 78, 82, 86, or 90 weeks. Improvement may be observed after the subject is administered the pharmaceutical composition for at least about 1, 2, 3, 4, 8, 12, 16, 20, 24, 28, 32, 36, 40, 44, 48, 52, 56, 60, 64, 70, 74, 78, 82, 86, or 90 weeks. Improvement may be observed after the subject is administered the pharmaceutical composition for at least one week.

A subject may be administered the pharmaceutical composition with meals. A subject may be administered the pharmaceutical composition daily. A subject may be administered the pharmaceutical composition 1, 2, 3, 4, 5, 6, 7, 8, 9, 10, 11 or 12 times a day. A subject may be administered the pharmaceutical composition once a day, twice a day, or three time a day. A subject may be administered the pharmaceutical composition for at least 12 weeks. A subject may be administered the pharmaceutical composition for at least 3-6 months, 6-12 months, 12-18 months, or 18-24 months. A subject may be administered the pharmaceutical composition for at least 1, 2, 3, 4, 5, 6, 7, 8, 9, or 10 years.

The therapeutic composition may comprise proteases, amylases, and lipases. In one embodiment, the proteases comprise trypsin and/or chymotrypsin. In another embodiment, the therapeutic composition may be pancreatin. In another embodiment, the therapeutic composition may be a solid form of pancreatin. In another embodiment, the therapeutic composition may be a crystalline form of pancreatin.

In one embodiment, the pharmaceutical composition does not produce a sedating effect, an increase in dizziness, Parkinson's disease symptoms, dystonia, akathisia, somnolence, fatigue, extrapyramidal disorders, tremor, or drooling.

In another embodiment, the therapeutic composition comprises a coating. The coating may be an enteric coating. The coating may comprise a lipid, a lipid mixture, a blend of lipid and emulsifiers, or a polymer. In one embodiment, the coating comprises a soy lipid. The coating may mask the task and/or smell of the therapeutic composition. In another embodiment, the enteric coating comprises hypromellose phthalate, dimethicone 1000, dibutyl phthalate, or a combination thereof. The coating may further comprise an emulsifier. The one or more excipients may comprise a cellulose.

The therapeutic composition may be released into the proximal small intestine following administration to the subject. The therapeutic composition may be released into the duodenum or jejunum portion of small intestine following administration to the subject. The therapeutic composition may be released into the ileum portion of small intestine following administration to the subject. The pharmaceutical composition may be administered by oral administration. The pharmaceutical composition may be administered directly into the gastrointestinal system. The pharmaceutical composition may be administered through a nasal-gastrointestinal-tube (NG Tube) or gastrointestinal-tube (G-tube).

A subject administered the pharmaceutical composition may exhibit improvement in two, three, four, five, six, or more symptoms of an ASD.

A subject may be between the ages of 1-18 years of age. A subject may be between the ages of 2-16 years of age. A subject may be between the ages of 1-10 years of age. A subject may be between the ages of 3-8 years of age. A subject may be between the ages of 9-12 years of age.

A subject may exhibit an increase in vitamin intake (by weight) of B6, B 12, A, C, E, K, Copper, Iron, Cholesterol, Niacin, Riboflavin, Thiamin, or Zinc after administration of the pharmaceutical composition.

A subject may exhibit an increase in vitamin intake of one or more of plant based vitamin A, Carotenoids (alpha and beta carotene), vitamin K, vitamin E, vitamin C, Selenium, copper, folate, lutein, lycopene, magnesium, potassium, phosphorus, sodium, polyunsaturated fatty acids, monounsaturated fatty acids, saturated fats, cholesterol, and/or Theobromine. The increase in vitamin intake by the subject may be in comparison to before the subject began administration of the pharmaceutical composition.
The increase in vitamin intake by the subject may be in comparison to the subject administered the placebo.

A subject may exhibit an increase in B6, B 12, A, C, E, K, Copper, Iron, Cholesterol, Niacin, Riboflavin, Thiamin, or Zinc blood levels after administration of the pharmaceutical composition

A subject may exhibit an increase in fecal levels of plant based vitamin A, Carotenoids (alpha and beta carotene), vitamin K, vitamin E, vitamin C, Selenium, copper, folate, lutein, lycopene, magnesium, potassium, phosphorus, sodium, polyunsaturated fatty acids, monounsaturated fatty acids, saturated fats, cholesterol, and/or Theobromine. The increase in blood or fecal levels of the subject may be in comparison to before the subject began administration of the pharmaceutical composition. The increase in blood or fecal levels by the subject administered the pharmaceutical composition may be in comparison to the subject administered the placebo.

In another embodiment, the subject further has a vitamin K deficiency. The vitamin K deficiency may be measured by detecting levels of gamma carboxylated proteins in the blood.

Disclosed herein but not claimed is a method of improving expressive and/or receptive language capabilities a subject with a symptom of an ASD, comprising administering a pharmaceutical composition comprising one or more excipients and a therapeutic composition, wherein the therapeutic composition comprises protease, amylase and/or lipase. A subject may exhibit an improvement in expressive and/or receptive language capabilities after administration of the pharmaceutical composition in comparison to before the subject was administered the pharmaceutical composition. A subject may exhibit a greater improvement in expressive and/or receptive language capabilities after administration of the pharmaceutical composition than a subject with a symptom of an ASD administered a placebo. The expressive and/or receptive language capabilities may be measured with an Expressive Vocabulary Test (EVT) and/or Peabody Picture Vocabulary Test (PPVT). In one embodiment, the EVT test may be an EVT-A or EVT-B test. In one embodiment, the PPVT test may be a PPVT-A or PPVT-B test. A subject administered the pharmaceutical composition may exhibit a greater improvement in expressive language capabilities than receptive language capabilities.

Disclosed herein but not claimed is a method of treating a subject with one or more symptoms of an ASD, comprising: administering to the subject a pharmaceutical composition comprising one or more excipients and a therapeutic composition, wherein the therapeutic composition comprises protease, amylase and/or lipase, wherein the subject exhibits improvement in one or more symptoms of an ASD comprising: (a) protein intake, fat intake, carbohydrate intake, vitamin intake, seizures, and/or bone fragility; and/or (b) irritability, agitation, lethargy, hypersensitivity, non-compliance, aggression, impulsivity, conduct disorder, or oppositional defiance and/or social withdrawal and wherein the subject has at least a 10% or greater improvement in the one or more symptoms of an ASD after administration of the pharmaceutical composition. A subject may exhibit increases overall fat intake (by weight) of at least about 10% after administration of the pharmaceutical composition. A subject may exhibit increases overall protein intake (by weight) of at least about 10% after administration of the pharmaceutical composition. A subject may exhibit increases overall carbohydrate intake (by weight) of at least about 10% after administration of the pharmaceutical composition.

Disclosed herein but not claimed is a method of increasing body mass or physical growth in a subject with a symptom of an ASD, comprising administering a pharmaceutical composition comprising one or more excipients and a therapeutic composition, wherein the therapeutic composition comprises protease, amylase and/or lipase.

A subject may be diagnosed as having an ASD before administration of the pharmaceutical composition. A subject may be diagnosed as having an ASD by a DSM-IV, SCQ, or ADI-R screen. A subject may be diagnosed as having an ASD by a DSM-IV, SCQ, or ADI-R screen, prior to administration of the pharmaceutical composition.
A subject may be diagnosed as having a PDD, ADD, or ADHD. A subject may be diagnosed as having autism.

One or more symptoms of an ASD may be measured for the subject using an Aberrant Behavior Checklist (ABC) scale, Conners test, Expressive Vocabulary Test (EVT) test, Peabody Picture Vocabulary Test (PPVT), Social Communications Questionnaire (SCQ), ADI-R test, or DSM-IV test. In one embodiment, the EVT may be an EVT-2 test. In another embodiment, the PPVT may be a PPVT-4 test. Hyperactivity may be measured by a Conners test. In another embodiment, the Conners test may be a Conners-3 test.

Hyperactivity may be measured by comparing the Conners 3 test results with hyperactivity results measured on the ABC scale. The one or more symptoms of an ASD may be measured prior to administration of the pharmaceutical composition. The one or more symptoms of an ASD may be measured after or during administration of the pharmaceutical composition. Improvement may be observed in the subject administered the pharmaceutical composition 12 weeks or more after the subject began administration of the pharmaceutical composition. A subject administered the pharmaceutical composition may exhibit a greater improvement in an EVT score than a PPVT score. A subject administered the pharmaceutical composition may exhibit a greater improvement in EVT or PPVT score and the subject may exhibit increased protein intake after the subject began administration of the pharmaceutical composition. Improvement may be observed 12 or more weeks after the subject begins administration of the pharmaceutical composition.

A subject may exhibit greater than a 10%, 20%, 30%, 40%, 50%, 60%, 70%, 80%, 90% or 100% improvement in one or more additional symptoms of an ASD after administration of the pharmaceutical composition. Total daily protein, fat, carbohydrate and/or vitamin intake (by weight) by the subject may increase following administration of the pharmaceutical composition increases in comparison to protein, fat, carbohydrate, and/or vitamin intake by the subject before administration of the pharmaceutical composition. Total daily carbohydrate intake (by weight) by the subject may decrease following administration of the pharmaceutical composition in comparison to carbohydrate intake by the subject before administration of the pharmaceutical composition. A subject may exhibit a greater increase in daily protein, fat, carbohydrate and/or vitamin intake (by weight) following administration of the pharmaceutical composition in comparison to a subject with one or more symptoms of an ASD following administration of a placebo. The greater the amount of daily protein, fat, carbohydrate and/or vitamin intake (by weight) consumed by the subject after administration of the pharmaceutical composition, the greater the subject's improvement in one or more symptoms of an ASD. The daily protein, fat, carbohydrate and/or vitamin intake (by weight) consumed by the subject may be measured before the subject was administered the pharmaceutical composition and at about 2, 4, 6, 8, 10, 12, 14, 16, 18, 20, 24, 36, 48, or 52 weeks after administration of the pharmaceutical composition begins. A subject may have a greater increase in daily vitamin intake (by weight) following administration of the pharmaceutical composition in comparison to a subject with one or more symptoms of an ASD following administration of a placebo. A subject may have a greater increase in daily carbohydrate intake (by weight) following administration of the pharmaceutical composition in comparison to a subject with one or more symptoms of an ASD following administration of a placebo. The total daily calories consumed by the subject may increase after administration of the pharmaceutical composition in comparison to total daily calories consumed by the subject before administration of the pharmaceutical composition. The more daily calories the subject consumed after administration of the pharmaceutical composition, the greater the subject's improvement in one or more symptoms of an ASD. The total amount of daily calories consumed by the subject may be measured before the subject may be administered the pharmaceutical composition and at about 2, 4, 6, 8, 10, 12, 14, 16, 18, 20, 24, 36, 48 or 52 weeks after administration of the pharmaceutical composition begins. A subject may consume at least 800 kcalories per day after about 12 weeks of administration of the pharmaceutical composition. A subject may further exhibit an improvement of a symptom comprising diarrhea, constipation, seizures, bone fragility, and/or hyperactivity.

A subject may have a more alkaline stool pH after administration of the pharmaceutical composition than prior to administration of the pharmaceutical composition. A subject may have a more alkaline stool pH after at least four weeks of administration of the pharmaceutical composition than prior to administration of the pharmaceutical composition. A subject may have a more alkaline stool pH after administration of the pharmaceutical composition than the subject administered the placebo. A subject may have a more alkaline stool pH after at least four weeks of administration of the pharmaceutical composition than prior to administration of the pharmaceutical composition. A subject may have an abnormal fecal chymotrypsin level before administration of the pharmaceutical composition. A subject may have a fecal chymotrypsin level of less than 13 U/g as measured in a frozen stool sample before administration of the pharmaceutical composition. A subject may have a fecal chymotrypsin level of less than 12.6 U/g as measured in a frozen stool sample before administration of the pharmaceutical composition. A subject may have a fecal chymotrypsin level of less than 10 U/g as measured in a fresh stool sample before administration of the pharmaceutical composition. A subject may have a fecal chymotrypsin levels less than 9 U/g as measured in a fresh stool sample before administration of the pharmaceutical composition. A subject may have an increased fecal chymotrypsin levels after administration of the pharmaceutical composition. A subject may have an increased fecal chymotrypsin levels after at least 12 weeks of administration of the pharmaceutical composition.

A subject may further have an improvement in one or more additional symptoms of an ASD. The one or more symptoms of an ASD may be measured prior to administration of the pharmaceutical composition. The one or more symptoms of an ASD may be measured after or during administration of the pharmaceutical composition. The one or more symptoms of an ASD may be measured one or more times after administration of the pharmaceutical composition for 1, 2, 3, 4, 8, 12, 16, 20, 24, 28, 32, 36, 40, 44, 48, 52, 56, 60, 64, 70, 74, 78, 82, 86, or 90 weeks. Improvement may be observed after the subject may be administered the pharmaceutical composition for at least 1, 2, 3, 4, 8, 12, 16, 20, 24, 28, 32, 36, 40, 44, 48, 52, 56, 60, 64, 70, 74, 78, 82, 86, or 90 weeks. Improvement may be observed after the subject may be administered the pharmaceutical composition for at least one week.

A subject may be administered the pharmaceutical composition with meals. A subject may be administered the pharmaceutical composition daily. A subject may be administered the pharmaceutical composition 1, 2, 3, 4, 5, 6, 7, 8, 9, 10, 11, or 12 times a day. A subject may be administered the pharmaceutical composition once a day. A subject may be administered the pharmaceutical composition for at least 12 weeks. A subject may be administered the pharmaceutical composition for at least 3-6 months, 6-12 months, 12-18 months, or 18-24 months. A subject may be administered the pharmaceutical composition for at least 1, 2, 3, 4, 5, 6, 7, 8, 9, or 10 years.

In one embodiment, a therapeutic composition comprises proteases, amylases and lipases. In another embodiment, the proteases comprise trypsin and/or chymotrypsin. In another embodiment, the therapeutic composition may be pancreatin. In another embodiment, the therapeutic composition may be a solid form of pancreatin. In another embodiment, the therapeutic composition may be a crystalline form of pancreatin.

In another embodiment, the pharmaceutical composition does not produce a sedating effect, an increase in dizziness, Parkinson's disease symptoms, dystonia, akathisia, somnolence, fatigue, extrapyramidal disorders, tremor, or drooling.

The therapeutic composition may comprise a coating. The coating may be an enteric coating. The coating may comprise a lipid, a lipid mixture, a blend of lipid and emulsifiers, or a polymer. In one embodiment, coating comprises a soy lipid. The coating may mask the task and/or smell of the therapeutic composition. In one embodiment, the enteric coating comprises hypromellose phthalate, dimethicone 1000, dibutyl phthalate, or a combination thereof. The coating may further comprise an emulsifier. In any of such embodiments described herein, the one or more excipients comprise a cellulose.

The therapeutic composition may be released into the proximal small intestine following administration to the subject. The therapeutic composition may be released into the duodenum or jejunum portion of small intestine following administration to the subject. The therapeutic composition may be released into the ileum portion of small intestine following administration to the subject. The pharmaceutical composition may be administered by oral administration. The pharmaceutical composition may be administered directly into the gastrointestinal system. The pharmaceutical composition may be administered through a nasal-gastrointestinal-tube (NG Tube) or gastrointestinal-tube (G-tube).

A subject administered the pharmaceutical composition may exhibit improvement in two, three, four, five, six or more symptoms of an ASD.

A subject may be between the ages of 1-18 years of age. A subject may be between the ages of 2-16 years of age. A subject may be between the ages of 1-10 years of age. A subject may be between the ages of 3-8 years of age. A subject may be between the ages of 9-12 years of age.

A subject may exhibit an increase in vitamin intake (by weight) of B6, B12, A, C, E, K, Copper, Iron, Cholesterol, Niacin, Riboflavin, Thiamin, or Zinc after administration of the pharmaceutical composition. A subject may exhibit an increase in vitamin intake of one or more of plant based vitamin A, Carotenoids (alpha and beta carotene), vitamin K, vitamin E, vitamin C, Selenium, copper, folate, lutein, lycopene, magnesium, potassium, phosphorus, sodium, polyunsaturated fatty acids, monounsaturated fatty acids, saturated fats, cholesterol, and/or Theobromine. The increase in vitamin intake by the subject may be in comparison to before the subject began administration of the pharmaceutical composition. The increase in vitamin intake by the subject may be in comparison to the subject administered the placebo.

A subject may exhibit an increase in B6, B 12, A, C, E, K, Copper, Iron, Cholesterol, Niacin, Riboflavin, Thiamin, or Zinc blood levels after administration of the pharmaceutical composition. A subject may exhibit an increase in fecal levels of plant based vitamin A, Carotenoids (alpha and beta carotene), vitamin K, vitamin E, vitamin C, Selenium, copper, folate, lutein, lycopene, magnesium, potassium, phosphorus, sodium, polyunsaturated fatty acids, monounsaturated fatty acids, saturated fats, cholesterol, and/or Theobromine. The increase in blood or fecal levels of the subject may be in comparison to before the subject began administration of the pharmaceutical composition. The increase in blood or fecal levels by the subject administered the pharmaceutical composition may be in comparison to the subject administered the placebo.

Disclosed herein but not claimed is a method of treating a subject with a PDD, ADD, or ADHD in need thereof with a pharmaceutical composition comprising: administering the pharmaceutical composition comprising one or more pancreatic digestive enzymes to the subject, wherein the subject shows a reduction in the severity or frequency of one or more symptoms associated with a PDD, ADD, or ADHD after administration of the pharmaceutical composition.

Disclosed herein but not claimed is a method of treating a subject with a PDD, ADD, or ADHD in need thereof with a pharmaceutical composition comprising:
administering the pharmaceutical composition comprising one or more pancreatic digestive enzymes to the subject, wherein the subject has a vitamin K deficiency, and
wherein the subject shows a reduction in the severity or frequency of one or more symptoms associated with a ASD, ADD, or ADHD after administration of the pharmaceutical composition. The ASD may be autism. The vitamin K deficiency may be measured by detecting levels of gamma carboxylated proteins in the blood.

Disclosed herein but not claimed is a method of treating a subject with a ASD, ADD, or ADHD in need thereof with a pharmaceutical composition comprising: administering the pharmaceutical composition comprising: 1) one or more pancreatic digestive enzymes; 2) glutamate enhancing therapy to the subject, wherein the subject has a vitamin K deficiency or an abnormal fecal chymotrypsin level, and wherein the subject shows a reduction in the severity or frequency of one or more symptoms associated with a ASD, ADD, or ADHD after administration of the pharmaceutical composition. The ASD may be autism. The vitamin K deficiency may be measured by detecting levels of gamma carboxylated proteins in the blood.

Disclosed herein but not claimed is a method of treating a subject with a ASD, ADD, or ADHD in need thereof with a pharmaceutical composition comprising: administering the pharmaceutical composition comprising one or more pancreatic digestive enzymes to the subject, wherein the subject has a vitamin K deficiency, and wherein the subject shows a reduction in the severity or frequency of one or more symptoms associated with a ASD, ADD, or ADHD after administration of the pharmaceutical composition.

Disclosed herein but not claimed is a method of treating a subject with one or more symptoms of an ASD, comprising: administering to the subject a pharmaceutical composition comprising one or more excipients and a therapeutic composition, wherein the therapeutic composition comprises protease, amylase and/or lipase, wherein the subject exhibits improvement in one or more symptoms of an ASD, wherein the subject has one or more allergies and has a larger intake of fiber, calories, fat, protein, and carbohydrates after administration of the pharmaceutical composition than a subject that did not have allergies.

### BRIEF DESCRIPTION OF THE DRAWINGS

The invention is defined in the claims. Any of the following figures that do not fall within the scope of the claims are not part of the invention and are only provided for informative or comparative purposes.

The novel features of the embodiments are set forth with particularity in the appended claims. A better understanding of the features and advantages of the present embodiments will be obtained by reference to the following detailed description that sets forth illustrative examples, in which the principles of the embodiments are utilized, and the accompanying drawings of which:
Figure 1 shows a bar graph of the % lipase activity in the raw digestive enzyme particles, and following encapsulation, for coated enzyme preparations containing 70%, 80%, and 90% digestive enzymes by weight.
Figure 2 shows a bar graph of the % enzyme release for the enzyme preparations containing 70%, 80%, and 90% digestive enzymes by weight, at the times indicated on the y-axis.
Figure 3 shows a bar graph of the particle size distributions of the raw digestive enzyme particles compared with the particle size distributions in coated enzyme preparations containing 70% or 80% digestive enzymes by weight.
Figure 4 shows the flow chart for a process that can be used to encapsulate digestive enzyme particles.

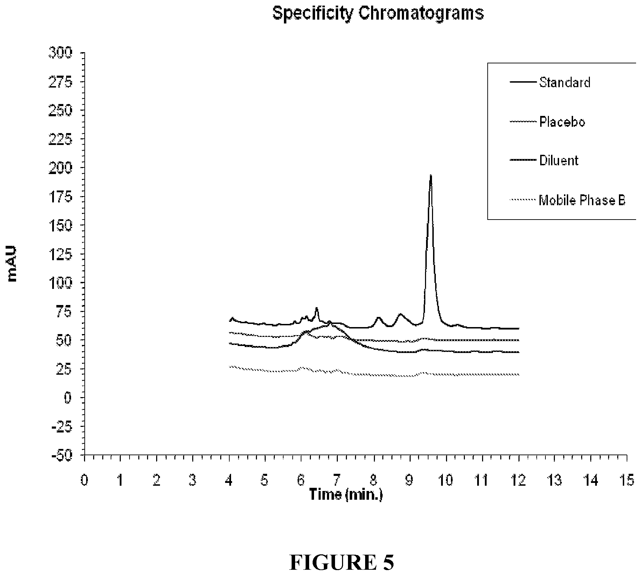

Figure 5 shows a chromatogram of peak area (mAU) vs. time for working standard (top line), diluent (line that starts third from the top when time is 4 minutes), mobile phase used in the HPLC (bottom line at 4 minutes) and placebo (second to the top line when time is 4 minutes), which demonstrate no interference with the standard trypsin peak.
Figure 6 shows FCT levels measured in 26 subjects with symptoms of autism.
Figure 7 shows FCT levels measured in 46 subjects. 25 of the subjects had symptoms of autism, while 21 subjects did not have symptoms of autism.

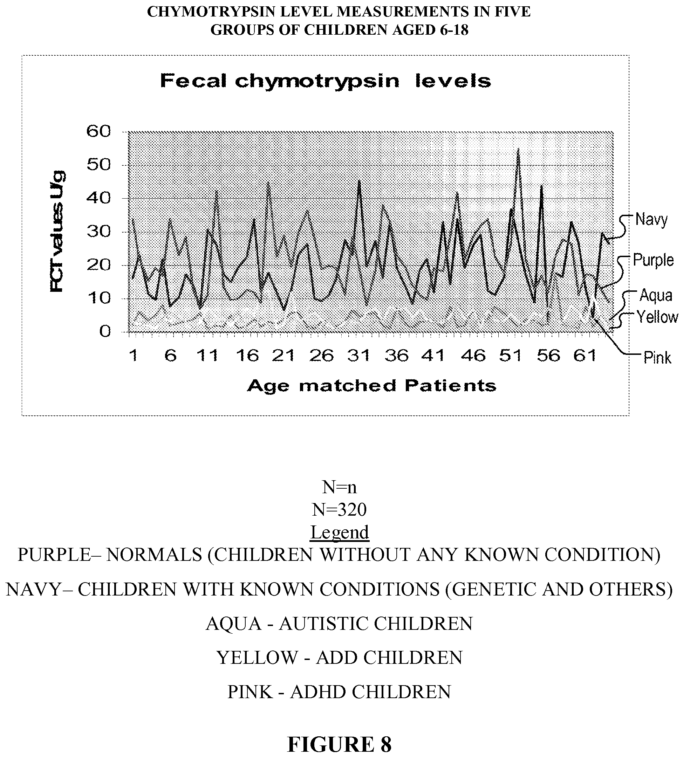

Figure 8 shows fecal chymotrypsin levels measured in 320 age-matched subjects. The navy line (in grayscale, the upper, black line) shows FCT levels for subjects with known conditions (genetic and other conditions). The purple line (in grayscale, the upper, dark gray line), shows FCT levels for normal subjects without any known condition. The aqua line, (in gray scale, the lower, medium gray line), shows FCT levels for subjects with autism. The pink line (in gray scale, the lower, dark gray line), shows FCT measurements for subjects with ADHD. The yellow line (in grayscale, the lower, light gray line), shows FCT measurements for subjects with ADD.

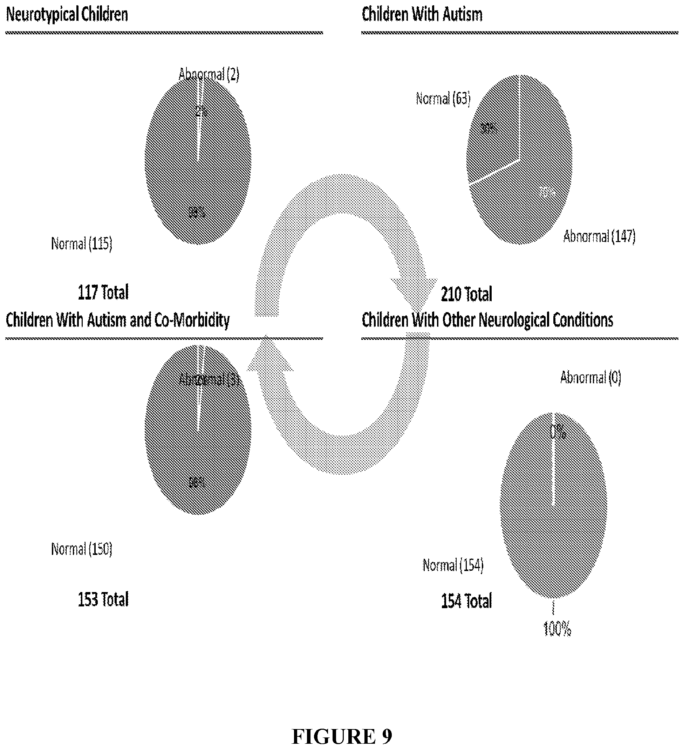

Figure 9 illustrates abnormal chymotrypsin levels in various subject populations.

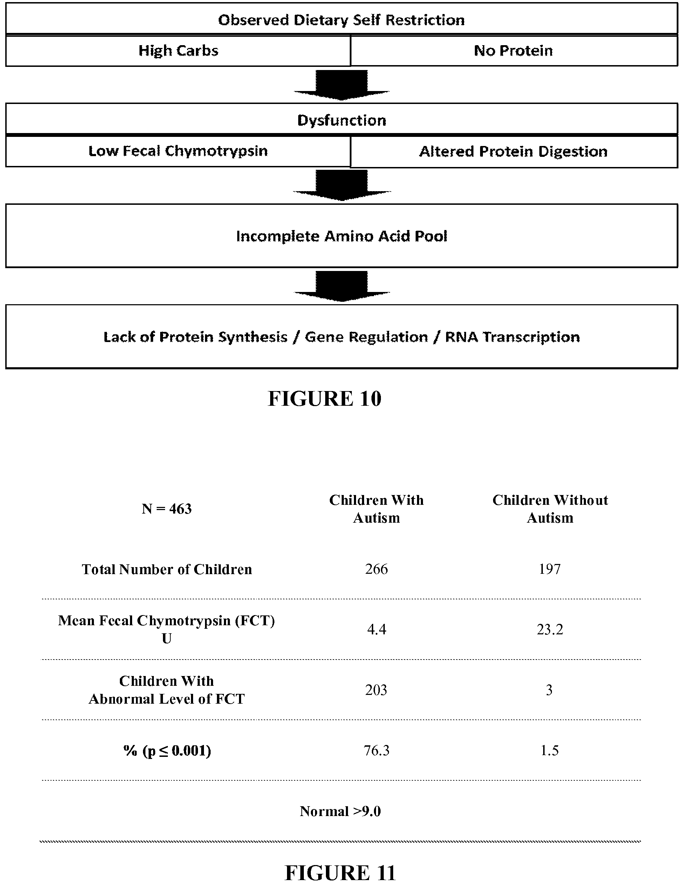

Figure 10 provides an exemplary dietary outcome of self-imposed dietary restrictions observed in autistic subjects.
Figure 11 provides fecal chymotrypsin levels.

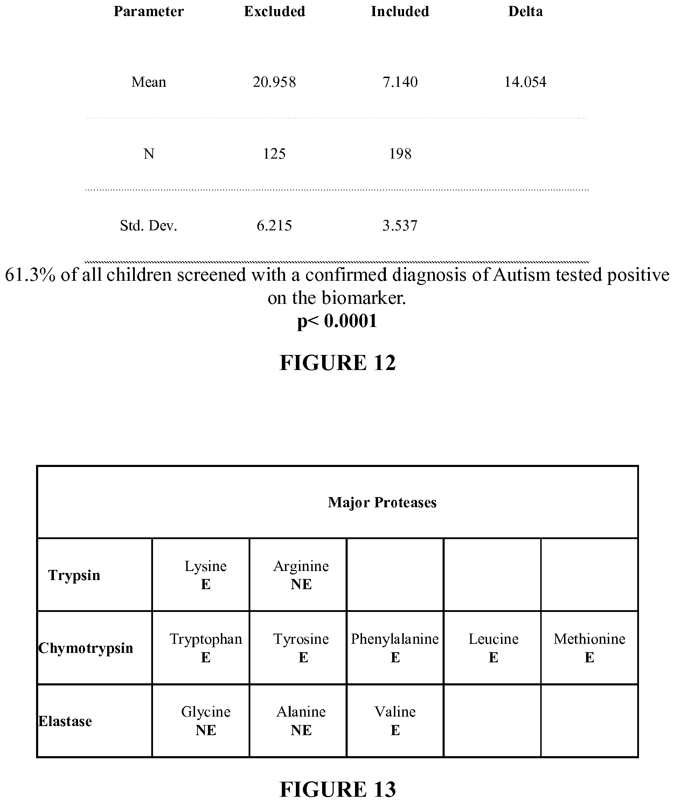

Figure 12 provides fecal chymotrypsin levels.
Figure 13 provides exemplary major proteases.

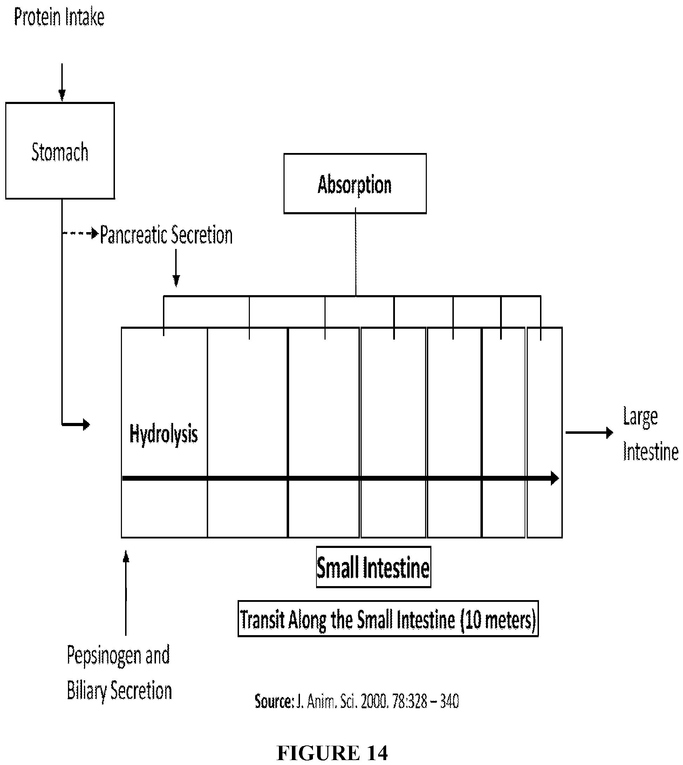

Figure 14 illustrates representative movement of protein through the body after intake.
Figure 15 shows the changes in fecal chymotrypsin levels from baseline in subjects administered the Formulation 1 *versus* subjects administered the placebo.
Figure 16 illustrates the pH change in subjects treated with Formulation 1 versus placebo during the 12-week clinical trial.
Figure 17 shows the PPVT results the growth scores for subjects on Formulation 1 exceeded those on the placebo. Growth scores are adjusted for age and for standardization of scores across the entire study.
Figure 18 shows the EVT scores demonstrate a large difference between subjects administered the Formulation 1 and subjects administered the placebo.
Figure 19 illustrates the EVT mean standard score change as a function of protein intake (g) at week 12.

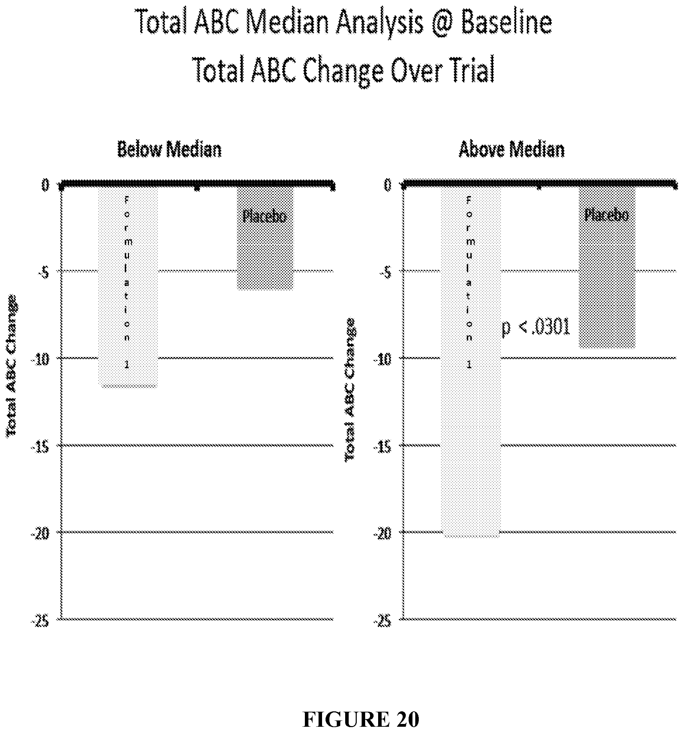

Figure 20 illustrates the total ABC median analysis at baseline total ABC change over the course of the 12-week trial.

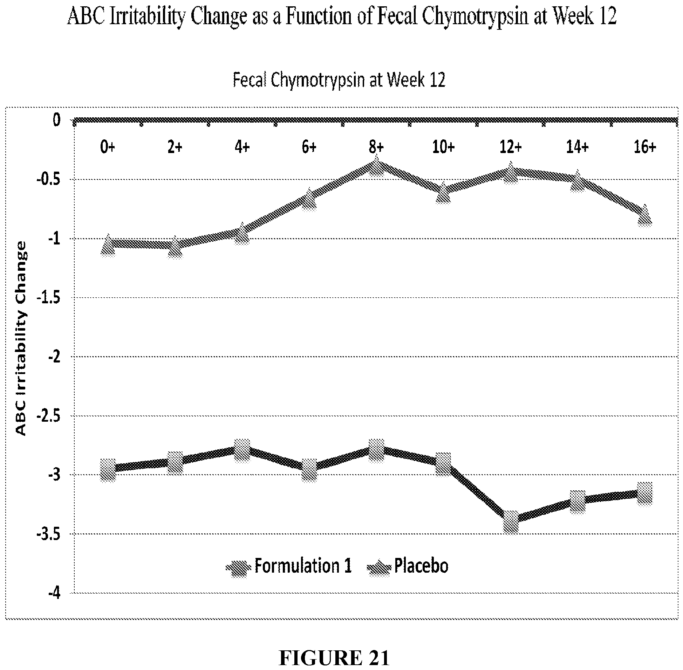

Figure 21 illustrates the ABC irritability change as a function of fecal chymotrypsin at week 12. At week 12 there is a statistically significant difference between placebo and Formulation 1 subjects in the irritability scores on the ABC regardless of their level of fecal chymotrypsin at week 12.

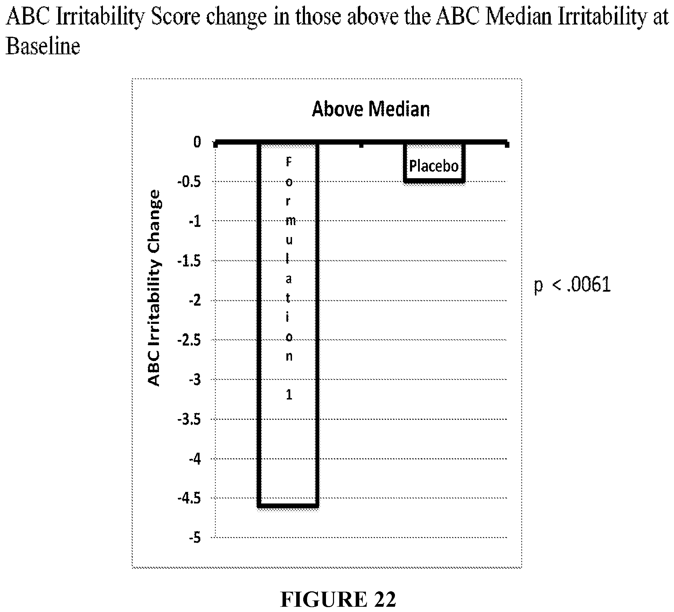

Figure 22 illustrates the ABC irritability score change in subjects above the ABC median irritability at baseline. In subjects whose baseline median ABC subscale for irritability was above the median at baseline, Formulation 1 improved significantly over placebo.

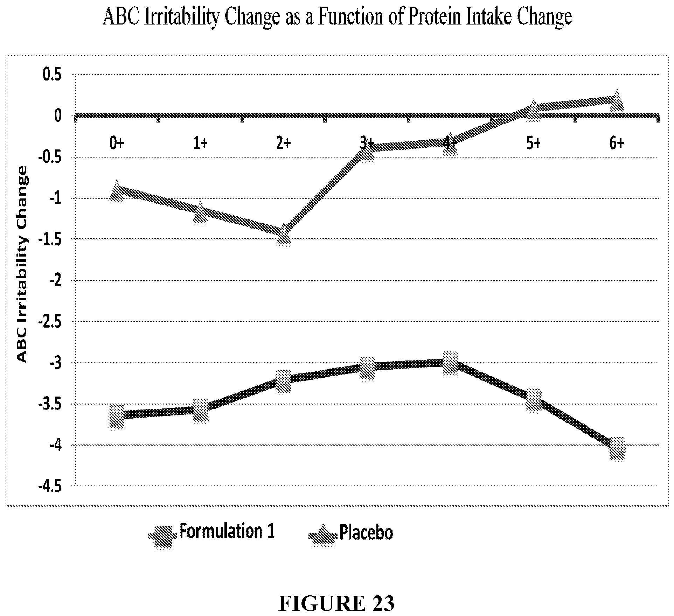

Figure 23 illustrates the ABC irritability change as a function of protein intake change at week 12. There is a difference between the Formulation 1 subjects and the placebo subjects with respect to the ABC irritability changes during the trial. The subjects on Formulation 1 had a greater change compared to the placebos regardless of the protein intake change over the course of the trial. Of note at about a 4 g+ increase in protein intake per day, the ABC irritability change continues to improve in the Formulation 1 subjects, and it worsens in the placebo subjects.

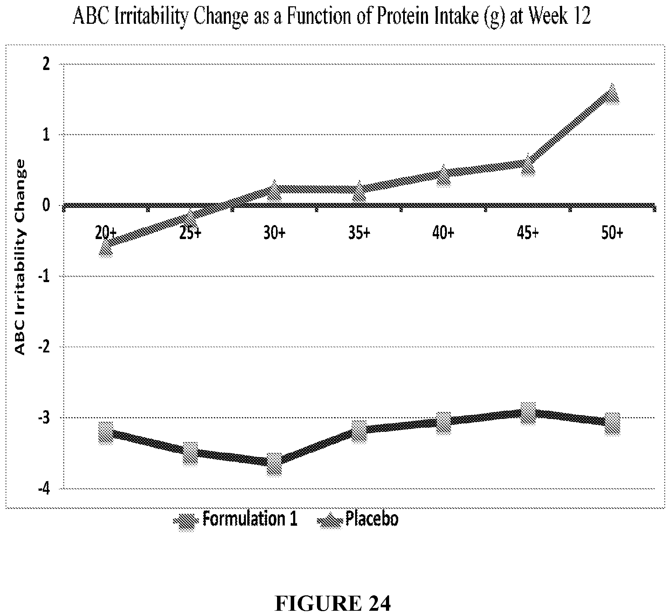

Figure 24 illustrates the ABC irritability change as a function of protein intake (g) at week 12. There is a difference between the Formulation 1 subjects and the placebo subjects with respect to the ABC irritability changes during the trial. The subjects on Formulation 1 had a greater improvement in ABC irritability scores compared to the placebos regardless of the protein intake at week 12. Of note, at about a 40 g+ in overall protein intake, the ABC irritability change remains relatively constant in the Formulation 1 subjects, and it worsens in the placebo subjects. The greater the protein intake at week 12 in the Formulation 1 subjects the ABC irritability scores are constant, and in the placebo the greater the protein intake at week 12, the worse subjects did on the ABC irritability scale, and the change in the placebo scores went down.

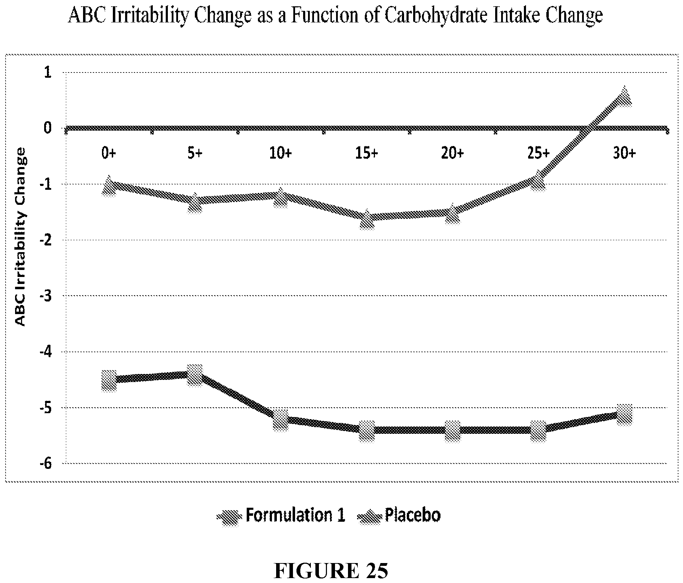

Figure 25 illustrates the ABC irritability change as a function of carbohydrate intake change. Regardless of the carbohydrate intake change during the trial, subjects administered Formulation 1 improved significantly (lower number) over subjects on the placebo. There also was a worsening in subjects on placebo as the carbohydrate (CHO) levels increase above 25+ grams.

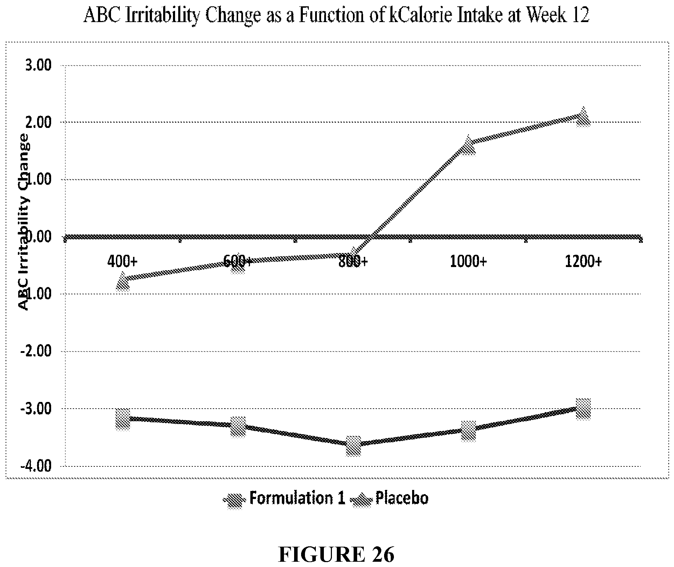

Figure 26 illustrates the ABC irritability change as a function of kCalorie intake at week 12. ABC irritability change improvement was greater in subjects on Formulation 1 regardless of the levels of calorie intake as measured at week 12. The Formulation 1 group irritability improvement change remained steady regardless of the caloric intake level as measured during week 12. The placebo group worsened in their irritability change as the level of calories measured was higher. It was especially noticeable at 800+ total calorie intake. It is important to note that these levels below 1200 calories are all abnormal levels of caloric intake.

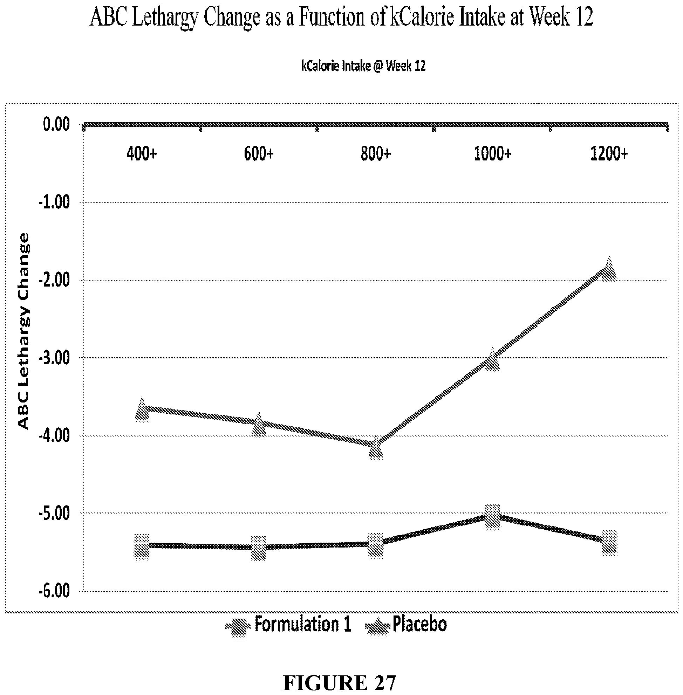

Figure 27 illustrates the ABC lethargy change as a function of kCalorie intake at week 12. Subjects on Formulation 1 had a great improvement in social withdrawal/ lethargy compared to subjects on placebo regardless of amount caloric intake. When subjects on placebo reached approximately 800+ calories per day in intake they started to worsen.

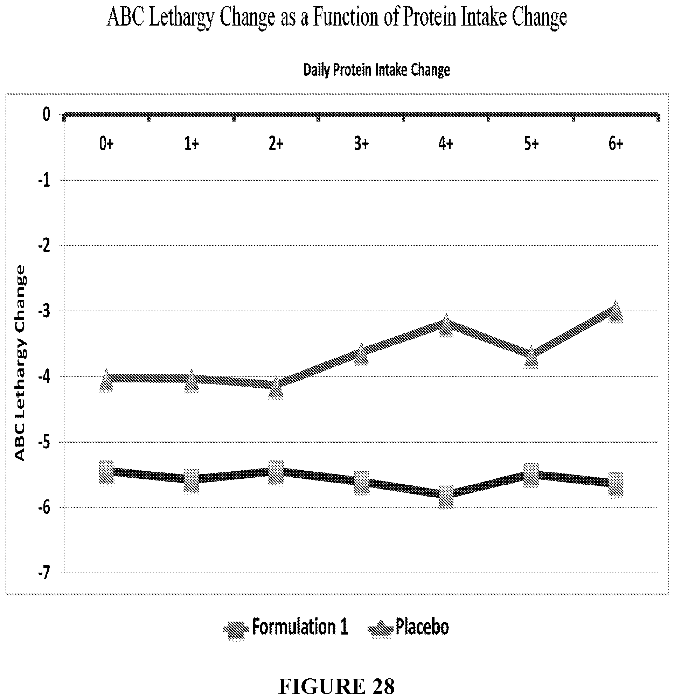

Figure 28 illustrates the ABC lethargy change as a function of protein intake change. Subjects on Formulation 1 had greater improvement in lethargy/social withdrawal compared to subjects on the placebo regardless of the protein intake. Subjects who had a 2+ gram change on the placebo continued to worsen with increasing amount of change from baseline in protein consumption.

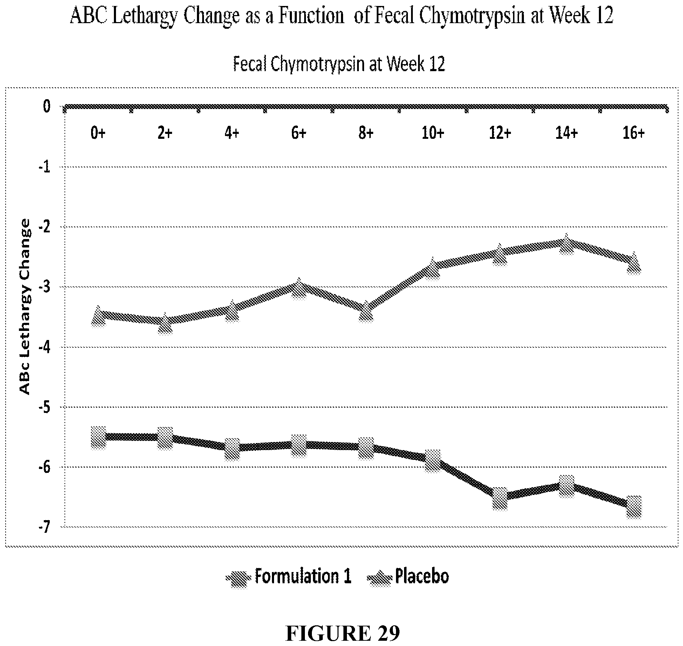

Figure 29 illustrates the ABC lethargy change as a function of fecal chymotrypsin at week 12. Subjects on Formulation 1 had greater improvement in lethargy/social withdrawal compared to subjects on the placebo regardless of their fecal chymotrypsin levels at week 12. Subjects who had a fecal chymotrypsin level of 8+ units/g or greater at week 12 and received Formulation 1 improved more than subjects administered a placebo, who had levels of fecal chymotrypsin at 8+ U/g, improved even more. Subjects who were on the placebo continued to worsen with increasing levels of FCT at the end of the trial.

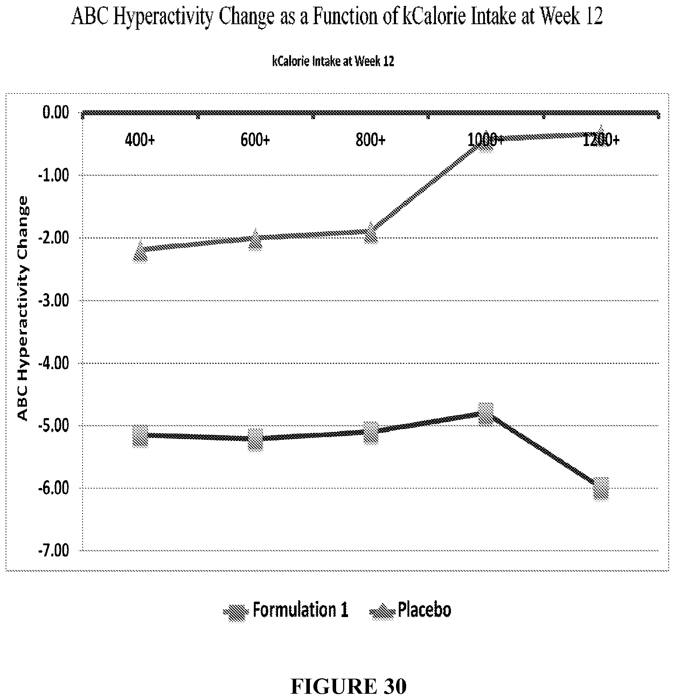

Figure 30 illustrates the ABC hyperactivity change as a function of kCalorie intake at week 12.

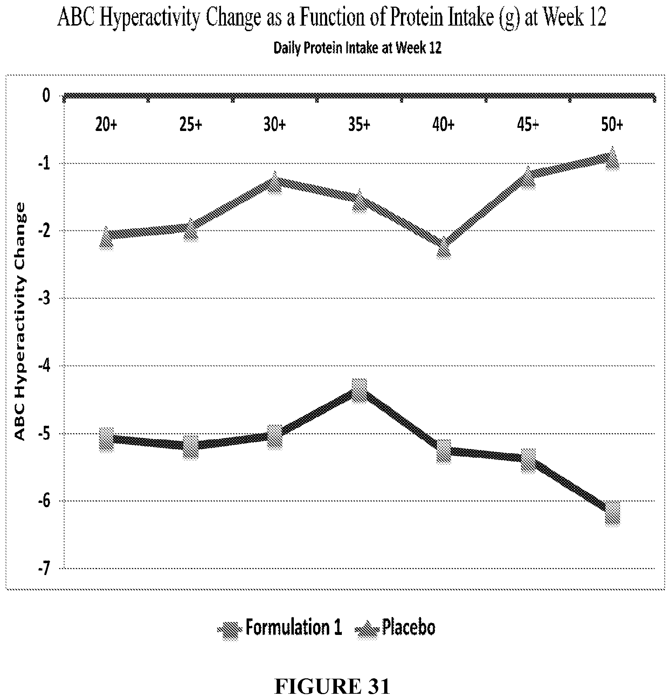

Figure 31 illustrates the ABC hyperactivity change as a function of protein intake (g) at week 12.

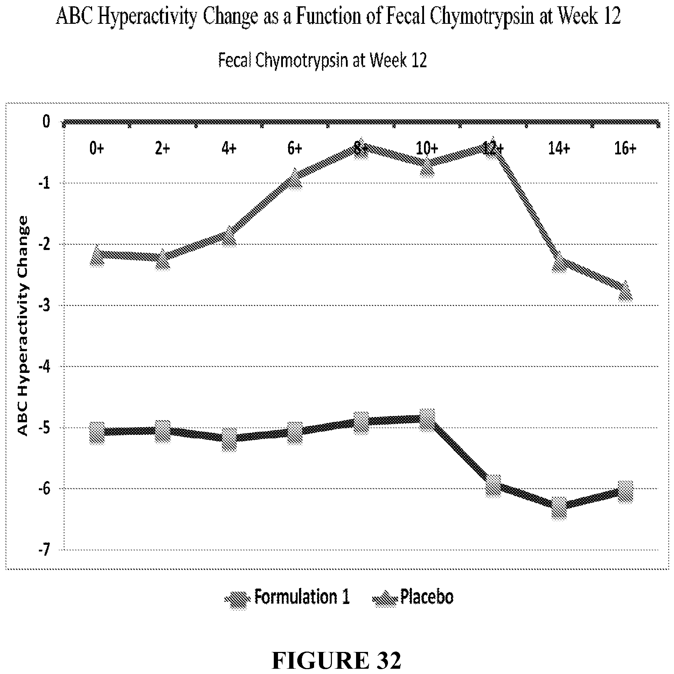

Figure 32 illustrates the ABC hyperactivity change as a function of fecal chymotrypsin at week 12.

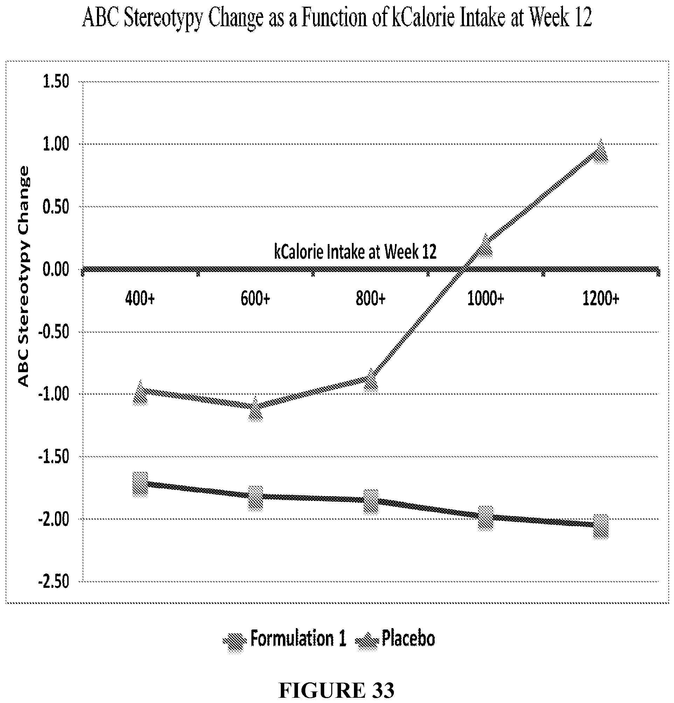

Figure 33 illustrates the ABC stereotypy change as a function of kCalorie intake at week 12.

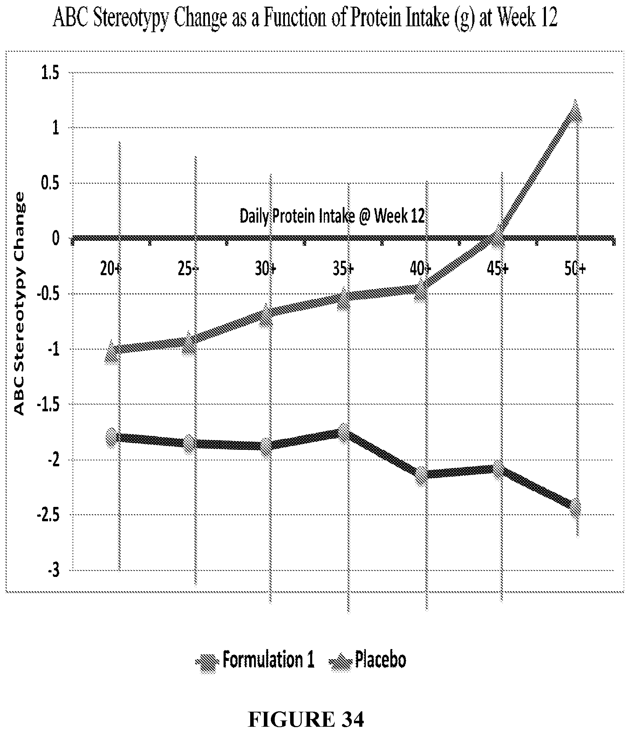

Figure 34 illustrates the ABC stereotypy change as a function of protein intake (g) at week 12.

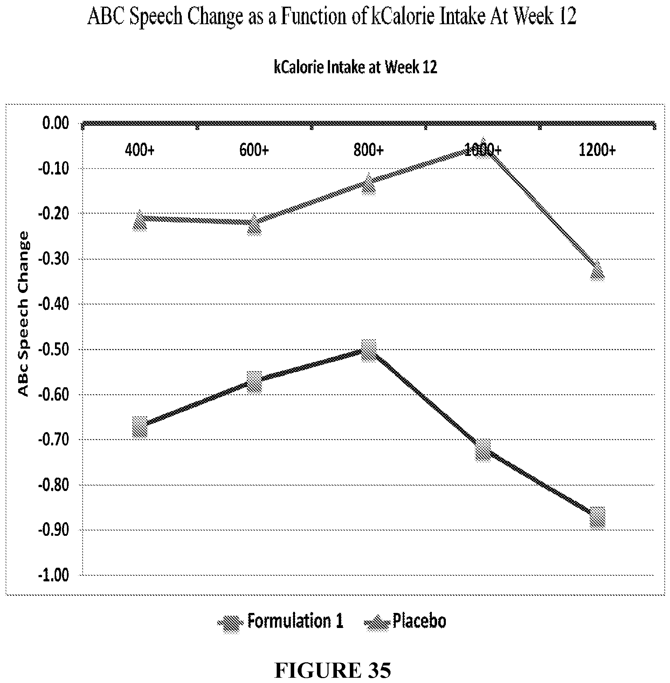

Figure 35 illustrates the ABC speech change as a function of kCalorie intake at week 12.

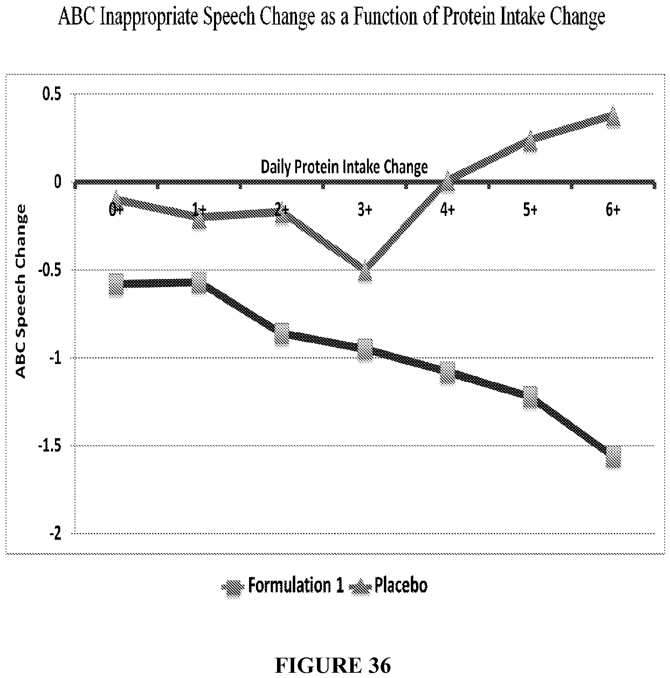

Figure 36 illustrates the ABC inappropriate speech change as a function of protein intake change at week 12.

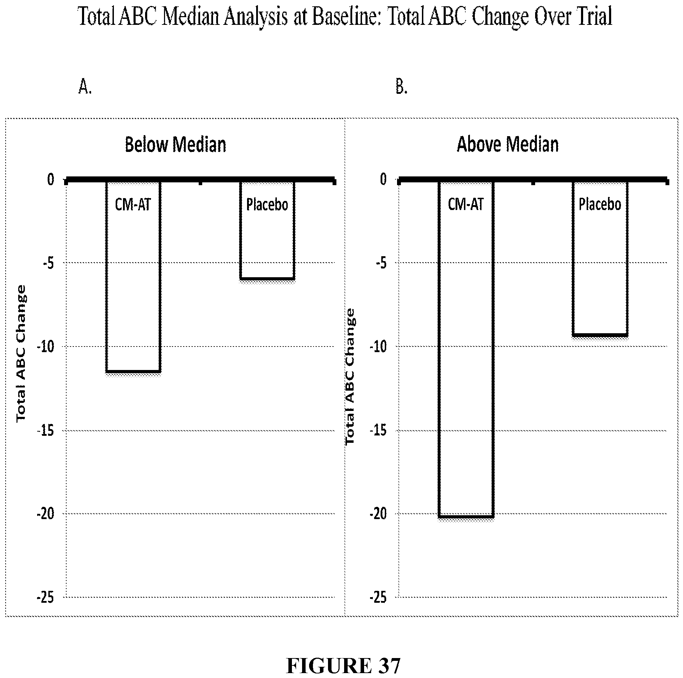

Figures 37A-B illustrate the total ABC median analysis at baseline: total ABC change over the 12-week trial. The overall measure of ABC change demonstrated that subjects above the median at baseline responded more robustly to Formulation 1 over subjects treated with placebo. Results below the median are shown in Figure 37A; results above the median are shown in Figure 37B.

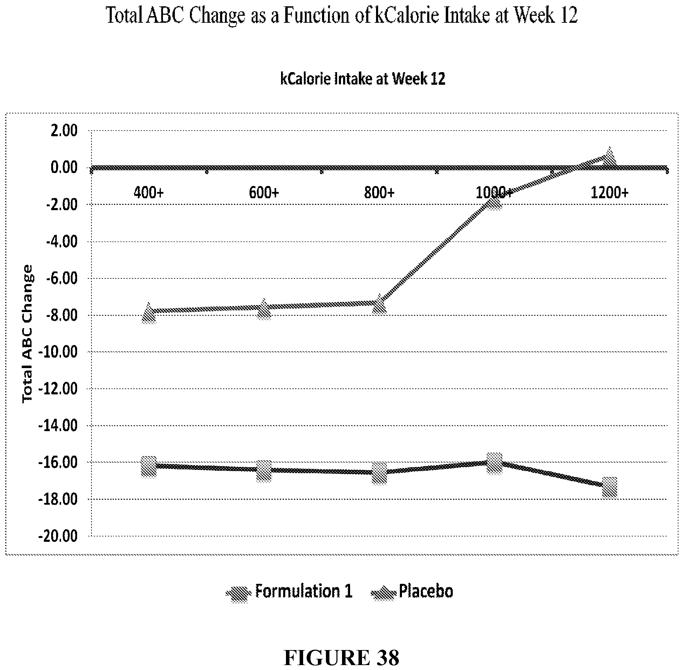

Figure 38 illustrates the total ABC change as a function of kCalorie intake at week 12.

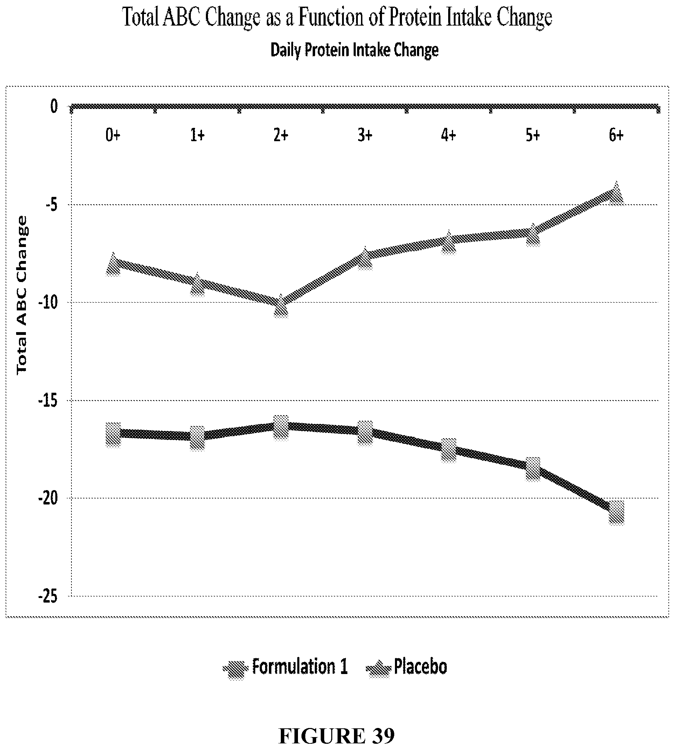

Figure 39 illustrates the total ABC change as a function of protein intake change at week 12.

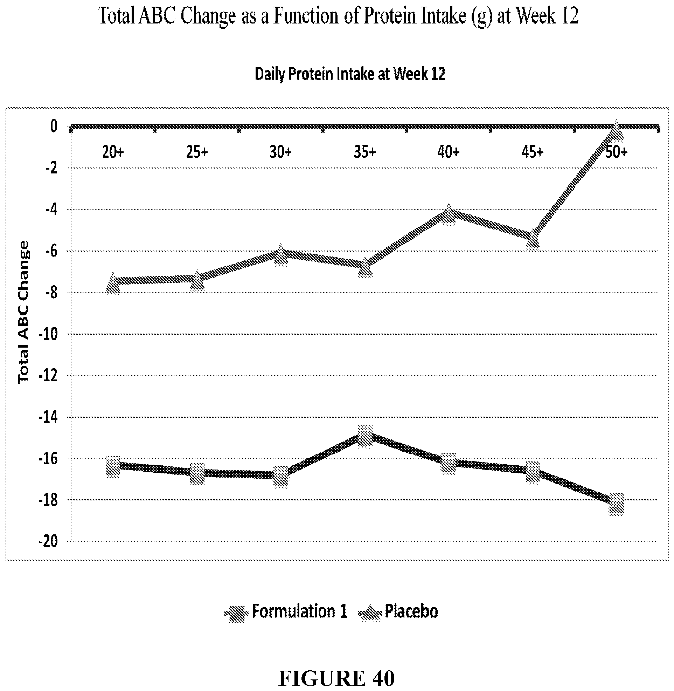

Figure 40 illustrates the total ABC change as a function of protein intake (g) at week 12.

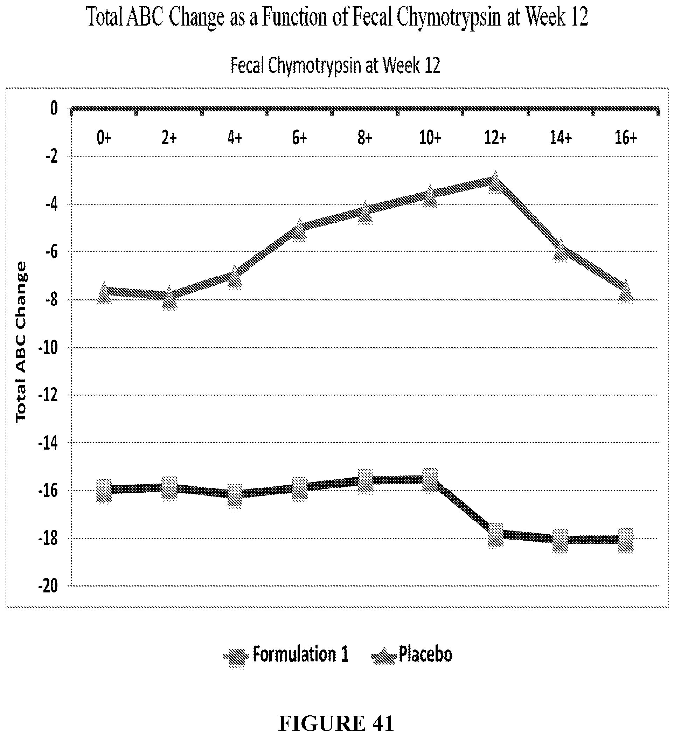

Figure 41 illustrates the total ABC change as a function of fecal chymotrypsin at week 12. There is a significant difference between subjects administered Formulation 1 and subjects administered placebo in their total ABC change regardless of their level of fecal chymotrypsin at week 12. This is suggestive of the fact that it is not just the chymotrypsin levels driving the process of change from the enzyme composition.

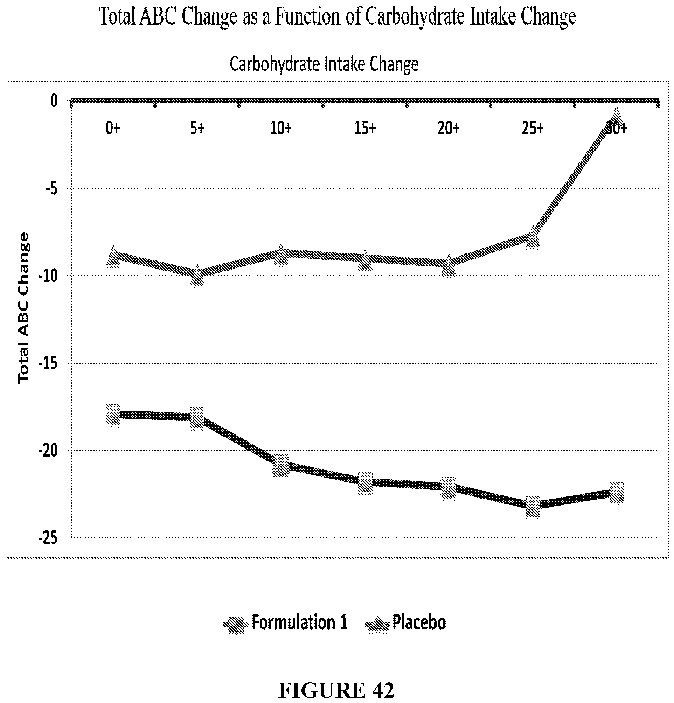

Figure 42 illustrates the total ABC change as a function of carbohydrate intake change at week 12.

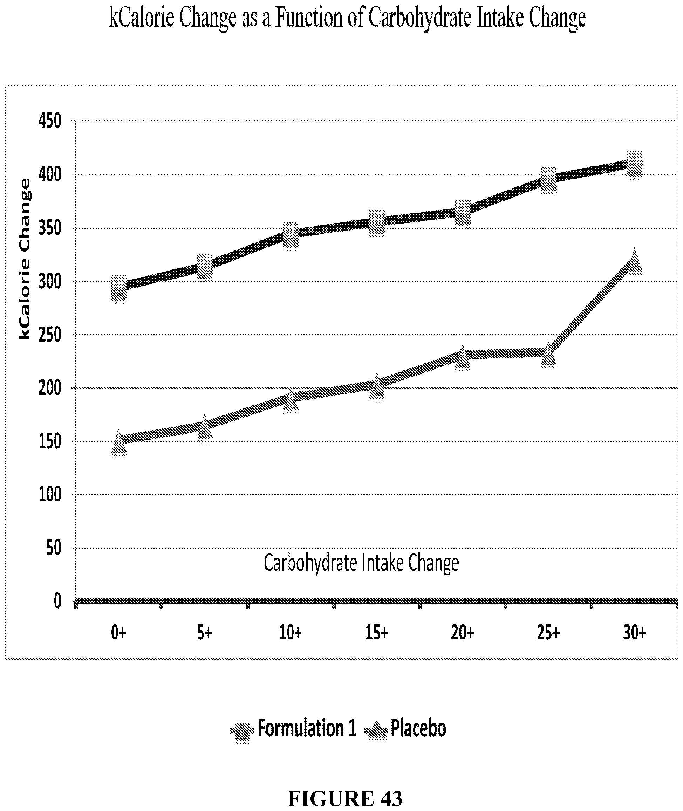

Figure 43 illustrates the kCalorie change as a function of carbohydrate intake change. Subjects treated with Formulation 1 in the trial had a greater change in their kcal consumption compared to the placebo subjects regardless of the level of the increase in change in carbohydrate (CHO) consumption. There continues to be a separation between the Formulation 1 and subjects treated with placebo over the course of treatment.

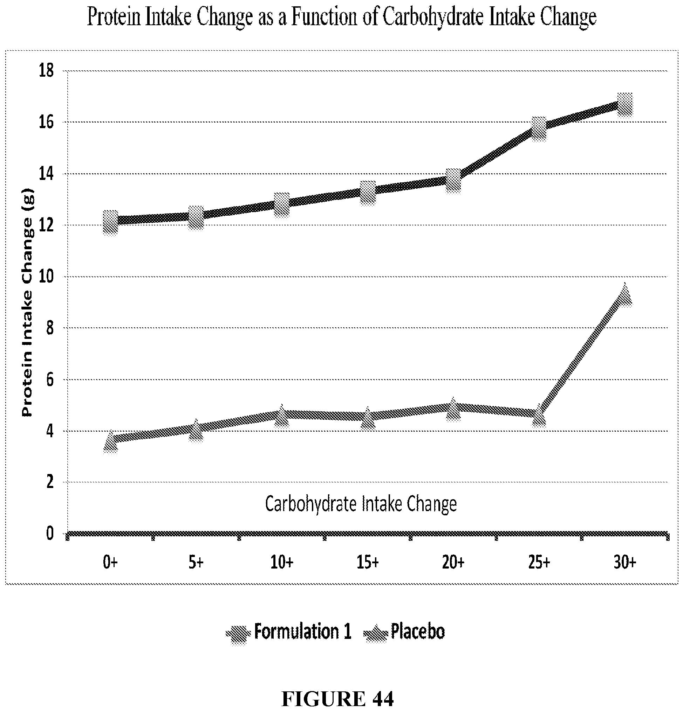

Figure 44 illustrates the protein intake change as a function of carbohydrate intake change. Regardless of the change in protein intake over the course of the trial or the CHO intake, there continues to be a separation between the two groups. Furthermore, regardless of the protein intake change, overall protein intake is greater in subjects treated with Formulation 1 group over subjects treated with placebo, regardless of the change in CHO consumption.

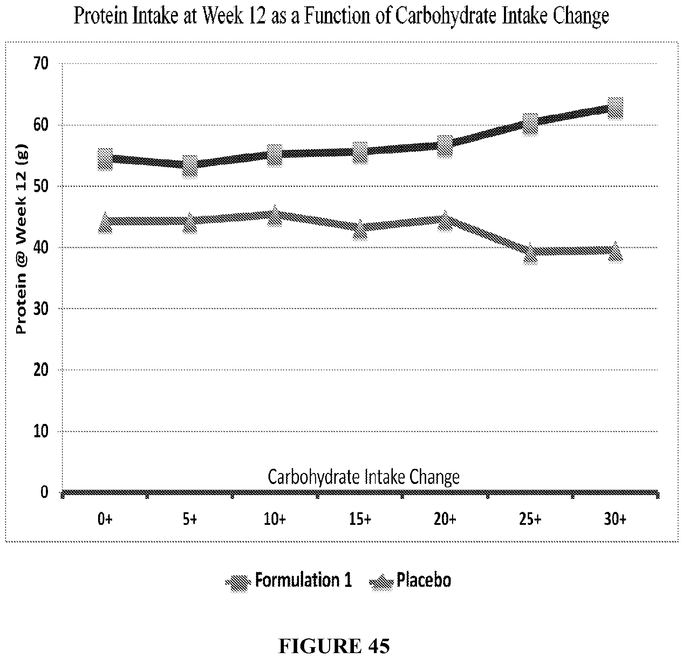

Figure 45 illustrates the protein intake at week 12 as a function of carbohydrate intake change. The absolute amount of protein ingested by subjects at week 12 of the trial was greater in subjects on Formulation 1 compared to subjects receiving placebo. The CHO intake change over the course of the trial does not drive the amount of protein subjects ate at week 12. Once again, it was observed that the changes occurred in subjects on the drug compared to subjects on placebo. Of note is that once 20 g+ change in the CHO ingestion over the trial, there is a greater separation between subjects receiving Formulation 1 versus placebo.

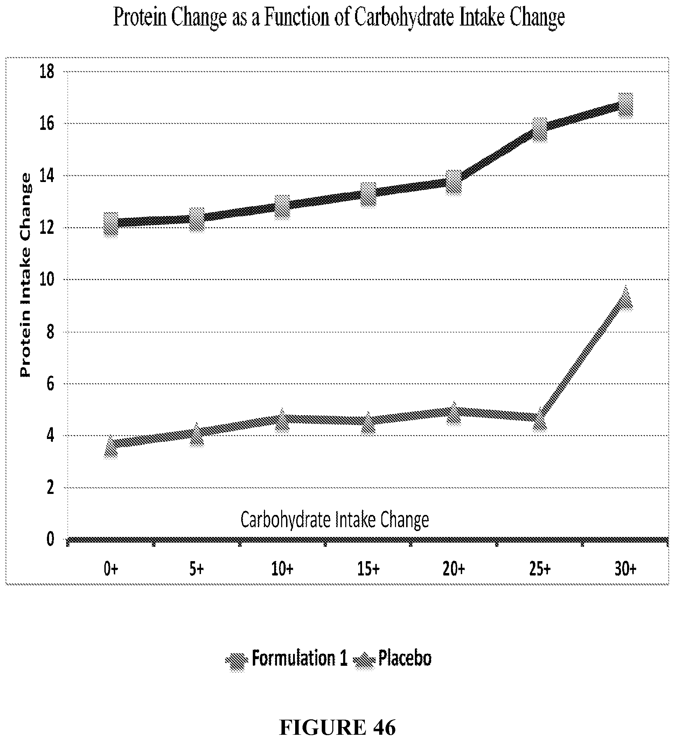

Figure 46 illustrates the protein change as a function of carbohydrate intake change. Protein intake change over the course of the trial was greater in subjects administered Formulation 1 than in subjects administered placebo regardless of the level of CHO intake change over the course of the trial. The increases in protein intake in subjects administered the drug were not driven by the CHO intake.

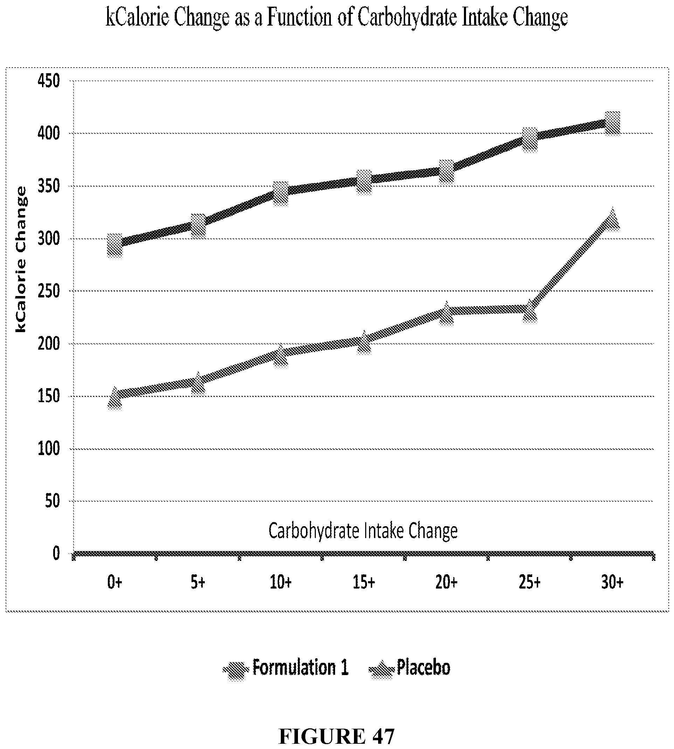

Figure 47 illustrates the kCalorie Change as a Function of Carbohydrate Intake Change. The graph demonstrates that subjects treated with Formulation 1 have a greater kcal change across all increases in CHO ingestion during the trial. Subjects treated with Formulation 1 exhibited increases in CHO ingestion during the trial also had a greater kcal change compared to subjects treated with placebo. This demonstrates that the carbohydrate ingestion during the trial even great changes up to 30+ g/day did not drive the increases in kcal consumption during the 12 weeks of the trial.

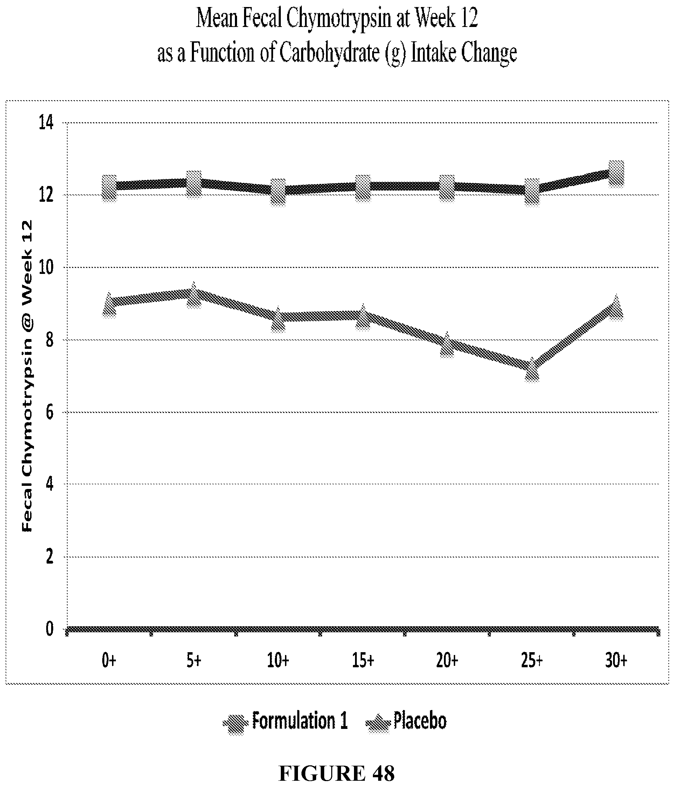

Figure 48 illustrates the mean fecal chymotrypsin at week 12 as a function of carbohydrate (g) intake change: both subjects treated with Formulation 1 and subjects treated with placebos exhibited means similar at baseline. The mean of the absolute fecal chymotrypsin levels at week 12 are close to the "normal threshold" of chymotrypsin (12.6 in frozen stool) regardless of the increase in CHO intake over the course of the trial. The subjects treated with placebo continue to remain abnormal with respect to their level of chymotrypsin at week 12 regardless of increase in the amount of CHO change over the course of the trial.

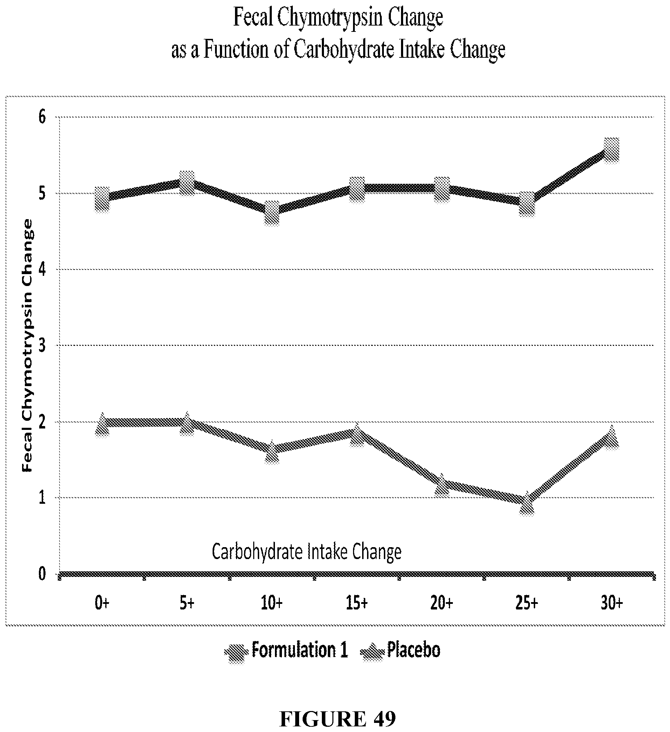

Figure 49 illustrates the fecal chymotrypsin change as a function of carbohydrate intake change: there is a significant difference between subjects who are replaced with Chymotrypsin in the trial (i.e., subjects administered Formulation 1) and subjects administered a placebo. This change occurs regardless of the amount of increase in carbohydrate intake over the course of the 12 week trial. Importantly, food intake, in this case CHO, did not induce natural chymotrypsin into subjects on placebo. It is likely that the carbohydrate changes during the trial as subjects administered Formulation 1 and placebo could have an increase in carbohydrate intake due to differing requirements for calories.

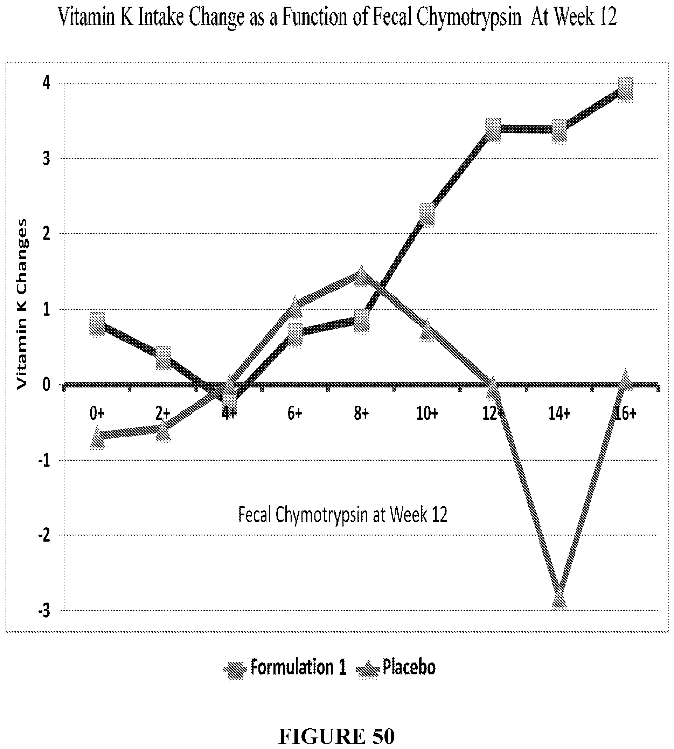

Figure 50 illustrates the vitamin K intake change as a function of fecal chymotrypsin at week 12. The vitamin K intake levels in subjects administered placebo fluctuate between -3 and 1.5 g at week 12. For subjects administered placebo who are in the normal range for fecal chymotrypsin at week 12, their vitamin K intake ranges from 0 to -3 g. For subjects on Formulation 1, the vitamin K intake ranged from -0.1 to 4 g. For subjects in the normal range of FCT at week 12, their vitamin K intake ranged from 3.5 to 4 grams. Starting at 8.0 U/g of activity of chymotrypsin the range went from 0.8 to 4.

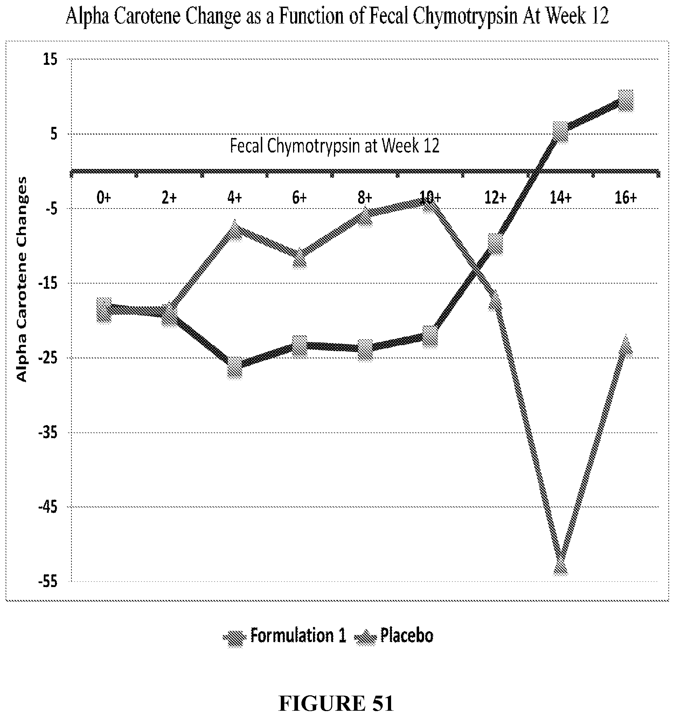

Figure 51 illustrates the alpha carotene change as a function of fecal chymotrypsin at week 12.

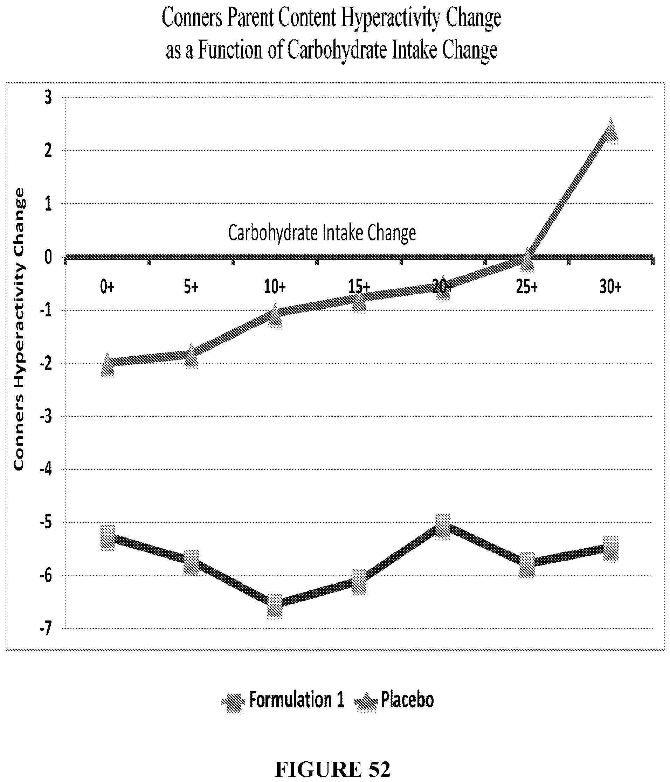

Figure 52 illustrates the Conners parent content hyperactivity change as a function of carbohydrate intake change. The subjects on Formulation 1 had a greater improvement change (negative "-" is improvement) on parent content hyperactivity scores compared to subjects on placebo. Additionally after a 20 g+ change in CHO intake, the change in Formulation 1 subjects remains constant, and the change in placebo subjects demonstrates a worsening of symptoms (positive "+" change on the scale).

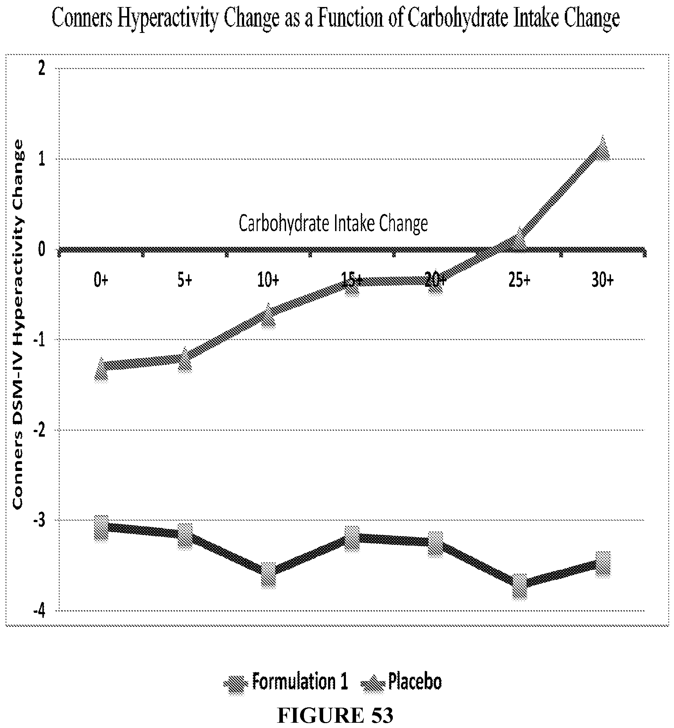

Figure 53 illustrates the Conners hyperactivity change as a function of carbohydrate intake change at week 12.

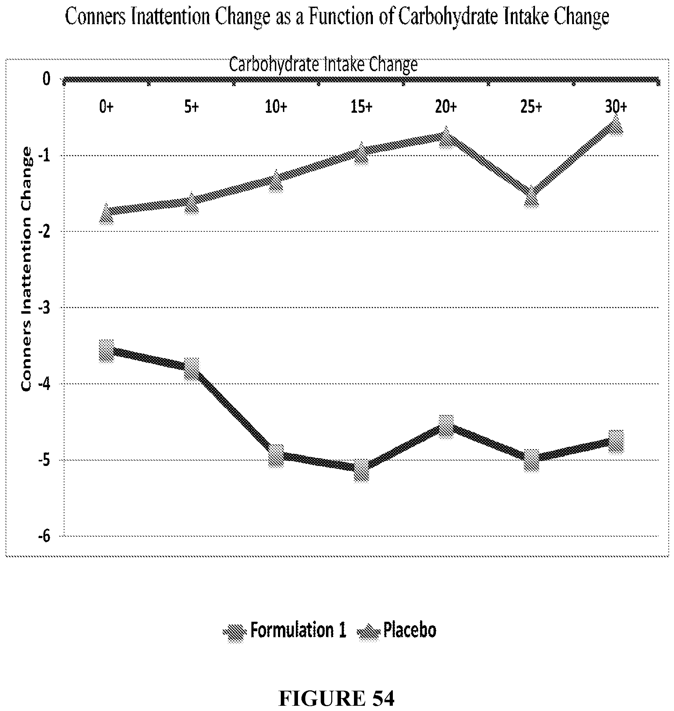

Figure 54 illustrates the Conners inattention change as a function of carbohydrate intake change.

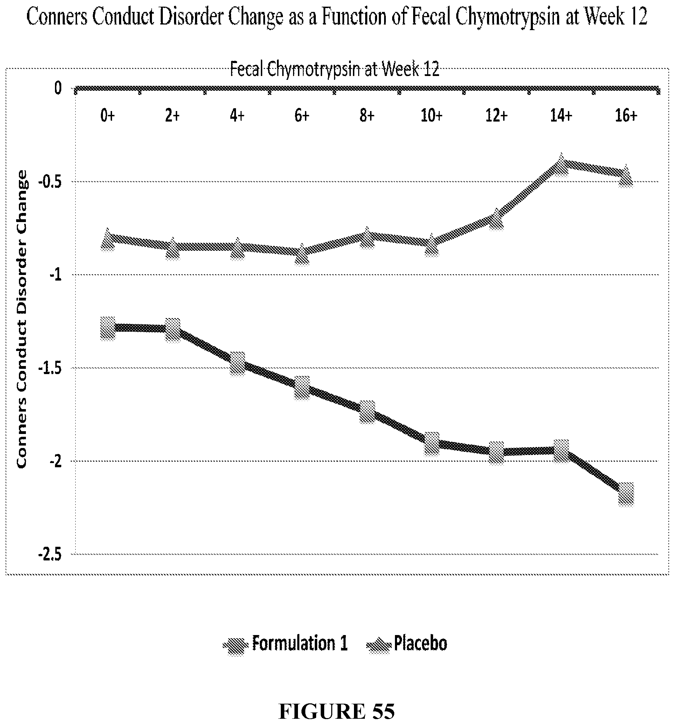

Figure 55 illustrates the Conners conduct disorder change as a function of fecal chymotrypsin at week 12.

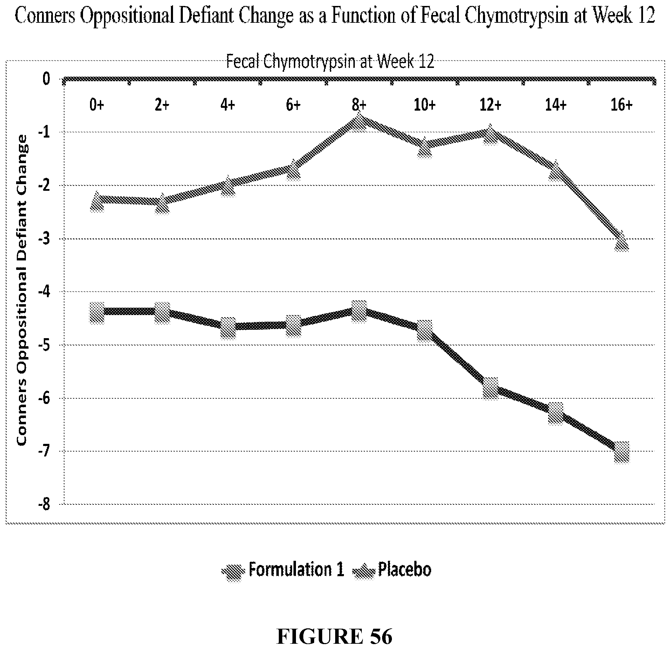

Figure 56 illustrates the Conners oppositional defiant change as a function of fecal chymotrypsin at week 12.

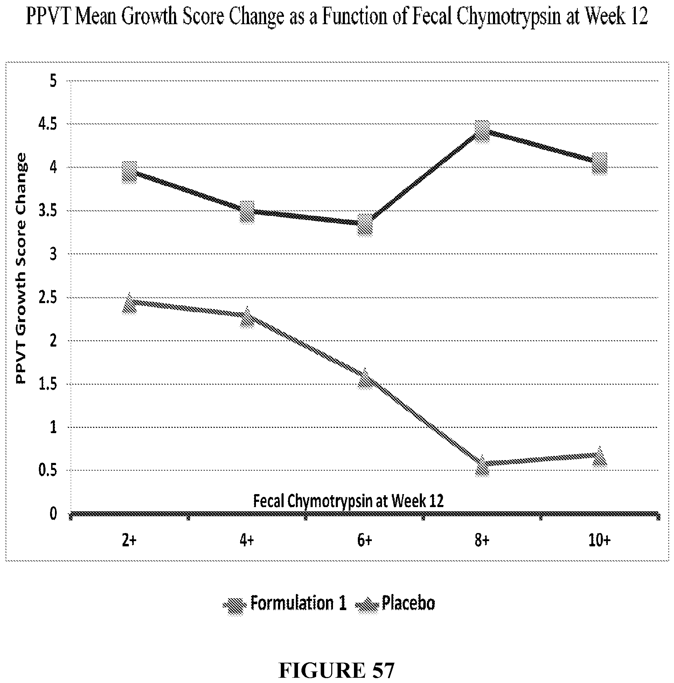

Figure 57 illustrates the PPVT mean growth score change as a function of fecal chymotrypsin at week 12.

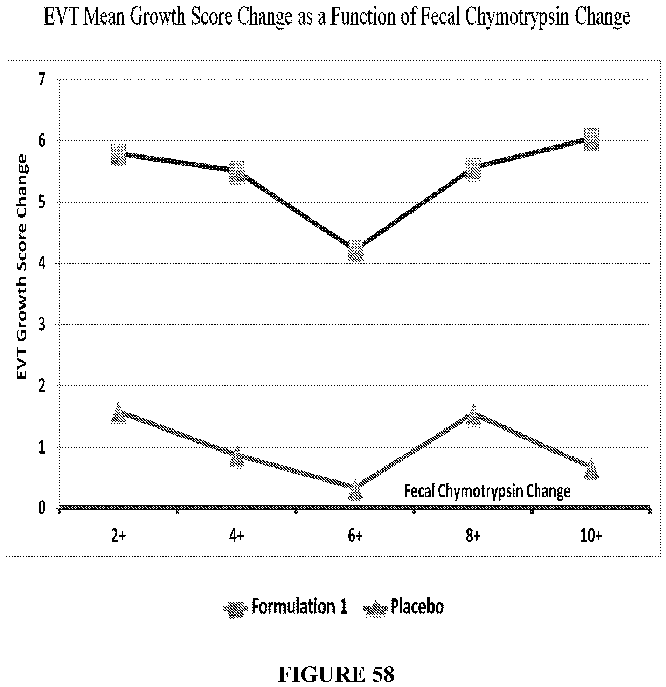

Figure 58 illustrates the EVT mean growth score change as a function of fecal chymotrypsin change at week 12.

### DETAILED DESCRIPTION OF THE INVENTION

Digestive enzymes are produced by the salivary glands, glands in the stomach, the pancreas, and glands in the small intestines. For example, digestive enzymes produced by the pancreas and secreted into the stomach and small intestine aid digestion. Digestive enzymes produced by the pancreas are secreted into the duodenum, or upper segment of the small intestine, where the pH needs to be approximately 5 to 6.6, whereby the enzymes can assist in the digestion of food components, including carbohydrates, lipids, proteins and nucleic acids and other food components. When food is consumed, it is exposed to a highly acidic environment in the stomach (pH 1-2). After the partial digestion of the food occurs in the stomach the food passes into the proximal portion of the small intestine (duodenum). The presence of the food in the duodenum (mechanoreceptors) and the very acidic pH (1-2) signals to the pancreas to secrete the enzymes precursors (pro-enzymes also known as zymogens) along with bicarbonate ions. The food is then exposed to the pancreatic secreted proenzymes along with bicarbonate ions, which turns the pH of the duodenal environment from a pH 1-2 to a pH 5-6.5. The proezymes are then activated from their inactive zymogen state to an active form (for example: trypsinogen is converted to trypsin, and chymotrypsinogen is converted into the active chymotrypsin).

Digestive enzymes have been administered to mammals to treat enzyme deficiencies caused by conditions affecting the pancreas, such as pancreatitis and pancreatic enzyme deficiency or insufficiency. Pancreatic enzymes administered to humans are commonly of porcine origin. Manufacturers of enzyme preparations have also used enteric coatings for compositions in subjects who require administration of lipases. Orally administered enzyme preparations which are comprised of pre activated enzymes are exposed to highly acidic conditions in the stomach, with a pH of around pH 1-2, as well as gastric proteases which denature and degrade the enzymes; especially the lipase portion of the enzyme mixture which is highly sensitive to water air and proteases degradation.

The preparations for lipase delivery have used enteric coatings containing, for example, hypromellose phthalate, dimethicone 1000, and dibutyl phthalate.

Certain methods for coating sensitive bioactive substances have been described. U.S. Patent No. 6,261,613 to Narayanaswamy et al. discloses particles that can contain yeast, coated in a shell of a fat in a beta prime form (*i.e.,* triglyceride crystals having a blocky symmetry). The coating material can further contain emulsifiers such as those found in hydrogenated vegetable oil. However, the coating only allows release of the yeast in a limited temperature range of about 40° C to about 55° C U.S. Pat. No. 6,251,478 to Pacifico et al. discloses certain sensitive substances including certain bioactive compounds encapsulated in a lipid material.

Described herein are coated digestive enzyme preparations, pharmaceutical compositions and enzyme delivery systems comprising coated digestive enzyme preparations, which are useful in the treatment of subjects with autism, ADD, ADHD, other neurological and behavioral diseases or conditions.

Autism or autistic disorder is the most common condition in a group of developmental disorders known as the autistic spectrum disorders (ASDs). Autism is characterized by impaired social interaction, problems with verbal and nonverbal communication, and unusual, repetitive, or severely limited activities and interests. Other ASDs include Asperger syndrome, Rett syndrome, childhood disintegrative disorder, and pervasive developmental disorder not otherwise specified (usually referred to as PDD-NOS). It has been estimated that 1 in 88 subjects in the US have some form of autism. The numbers worldwide vary from 1 in 32 to 2-3 in 1000. The compositions described herein are able to treat both the core and the non-core symptoms of autism.

The non-core symptoms of autism include the following: seizure disorders, sensory integration disorders, gastrointestinal disorders, proprioceptive disorders such as balance and other issues.

Attention deficit-hyperactivity disorder (ADHD) is a neurobehavioral disorder that affects 3-5 percent of all subjects in the US. A similar incidence can be found worldwide. ADHD interferes with a person's ability to stay on a task and to exercise age-appropriate inhibition (cognitive alone or both cognitive and behavioral). Some of the diagnostic signs of ADHD include failure to listen to instructions, inability to organize oneself and school work, fidgeting with hands and feet, talking too much, abandoning projects, chores and leaving homework unfinished, and having trouble paying attention to and responding to details. Dysgraphia and disinhibition are further symptoms found in ADHD. There are several types of ADHD: a predominantly inattentive subtype, a predominantly hyperactive-impulsive subtype, and a combined subtype. ADHD is usually diagnosed in childhood, although the condition can continue into the adult years. ADHD is presently comprised of what was at one time known as Attention Deficit Disorder (ADD) and Attention Deficit hyperactivity disorder (ADHD).

**Criteria of Neurological or Mental Health DisordersThe** American Psychiatric Association's Diagnostic and Statistical Manual-IV, Text Revision (DSM-IV-TR) 1 provides standardized criteria to help diagnose Autistic Spectrum Disorders (ASDs). Psychiatric Diagnoses are categorized by the Diagnostic and Statistical Manual of Mental Disorders, 4th. Edition. Better known as the DSM-IV, the manual is published by the American Psychiatric Association and covers all mental health disorders for both subjects and adults. It also lists known causes of these disorders, statistics in terms of gender, age at onset, and prognosis as well as some research concerning the optimal treatment approaches. Mental Health Professionals use this manual when working with subjects in order to better understand their illness and potential treatment and to help 3rd party payers (*e.g.,* insurance) understand the needs of a subject. The book is typically considered the `bible' for any professional who makes psychiatric diagnoses in the United States and many other countries. Much of the diagnostic information on these pages is gathered from the DSM IV.

The DSM IV is published by the American Psychiatric Association. Much of the information from the Psychiatric Disorders pages is summarized from the pages of this text. Should any questions arise concerning incongruencies or inaccurate information, you should always default to the DSM as the ultimate guide to mental disorders. The DSM uses a multiaxial or multidimensional approach to diagnosing because rarely do other factors in a person's life not impact their mental health. Pervasive development disorders can be classified under the DSM-IV-TR classification as an autistic disorder, Asperger's Disorder, PDD-NOS (including atypical autism), Rett's Disorder and Childhood Disintegrative Disorder. The mental health community is presently poised to convert their classifications of all mental health diagnostic criteria from the DSM-IV to the DSM-V. Both are outlined below. The DSM-V criteria are inclusive of PDD-NOS and Asperger's disorder, and also include sensory integration dysfunction as a part for the diagnostic criteria.

### DSM-IV Diagnostic Criteria for Autism Spectrum Disorder

A subject must meet criteria A, B, C, and D:
A. Persistent deficits in social communication and social interaction across contexts, not accounted for by general developmental delays, and manifest by all 3 of the following: **A1)** Deficits in social-emotional reciprocity; ranging from abnormal social approach and failure of normal back and forth conversation through reduced sharing of interests, emotions and affect and response to total lack of initiation of social interaction; **A2)** Deficits in nonverbal communicative behaviors used for social interaction; ranging from poorly integrated- verbal and non-verbal communication, through abnormalities in eye contact and body-language, or deficits in understanding and use of non-verbal communication, to total lack of facial expression or gestures; and **A3)** Deficits in developing and maintaining relationships, appropriate to developmental level (beyond those with caregivers); ranging from difficulties in sharing imaginative play and in making friends to an apparent absence of interest in people.
**B.** Restricted, repetitive patterns of behavior, interest, or activities as manifested by at least two of the following: **B1)** Stereotyped or repetitive speech, motor movements, or use of objects (such as simple motor stereotypes, echolalia, repetitive use of objects or idiosyncratic phrases). **B2)** Excessive adherence to routines, ritualized patterns of verbal or nonverbal behavior, or excessive resistance to change (such as motoric rituals, insistence on same route or food, repetitive questioning or extreme distress at small changes). **B3)** Highly restricted, fixated interests that are abnormal in intensity of focus (*e.g.,* strong attachment to or preoccupation with unusual objects, excessively circumscribed or perseverative interests). **B4)** Hyper- or hypo-reactivity to sensory input or unusual interest in sensory aspects of environment (*e.g.,* apparent indifference to pain/heat/cold, adverse response to specific sounds or textures, excessive smelling or touching of objects, fascination with lights or spinning objects).
**C.** Symptoms must be present in early childhood (but may not become fully manifest until social demands exceed limited capacities).
**D.** Symptoms together limit and impair everyday functioning.

### DSM-IV Diagnostic Criteria for Pervasive Developmental Disorder Not

### Otherwise Specified (Including Atypical Autism)

This category should be used when there is a severe and pervasive impairment in the development of reciprocal social interaction associated with impairment in either verbal or nonverbal communication skills or with the presence of stereotyped behavior, interests, and activities, but the criteria are not met for a specific Pervasive Developmental Disorder, Schizophrenia, Schizotypal Personality Disorder, or Avoidant Personality Disorder. For example, this category includes "atypical autism" - presentations that do not meet the criteria for Autistic Disorder because of late age at onset, atypical symptomatology, or sub-threshold symptomatology, or all of these.

*DSM-IV Diagnostic Criteria for Rett's Disorder***A***.* All of the following: **A1)** apparently normal prenatal and perinatal development; **A2)** apparently normal psychomotor development through the first 5 months after birth; and **A3)** normal head circumference at birth.

**B.** Onset of all of the following after the period of normal development: **B1)** deceleration of head growth between ages 5 and 48 months; **B2)** loss of previously acquired purposeful hand skills between 5 and 30 months with the subsequent development of stereotyped hand movements (*e.g.,* hand-wringing or hand washing); **B3)** loss of social engagement early in the course ( although often social interaction develops later); **B4)** appearance of poorly coordinated gait or trunk movements; and **B5)** severely impaired expressive and receptive language development with severe psychomotor retardation.

### DSM-IV Diagnostic Criteria for Childhood Disintegrative Disorder

**A.** Apparently normal development for at least the first 2 years after birth as manifested by the presence of age-appropriate verbal and nonverbal communication, social relationships, play, and adaptive behavior.
**B.** Clinically significant loss of previously acquired skills (before age 10 years) in at least two of the following areas: **B1)** expressive or receptive language; **B2)** social skills or adaptive behavior; **B3)** bowel or bladder control; **B4)** play; and **B5)** motor skills.
**C.** Abnormalities of functioning in at least two of the following areas: **C1**) qualitative impairment in social interaction (*e.g.,* impairment in nonverbal behaviors, failure to develop peer relationships, lack of social or emotional reciprocity); **C2)** qualitative impairments in communication (*e.g.,* delay or lack of spoken language, inability to initiate or sustain a conversation, stereotyped and repetitive use of language, lack of varied make-believe play); and **C3)** restricted, repetitive, and stereotyped patterns of behavior, interest, and activities, including motor stereotypes and mannerisms.
**D.** The disturbance is not better accounted for by another specific Pervasive Developmental Disorder or by Schizophrenia.

### Diagnostic Criteria for Asperger's Disorder

**A.** Qualitative impairment in social interaction, as manifested by at least two of the following: **A1)** marked impairment in the use of multiple nonverbal behaviors such as eye-to eye gaze, facial expression, body postures, and gestures to regulate social interaction; **A2)** failure to develop peer relationships appropriate to developmental level; **A3)** a lack of spontaneous seeking to share enjoyment, interests, or achievements with other people (*e.g.,* by a lack of showing, bringing, or pointing out objects of interest to other people); and **A4)** lack of social or emotional reciprocity.
**B.** Restricted repetitive and stereotyped patterns of behavior, interests and activities, as manifested by at least one of the following: **B1)** encompassing preoccupation with one or more stereotyped and restricted patterns of interest that is abnormal either in intensity or focus; **B2)** apparently inflexible adherence to specific, nonfunctional routines or rituals; **B3)** stereotyped and repetitive motor mannerisms (*e.g.,* hand or finger flapping or twisting, or complex whole-body movements); and **B4)** persistent preoccupation with parts of objects.
**C.** The disturbance causes clinically significant impairment in social, occupational, or other important areas of functioning.
**D.** There is no clinically significant general delay in language (*e.g.,* single words used by age 2 years, communicative phrases used by age 3 years).
**E.** There is no clinically significant delay in cognitive development or in the development of age-appropriate self-help skills, adaptive behavior (other than in social interaction), and curiosity about the environment in childhood.
**F.** Criteria are not met for another specific Pervasive Developmental Disorder or Schizophrenia.

Additional tests which may be used to assess subjects include, but are not limited to: EVT, PPVT, ADI-R, DSM-IV, SCQ, Block food screener, ABC checklist, and the Conners test.

*DSM-V Diagnostic Criteria for Autistic Disorder* **A.** Six or more items from (1), (2), and (3), with at least two from (1), and one each from (2) and (3): **A1)** qualitative impairment in social interaction, as manifested by at least two of the following: **A1a**) marked impairment in the use of multiple nonverbal behaviors such as eye-to-eye gaze, facial expression, body postures, and gestures to regulate social interaction; **A1b)** failure to develop peer relationships appropriate to developmental level; **A1c)** a lack of spontaneous seeking to share enjoyment, interests, or achievements with other people (*e.g.,* by a lack of showing, bringing, or pointing out objects of interest); and **A1d)** lack of social or emotional reciprocity.

**A2)** qualitative impairments in communication as manifested by at least one of the following: **A2a)** delay in, or total lack of, the development of spoken language (not accompanied by an attempt to compensate through alternative modes of communication such as gesture or mime); **A2b)** in subjects with adequate speech, marked impairment in the ability to initiate or sustain a conversation with others; **A2c)** stereotyped and repetitive use of language or idiosyncratic language; and **A2d)** lack of varied, spontaneous make-believe play or social imitative play appropriate to developmental level.

**A3)** restricted repetitive and stereotyped patterns of behavior, interests, and activities, as manifested by at least one of the following: **A3a)** encompassing preoccupation with one or more stereotyped and restricted patterns of interest that is abnormal either in intensity or focus; **A3b)** apparently inflexible adherence to specific, nonfunctional routines or rituals; **A3c)** stereotyped and repetitive motor manners (*e.g.,* hand or finger flapping or twisting, or complex whole-body movements); and **A3d)** persistent preoccupation with parts of objects.

**B.** Delays or abnormal functioning in at least one of the following areas, with onset prior to age 3 years: (1) social interaction, (2) language as used in social communication, or (3) symbolic or imaginative play.

**C.** The disturbance is not better accounted for by Rett's Disorder or Childhood Disintegrative Disorder.

Further consideration with respect to levels of severity of ASD can be found in the following table:

| **Severity Level for ASD** | **Social Communication** | **Restricted Interests and Repetitive behaviors** |
|---|---|---|
| Level 3 | Severe deficits in verbal and non-verbal communication skills cause severe impairments in functioning; very limited initiation of social interactions and minimal response to social overtures from others. | Preoccupations, fixated rituals and/or repetitive behaviors markedly interfere with functioning in all spheres. Marked distress when rituals or routines are interrupted; very difficult to redirect from fixated interest or returns to it quickly. |
| Requiring very substantial support | | |
| Level 2 | Marked deficits in verbal and non-verbal social communication skills; social impairments apparent even with supports in place; limited initiation of social interactions and reduced or abnormal response to social overtures from others. | RRBs and/or other preoccupations or fixated interests appear frequently enough to be obvious to the casual observers and interfere with functioning in a variety of contexts. |
| Requiring substantial support | | |
| | | Distress or frustration is apparent when RRBs are interrupted; difficult to redirect from fixated interest. |
| Level 1 | Without supports in place, deficits in social communication cause noticeable impairments. | Rituals and repetitive behaviors (RRBs) cause significant interference with functioning in one or more contexts. Resists attempts by others to interrupt RRBs or to be redirected from fixated interest. |
| Requiring support | | |
| | Has difficulty initiating social interactions and demonstrates clear examples of atypical or unsuccessful responses to social overtures of others. | |
| | May appear to have decreased interest in social interactions. | |

### Diagnostic Testing and Assessment of Autism Severity

Diagnostic Testing Assessment tools can be utilized to diagnose the presence of autism as well as the severity of autism. The two most comprehensive testing methods are the ADOS and the Autism Diagnostic Interview-Revised (ADI-R). Both are comprehensive and considered a specific diagnostic tool for the determination of the severity of autism. ADI-R has the added benefit of necessitating training for those who administer the test. That training is standardized, which allows for the standardization across multiple clinical sites for example in a clinical trial.

### Autism Diagnostic Interview-Revised (ADI-R)

The ADI-R test may be used in the methods described herein to assess autism in subjects and adults and has been described by Anne Le Couteur, Catherine Lord, and Michael Rutter, (Western Psychological Services, 2003).

The Autism Diagnostic Interview-Revised (ADI-R) is a clinical diagnostic instrument for assessing autism in subjects and adults. The ADI-R provides a diagnostic algorithm for autism as described in both the ICD-10 and DSM-IV. The instrument focuses on behavior in three main areas: qualities of reciprocal social interaction; communication and language; and restricted and repetitive, stereotyped interests and behaviors. The ADI-R is appropriate for subjects and adults with mental ages from about 18 months and above.

The ADI-R (Lord *et al.,* 1994; Rutter, LeCouteur, et al., 2003) is a comprehensive parent interview that probes for symptoms of autism. It is administered by a trained clinician using a semi-structured interview format. The research or long version of the ADI-R requires approximately 3 hours (hr) to administer and score.

The ADI-R elicits information from the parent on current behavior and developmental history. It is closely linked to the diagnostic criteria set forth in the DSM-IV-TR and International Classification of Diseases-10. The ADI-R is a very helpful tool, but it does have some limitations. It is not sensitive to differences among subjects with mental ages below 20 months or IQs below 20 (Cox *et al.,* 1999; Lord, 1995) and is not advised for use with such subjects. In particular, its sensitivity to the milder ASDs (AS and PDDNOS) is low at age 2, but good by 3.5 years. It is not designed to assess change through repeated administrations and is best suited to confirm the initial diagnosis of autism (Arnold *et al.,* 2000). Finally, and perhaps most important, it is labor intensive and requires more administration time than many practitioners may be able to allot.

The ADI-R is a standardized, semi-structured clinical review for caregivers of subjects and adults. The interview contains 93 items and focuses on behaviors in three content areas or domains: quality of social interaction (*e.g.,* emotional sharing, offering and seeking comfort, social smiling and responding to other subjects); communication and language (*e.g.,* stereotyped utterances, pronoun reversal, social usage of language); and repetitive, restricted and stereotyped interests and behavior (*e.g.,* unusual preoccupations, hand and finger mannerisms, unusual sensory interests). The measure also includes other items relevant for treatment planning, such as self-injury and over-activity. Responses are scored by the clinician based on the caregiver's description of the child's behavior. Questions are organized around content area, and definitions of all behavioral items are provided. Within the area of communication, for example, "Delay or total lack of language not compensated by gesture" is further broken down into specific behavioral items: pointing to express interest, conventional gestures, head nodding, and head shaking. Similarly, within the area of reciprocal social interaction, lack of socio-emotional reciprocity and modulation to context include the following behaviors: use of other's body, offering comfort, inappropriate facial expressions, quality of social overtures, and appropriateness of social response.

The interview starts with an introductory question followed by questions about a subject's early development. The next 41 questions cover verbal and nonverbal communication. Questions 50 through 66 ask about social development and play. The next 13 questions deal with interests and behaviors. The final 14 questions ask about "general behavior," including questions about memory skills, motor skills, over-activity and fainting.

The ADI-R interview generates scores in each of the three content areas (i.e., communication and language, social interaction, and restricted, repetitive behaviors). Elevated scores indicate problematic behavior in a particular area. Scores are based on the clinician's judgment following the caregiver's report of the child's behavior and development. For each item, the clinician gives a score ranging from 0 to 3. A score of 0 is given when "behavior of the type specified in the coding is not present"; a score of 1 is given when "behavior of the type specified is present in an abnormal form, but not sufficiently severe or frequent to meet the criteria for a 2"; a score of 2 indicates "definite abnormal behavior" meeting the criteria specified; and a score of 3 is reserved for "extreme severity" of the specified behavior. (The interviewers of the measure recode 3 as a 2 in computing the algorithm.) There are also scores of 7 ("definite abnormality in the general area of the coding, but not of the type specified"), 8 ("not applicable"), and 9 ("not known or not asked") given under certain circumstances, which all are converted to 0 in computing the algorithm.

This interviewer-based instrument requires substantial training in administration and scoring. A highly trained clinician can administer the ADI-R to the parent of a 3- or 4-year old suspected of autism in approximately 90 minutes. The interview may take somewhat longer when administered to parents of older subjects or adults. Training workshops are available in the United States as well as internationally. The ADI-R and related materials are available from Western Psychological Services.

Inter-rater and test-retest reliability, as well as internal validity, have been demonstrated for the ADI-R. A detailed bibliography (with abstracts) describing the psychometric properties of the ADI-R can be found on the University of Michigan Autism Communication and Disorders Center website.

### The Social Communication Questionnaire

The Social Communication Questionnaire (SCQ; Rutter, Bailey, & Lord, 2003), previously known as the Autism Screening Questionnaire (ASQ), was initially designed as a companion screening measure for the Autism Diagnostic Interview-Revised (ADI-R; Rutter, Le Couteur & Lord). The SCQ is a parent/caregiver dimensional measure of ASD symptomatology appropriate for subjects of any chronological age older than four years. It can be completed by the informant in less than 10 minutes.

The Social Communication Questionnaire (is a parent-report questionnaire based on the ADI-R. It contains many of the same questions included on the ADI-R algorithm, presented in a briefer, yes/no format that parents can complete on their own.

The primary standardization data for the SCQ was obtained from a sample of 200 subjects who had participated in previous studies of ASD. The SCQ is available in two forms: Lifetime and Current; each with 40 questions presented in a yes or no format. Scores on the questionnaire provide an index of symptom severity and indicate the likelihood that a subject has an ASD. Questions include items in the reciprocal social interaction domain (*e.g.,* "Does she/he have any particular friends or best friend?"), the communication domain (*e.g.,* "Can you have a to and fro 'conversation' with him/her that involves administered turns or building on what you have said?") and the restricted, repetitive, and stereotyped patterns of behavior domain (*e.g.,* Has she/he ever seemed to be more interested in parts of a toy or an object [*e.g.,* spinning the wheels of a car], rather than using the object as intended?").

Compared to other screening measures, the SCQ has consistently demonstrated its effectiveness in predicting ASD versus non-ASD status in multiple studies. The scale has been found to have good discriminant validity and utility as an efficient screener for at-risk groups of school-age subjects. A threshold raw score of >15 is recommended to minimize the risk of false negatives and indicate the need for a comprehensive evaluation. Comparing autism to other diagnoses (excluding mental retardation), this threshold score resulted in a sensitivity value of .96 and a specificity value of .80 in a large population of subjects with autism and other developmental disorders. The positive predictive value was .93 with this cutoff. The authors recommend using different cut-off scores for different purposes and populations (*e.g.,* a cut-off of 22 when differentiating autism from other ASDs and a cut-off of 15 when differentiating ASD from non-ASD). Several studies (Allen *et al.,* 2007; Eaves *et al.,* 2006) have suggested that a cut-off of 11 may be more clinically useful (Norris & Lecavalier, 2010).

The SCQ is one of the most researched of the ASD-specific evaluation tools and can be recommended for screening and as part of comprehensive developmental assessment for ASD (Norris & Lecavalier, 2010; Wilkinson, 2010, 2011). The SCQ is an efficient screening instrument for identifying subjects with possible ASD for a more in-depth assessment. For clinical purposes, practitioners might consider a multistage assessment beginning with the SCQ, followed by a comprehensive developmental evaluation (Wilkinson, 2011). However, cut-off scores may need to be adjusted depending on the population in which it is used. The evidence also indicates that although the SCQ is appropriate for a wide age range, it is less effective when used with younger populations (*e.g.,* subjects two to three years). It was designed for subjects above the age of four years, and seems to perform best with subjects over seven years of age.

Its agreement with the more labor-intensive ADI-R on diagnostic categorization is high (Bishop & Norbury, 2002), and it is thus an efficient way to obtain diagnostic information or screen for autistic symptoms. There are two versions available: one for current behavior and one for lifetime behavior. The lifetime version is helpful for screening and diagnostic purposes, whereas the current version is more appropriate for assessment of change over time in a subject, A cutoff score of 15 differentiates between ASD and other diagnoses for subjects ages 4 years and older, whereas a cutoff of 22 discriminates subjects with autistic disorder from those with other ASDs (PDDNOS or AS). Using these cutoffs, sensitivity of 0.85 and specificity of 0.75 have been reported in a large sample of subjects and adults with autism and other developmental disorders (Berumentetal, 1999).

DSM-IV and DSM-V criteria ADI-R, SCQ and ADOS, for example, are measures of the presence and, in some cases, the severity of autism. Those which look at severity such as ADI-R, ADOS and SCQ which measure severity cannot be utilized as outcome measures for test and retest of subjects with autism when administered an intervention. Their use and validation are strictly relegated to diagnosis. There is no test re-test validation, and they are not designed to examine change seen in subjects with autism.

### Behavioral assessments

Behavioral testing can be utilized to examine changes in subjects with autism who are administered an intervention. The pre-test post-test format is not able to be utilized with the diagnostic measures, but can be utilized with many of the behavioral assessments.

### Aberrant Behavior Checklist

The Aberrant Behavior Checklist (ABC) scale is a standardized set of questions used for assessing subjects and can be found, for example, published in the 1994 Slosson Educational Publications, Inc. (Aman *et al.*) and the assessment serves as the standard for assessing subjects. Behavioral attributes are rated as follows: 0 = not at all a problem; 1 = the behavior is a problem, but slight in decree; 2 = the problem is moderately serious; or 3 = the problem is severe in degree.

The factors (subscales) of the ABC scale are as follows: (I) irritability, agitation, crying; (II) lethargy, social withdrawal; (III) stereotypic behavior; (IV) hyperactivity, noncompliance; and (V) inappropriate speech. A series of questions is asked with respect to each of the scales and each is rated 0-3.

Three scales have significant number of data points and validated: irritability / agitation (non-core) primary; lethargy / social withdrawal (core) secondary; and hyperactivity (non-core, high morbidity). Two minor scales are: stereotypic behavior and inappropriate speech. The numbers of questions on each subscale are as follows: 1) irritability, agitation, crying (15 items); 2) lethargy, social withdrawal (16 items); 3) stereotypic behavior (7 items); 4) hyperactivity, non-compliance (16 items); and 5) inappropriate speech (4 items)

The questions on the ABC test are as follows:

| | | | | | |
|---|---|---|---|---|---|
| 1. | Excessively active at home, school, work or elsewhere | 0 | 1 | 2 | 3 |
| 2. | Injures self on purpose | 0 | 1 | 2 | 3 |
| 3. | Listless, sluggish, inactive | 0 | 1 | 2 | 3 |
| 4. | Aggressive to other subjects or adults (verbally or physically) | 0 | 1 | 2 | 3 |
| 5. | Seeks isolation from others | 0 | 1 | 2 | 3 |
| 6. | Meaningless, recurring body movements | 0 | 1 | 2 | 3 |
| 7. | Boisterous (inappropriately noisy and rough) | 0 | 1 | 2 | 3 |
| 8. | Screams inappropriately | 0 | 1 | 2 | 3 |
| 9. | Talks excessively | 0 | 1 | 2 | 3 |
| 10. | Temper tantrums/outbursts | 0 | 1 | 2 | 3 |
| 11. | Stereotyped behavior; abnormal, repetitive movements | 0 | 1 | 2 | 3 |
| 12. | Preoccupied; stares into space | 0 | 1 | 2 | 3 |
| 13. | Impulsive (acts without thinking) | 0 | 1 | 2 | 3 |
| 14. | Irritable and whiny | 0 | 1 | 2 | 3 |
| 15. | Restless, unable to sit still | 0 | 1 | 2 | 3 |
| 16. | Withdrawn; prefers solitary activities | 0 | 1 | 2 | 3 |
| 17. | Odd, bizarre in behavior | 0 | 1 | 2 | 3 |
| 18. | Disobedient; difficult to control | 0 | 1 | 2 | 3 |
| 19. | Yells at inappropriate times | 0 | 1 | 2 | 3 |
| 20. | Fixed facial expression/ lacks emotional responsiveness | 0 | 1 | 2 | 3 |
| 21. | Disturbs others | 0 | 1 | 2 | 3 |
| 22. | Repetitive speech | 0 | 1 | 2 | 3 |
| 23. | Does nothing but sit and watch others | 0 | 1 | 2 | 3 |
| 24. | Uncooperative | 0 | 1 | 2 | 3 |
| 25. | Depressed mood | 0 | 1 | 2 | 3 |
| 26. | Resists any form of physical contact | 0 | 1 | 2 | 3 |
| 27, | Moves or rolls head back and forth repetitively | 0 | 1 | 2 | 3 |
| 28. | Does not pay attention to instructions | 0 | 1 | 2 | 3 |
| 29. | Demands must be met immediately | 0 | 1 | 2 | 3 |
| 30. | Isolates himself/herself from other subjects or adults | 0 | 1 | 2 | 3 |
| 31. | Disrupts group activities | 0 | 1 | 2 | 3 |
| 32. | Sits or stands in one position for a long time | 0 | 1 | 2 | 3 |
| 33. | Talks to self loudly | 0 | 1 | 2 | 3 |
| 34. | Cries over minor annoyances and hurts | 0 | 1 | 2 | 3 |
| 35. | Repetitive hand, body, or head movements | 0 | 1 | 2 | 3 |
| 36. | Mood changes quickly | 0 | 1 | 2 | 3 |
| 37. | Unresponsive to structured activities (does not react) | 0 | 1 | 2 | 3 |
| 38. | Does not stay in seat (e.g., during lesson or training periods, meals, etc.) | 0 | 1 | 2 | 3 |
| 39. | Will not sit still for any length of time | 0 | 1 | 2 | 3 |
| 40. | Is difficult to reach, contact, or get through to | 0 | 1 | 2 | 3 |
| 41. | Cries and screams inappropriately | 0 | 1 | 2 | 3 |
| 42. | Prefers to be alone | 0 | 1 | 2 | 3 |
| 43. | Does not try to communicate by words or gestures | 0 | 1 | 2 | 3 |
| 44. | Easily distractible | 0 | 1 | 2 | 3 |
| 45. | Waves or shakes the extremities repeatedly | 0 | 1 | 2 | 3 |
| 46. | Repeats a word or phrase over and over | 0 | I | 2 | 3 |
| 47. | Stamps feet or bangs objects or slams doors | 0 | 1 | 2 | 3 |
| 48. | Constantly runs or jumps around the room | 0 | 1 | 2 | 3 |
| 49. | Rocks body back and forth repeatedly | 0 | 1 | 2 | 3 |
| 50. | Deliberately hurts himself/herself | 0 | 1 | 2 | 3 |
| 51. | Pays no attention when spoken to | 0 | 1 | 2 | 3 |
| 52. | Does physical violence to self | 0 | 1 | 2 | 3 |
| 53. | Inactive, never moves spontaneously | 0 | 1 | 2 | 3 |
| 54. | Tends to be excessively active | 0 | 1 | 2 | 3 |
| 55. | Responds negatively to affection | 0 | 1 | 2 | 3 |
| 56. | Deliberately ignores directions | 0 | 1 | 2 | 3 |
| 57. | Has temper outbursts or tantrums | 0 | 1 | 2 | 3 |
| | when he/she does not get own way | | | | |
| 58. | Shows few social reactions to others | 0 | 1 | 2 | 3 |

The ABC has been utilized in autism drug clinical trials because it has the characteristics of validity, reliability and drug sensitivity. All three are necessary to make the ABC useable as an outcome measure for clinical trials for drugs for autism. It is a predictor of maladaptive behavior. Extensive psychometric assessment of the ABC has indicated that its subscales have high internal consistency, adequate reliability, and established validity.

| | |
|---|---|
| Reliability | Internal consistency: Aman *et al.* reported internal consistencies of 0.86-0.94 in the original development study. Generally, other studies have confirmed this range of internal consistencies. However, some studies have found internal consistencies as low as 0.19 (Freund, teacher form). |
| | Test-retest: The original development study reported test-retest reliabilities of 0.96-0.99. However, the subsequent studies failed to validate these findings. Generally, have been fairly good, ranging from 0.50-0.67 (Freund, teacher form) to 0.80-0.95 (Freund, parent form). |
| | Inter-rater: The original development study reported inter-rater reliabilities of 0. 17-0.90, with a mean of 0.60. Subsequent studies have found a wide variability of inter-rater reliabilities, ranging from 0.12 to 0.95 (both in Schroeder). |
| Validity | There has been extensive validation of the 5-factor structure. The original development study found that the ABC demonstrated moderate discriminative validity with a number of instruments, as well as convergent validity with behavioral observation reports. It also demonstrated adequate predictive validity. Subsequent studies have provided further evidence of predictive, convergent and discriminative validities. |

### The EVT-2 test

The EVT-2 test builds on the strength of the EVT in that is brief and easy to administer; the EVT-2 assessment has been designed to coordinate with the PPVT^{™}-4 (Peabody Picture Vocabulary Test, Fourth Edition) test. Together, these tools give provide a comprehensive system for comparing receptive and expressive vocabulary.

Unlike some other measures of vocabulary, the EVT-2 supplies two equivalent forms of the test which contain different vocabulary items-helping ensure a subject has not "learned" the test. One form can be used prior to intervention to assess subjects' vocabulary knowledge and the alternative form can be used for re-testing to evaluate and document progress. EVT-2 also includes a unique Growth Scale Value (GSV) which is sensitive to small changes over time. Users & Applications

The EVT-2 test is individually administered and norm-referenced. It meets the needs of a wide variety of professionals to help: (1) quickly assess expressive vocabulary with a test that requires no reading or writing; (2) evaluate English Language Learners' (ELL) vocabulary acquisition as a flexible measure of their English word knowledge; (3) make comparisons with receptive vocabulary to pinpoint a student's strengths/weaknesses and identify potential word retrieval concerns; (4) move immediately into evidence-based interventions using those embedded directly into and linked into the ASSIST software (SAS; www.sas.com/products/assist/index.html); (5) assess oral expression as a foundation of writing skills; and (5) measure progress using one or both parallel forms.

Features and benefits of the EVT-2 test include, for example, two parallel forms for easier progress monitoring; identical administration and scoring procedures to the EVT; broader and more mixed use of labeling and synonym item types; five levels of diagnostic analysis; and new Growth Scale Value (GSV) for measuring progress over time.

Developed over a five-year period, the EVT-2 test was co-normed along with the PPVT-4 test with a national sample of subjects ranging in age from 2:6-90+. More than 5,500 subjects were tested; data from approximately 3,500 subjects were used for the normative scores. The remaining data contributed to the validation studies. The sample was tightly controlled and was matched to the U.S. Census on gender, race/ ethnicity, region, socioeconomic status (SES), and clinical diagnosis or special education placement. The EVT-2 test provides extremely reliable scores, with reliability coefficients in the 0.90s for almost every age or grade. Additionally, the PPVT-4 test offers many enhancements to a vocabulary assessment that has been well respected for 50 years. This latest edition has been co-normed with the Expressive Vocabulary Test, Second Edition (EVT^{™}-2), allowing for direct comparisons between receptive and expressive vocabulary performance.

### The PPVT-2 test

Unlike some other measures of vocabulary, the PPVT-2 supplies two equivalent forms of the test which contain different vocabulary items, thereby helping ensure a subject has not "learned" the test. One form can be used prior to intervention to assess subjects' vocabulary knowledge and the alternative form can be used for re-testing to evaluate and document progress. PPVT-2 also includes a unique Growth Scale Value (GSV) which is sensitive to small changes over time.

The PPVT-4 test is individually administered and norm-referenced. It may be used to quickly evaluate receptive vocabulary with a test that requires no reading or writing; monitor progress using two parallel forms; directly compare receptive and expressive vocabulary when you also administer the EVT-2; move immediately into evidence-based interventions using those embedded directly into and linked into the

ASSIST software; and meet guidelines for universal screening, identifying strengths and weaknesses, and diagnostic testing in an RTI environment

In normally developing subjects the EVT and the PPVT should demonstrate similar growth. The growth scales seen over a 12 month period should keep pace with one another. In the case of neurologically or otherwise impaired subjects the scales will often not keep pace with one another.

### The Connors test

Changes in hyperactivity can be measured in the Conners' 3- which is the Gold Standard for diagnosing as well as adjusting established medications for Attention Deficit and Attention Deficit Hyperactivity Disorders. Connors' 3 can be administered according to conventional methods and scored according the T-score guidelines. Attributes that are assessed include: restless or overactive behavior; excitability and impulsiveness; failing to finish tasks; inattentiveness and ease of distraction; temper outbursts; fidgeting; disturbances of other subjects; demands to be met immediately and ease of frustration; ease and frequency of crying; and rapid and drastic mood changes. Each attribute is scored on a scale of 0-3 where 0 = never, seldom; 1 = occasionally; 2 = often, quite a bit; and 3 = very often; very frequent.

### Block Food Screeners for Ages 2-17

These screeners are designed to assess subject's intake by food group, with outcomes measured in number of servings. One version asks about food eaten "yesterday," and a second version about food eaten "last week." The focus of these tools is on intake of fruit and fruit juices, vegetables, potatoes (including French fries), whole grains, meat/poultry/fish, dairy, legumes, saturated fat, "added sugars" (in sweetened cereals, soft drinks, and sweets), glycemic load and glycemic index. A secondary analysis produces estimates for intake of sugary beverages (both kcal and frequency). Individual portion sizes are asked. This questionnaire was designed for self-administration by subjects with the assistance of parent or caregiver, as needed.

The block food screener and block food screener last week have been utilized in clinical trials as well as by the USDA to examine food intake in various populations including subjects.

The tests have been validated and have excellent internal and external validity. The questionnaires are machine scored, and the intake is determined through a series of questions that have been designed to look at food types as well as portions to determine the intake of nutrients.

### Enzyme Preparations and Uses Thereof

Digestive enzymes to be used in the compositions and methods described herein include, for example, pancreatic enzymes. There are two types of pancreatic enzymes which have U.S.P. designations: pancreatin and pancrealipase. Pancreatin is a substance containing enzymes, principally amylase, lipase, and protease, obtained from the pancreas of the hog *Sus scrofa* Linne var. *domesticus* Gray (Fam. Suidae) or of the ox *Bos Taurus* Linne (Fam. Bocidae). Pancreatin contains, in each mg, not less than 25 U.S.P. units of amylase activity, not less than 2 U.S.P. units of lipase activity, and not less than 25 U.S.P. of protease activity. More information on pancreatin is provided in Example 1 below. In contrast, pancrealipase U.S.P. refers to a cream-colored, amorphous powder, having a faint, characteristic (meaty), but not offensive odor, which contains lipase in an amount of not less than 24 U.S.P. Units/mg; protease in an amount of not less than 100 U.S.P. Units/mg; and amylase in an amount of not less than 100 U.S.P. Units/mg; with not more than 5% fat and not more than 5% loss on drying.

Enzyme preparations with non-lipid enteric coatings have been used to deliver lipases in subjects requiring administration of lipases to subjects in need of enzyme treatment. In addition, Fallon has described certain methods and enzyme compositions for use in treating subjects and other subjects, with autism, ADD, ADHD, and other neurological diseases or conditions, for example, U.S. Patent Nos. 7,138,123, 6,660,831, 6,632,429; and 6,534,063.

The nature of the human digestive tract creates challenges for the delivery of digestive enzymes to subjects with neurological and behavioral conditions susceptible to treatment with digestive enzymes. Multiple temperature and pH changes over the course of the digestive tract make specific delivery a necessity and a challenge. For instance, pH as low as 1 is encountered in the stomach, but rapidly increases to a more basic pH of 5-6 in the proximal small intestine through the addition of bicarbonate ions secreted by the pancreas. For example, generally the pH in the stomach is approximately 1.2, the pH in the duodenum is about 5.0 to 6.5; the pH in the jejunum is about 6.8, and the pH is about 7.2 in the proximal ileum and about 7.5 in the distal ileum. The low pH in the stomach which changes rapidly to a more basic pH of 5-6 in the proximal small intestines, call for a specific delivery method depending upon where the enzyme is to be delivered.

For example, it was observed that subjects with autism who need treatment with proteases benefit from delivery of those enzymes to the proximal small intestine.

Delivery of digestive enzymes can also be challenging due to the rapid degradation and denaturing of enzymes at ambient room temperature, as well as the enhanced degradation and denaturing that can occur with high temperature, pressure, humidity and/or exposure to light. Moisture and heat together can quickly destabilize enzymes, reducing their effectiveness, and shortening shelf life, leading to inaccurate dosing. Denaturization or destabilization of the enzymes can reduce their effectiveness by reducing the dose of active enzymes to less than the amount needed for effective treatment. Alternatively, attempting to compensate for the denaturization or destablization by increasing the dose to ensure an effective level of active enzyme, could risk an overdose or overfilling a capsule or other dosage form. To protect and stabilize the pancreatic/digestive enzymes from unfavorable conditions, such a penetration, decomposition, the pancreatic/digestive enzymes (core) can be coated or encapsulated in a continuous coating containing an emulsifiable lipid. Disclosed herein are new coated enzyme preparations with improved shelf life.

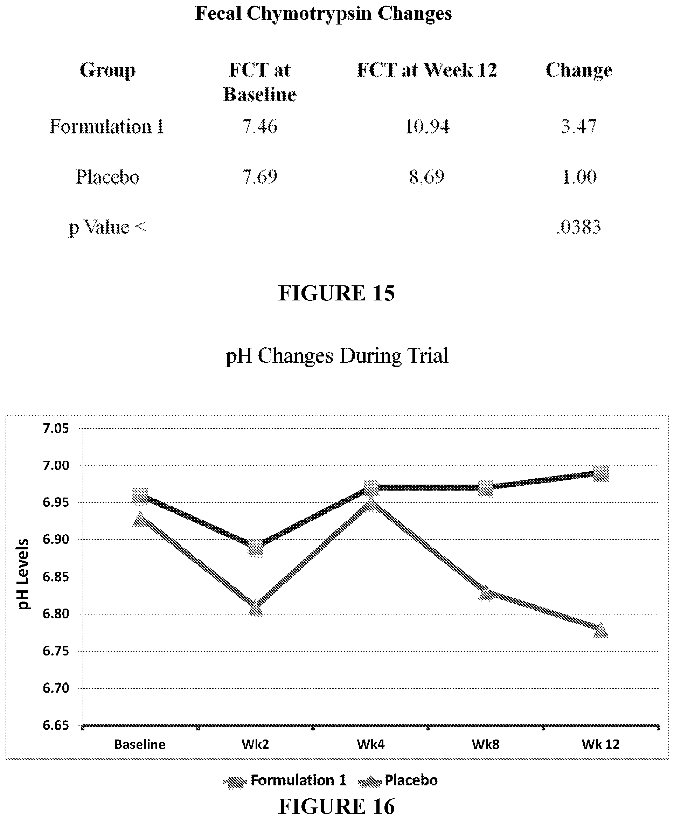

In one embodiment, a composition provided herein contains the major proteases shown in Figure 15. Compositions provided herein are safe for administration to human subjects, including subjects.

Compositions may be any form acceptable for administration to a subject including, but not limited to particles, sprinkles, powder, tablets, mini-tablets, capsules, *etc.*

Manufacturers of enzyme preparations have used enteric coatings to deliver lipases in subjects requiring administration of lipases. Because the porcine enzymes are delivered in a mixture of proteases, lipases and amylases, and because these compositions for human consumption were prepared for lipase delivery, the uses of these enteric coatings, which include such substances as hypromellose phthalate, dimethicone 1000, and dibutyl phthalate, preclude delivery of proteases at the proper location in the digestive tract. All other enzyme preparations presently on the market contain at least one of these enteric coating substances and/or other additives in the preparation. Some additives that enable manufacturing, such as additives to improve flow properties, can further risk subject reactivity or sensitivity to the enzyme preparation.

The use of phthalates which has been the state of the art for some time with respect to the delivery of enzymes which have been utilized to deliver lipases for pancreatitis. Phthalates have been implicated in a number of diseases including cancer and autism. The use of enteric coatings which are phthalate derived were not utilized in this formulation due to these potential side effects.

FDA has issued a draft guidance with respect to the use of phthalates and have requested that all pharmaceuticals which employ the use of phthalates be re-formulated to exclude the use of phthalates in all pharmaceutical preparations.

In one embodiment, a composition described includes a coated digestive enzyme preparation and/or composite, which, in some embodiments is an encapsulated pancreatic/digestive enzyme preparation. Also disclosed herein are enzyme delivery systems and pharmaceutical compositions comprising coated pancreatic/digestive enzyme preparations. These coated or encapsulated enzyme preparations contain cores comprising pancreatic or digestive enzyme particles, and a coating comprising an emulsifiable lipid.

The coatings in the digestive/pancreatic enzyme preparations create a barrier to degradation and denaturation, and allow more accurate levels of active enzymes to reach the treated subjects. The lipid coating of this disclosure provide a significant barrier to moisture, heat, humidity and exposure to light by allowing for a physical barrier as well as one that prevents and or reduces hydrolysis. The coated enzyme preparations undergo less hydrolysis as a result of protection from moisture in the environment by the lipid coating. As a result, pancreatic / digestive enzymes are provided which can tolerate storage conditions (e.g., moisture, heat, oxygen, *etc.)* for long periods of time thus enabling extended shelf life. The coating of the encapsulated enzyme preparation protects the enzyme from the environment and provides emulsification in a solvent without detracting from the abrasion resistance of the coating.

The coated enzyme preparations therefore reduce overfilling of the enzyme dosage, and enhance delivery of more accurate doses of the enzyme to subjects with autism, ADD, ADHD, and other neurological or behavioral conditions or diseases susceptible to treatment with pancreatic or digestive enzymes.

In addition, because subjects and other subjects with autism and other conditions often have multiple sensitivities to foods, additives, colorants and other carriers, excipients or substances used in drug formulations, it is a challenge to make an enzyme delivery system that avoids the use of allergens, and other carriers, excipients, extenders, colorants, *etc.*, that could potentially add to adverse symptoms or the morbidity of subjects. Furthermore, in very young subjects, an enzyme delivery system which allows ease and tolerability is paramount. A sachet delivery system for these enzyme preparations has also heretofore not been achieved.

Also disclosed is the use an enzyme preparation that is prepared without extenders colorants, dyes, flow enhancers and other additives to reduce the potential for allergens and other sensitivity reactions in subjects and other treated subjects. It has been discovered that in some embodiments, the digestive enzymes can surprisingly be encapsulated with a single lipid excipient to improve retention of enzyme activity, ease of administration, tolerability, and safety of administration, among other properties. It has been previously found that digestive enzyme particles containing lipases can be successfully encapsulated with coating consisting essentially of only hydrogenated soy oil.

In addition, porcine pancreatic /digestive enzymes possess a significant odor and taste, similar to that of cured/smoked pork. This taste can be strong and offensive to some subjects administered enzyme replacement, and especially to subjects. The addition of a lipid coating provides significant taste masking to the enzyme preparation, which allows for the tolerance of taste, as the lipid coating is odorless and tasteless. The use of this method of taste masking which does not involve the use of color, dyes, perfumes, recipients, or other substances is preferable for the administration of medications, which have an unpleasant or undesirable taste and odor. Coated digestive enzyme preparations described herein may have improved taste and smell.

In some embodiments, the coatings on the digestive enzyme particle cores are preferably continuous coatings. By "continuous," it is meant that the pancreatic/digestive enzyme is uniformity protected. The continuous coating of the fully surrounds or encapsulates the pancreatic/digestive enzymes. The encapsulation provides protection of the pancreatic/digestive enzyme from conditions such as moisture, temperature, and conditions encountered during storage.

In addition, the encapsulation also provides controlled-release of the pancreatic/digestive enzyme. The emulsification properties of the coating in a solvent allows for controlled release of the enzyme in the gastrointestinal system, preferably the region of the GI tract where the enzymes are to be utilized. The coating of the encapsulated composite protects the enzyme from the environment and provides emulsification in a solvent without detracting from the abrasion resistance of the coating. For example, for conditions requiring treatment with proteases, the release of the protease portion of the enzymes is necessary in the proximal small intestine, thereby necessitating a lipid encapsulation which has a dissolution profile between 30-90 minutes. The dissolution profile can also be about 30, 35, 40, 45, 50, 55, 60, 65, 70, 75, 80, 85, or 90 minutes. The dissolution profile can also be enhanced by this method to included longer or shorter dissolution profiles. Dissolution profiles can be obtained using methods and conditions known to those of skill in the art. For example, dissolution profiles can be determined at various pH's, including pH 1, 2, 3, 4, 5, 6, 7, 8, 9 or 10.

The rate of release of the bioactive substance can also be controlled by the addition of additives as described below. When the preparations are exposed to a solvent, the solvent interacts with the mollifiable lipid in the coating and results in emulsification of the coating and release of the bioactive substance.

"Encapsulate" as used herein means that the coating completely surrounds the pancreatic/digestive enzyme. In a population of encapsulated particles, encapsulated enzyme preparations can include contaminating or small portion of particles with a substantially continuous coating as long as the release profiles of the encapsulated particles are not significantly altered. A coated or encapsulated particle can contain one or more digestive enzyme particles enveloped in one coating to form one coated or encapsulated digestive enzyme particle in the coated or encapsulated digestive enzyme preparation.

Compositions described herein can be used for the treatment of neurological or behavioral disorders which have overlapping symptomotology. In addition to Autism and autism spectrum disorder, ADD, ADHD, and the other behavioral or neurological conditions or diseases susceptible to treatment with pancreatic or digestive enzymes or with overlapping symptomotology. By "susceptible to treatment with pancreatic or digestive enzymes," is meant that one or more symptoms of the disease or condition can be alleviated, treated, or reduced by administration of an effective amount of pancreatic or digestive enzymes.

There are often multiple co-morbid symptoms seen in behavioral or neurological conditions. For example, hyperactivity is 60% co-morbid in autism. Other symptoms which are often seen in multiple neurological conditions include seizure disorders. So seizures are a common occurrence in subjects with autism.

Compositions described herein can be used for the treatment of, for example, gastrointestinal issues (*e.g.*, constipation and/or diarrhea) associated with the neurological disorder, seizures (*e.g.*, "Grand Mal", absence, myoclonic, tonic, clonic and/or atonic seizures), sensory issues (*e.g.*, sight, sound, stimming, taste, touch and/or smell), speech issues such as expressive (*e.g.*, stereotyped and repetitive) and/or receptive speech, socialization issues (*e.g.*, lethargy, social reciprocity, non-verbal communication and/or peer relationships), obsessive compulsive disorder issues such as obsession (*e.g.*, thoughts, impulses and/or images) and compulsion (*e.g.*, mental and/or behavioral), irritability, fragile X, hypersensory issues, hyperactivity issues, or a combination thereof.

It has been previously found that selected coated enzyme preparations can be made by coating digestive enzyme particles with lipids not previously used in coated digestive enzyme preparations. The unique mixtures of emulsifiable lipids and enzymes can deliver certain components of the pancreatic / digestive enzymes to selected locations and/or at selected times during transit of the GI tract. Disclosed herein but not claimed are methods of delivering digestive enzymes to humans based upon dissolution profiles.

The emulsifiable lipid may be any lipid, lipid mixture, or blend of lipid and emulsifiers which emulsifies when exposed to a solvent, and has a melting point which allows the lipid to be a solid at typical storage temperatures. The emulsifiable lipid can be a vegetable or animal derived-lipid. In some embodiments, the emulsifiable lipid consists essentially of, or comprises one or more monoglycerides, diglycerides or triglycerides, or other components including, for example, emulsifiers found in hydrogenated vegetable oils. In another embodiment the lipid is a non-polar lipid.

As used herein, animal and/or vegetable "derived" lipids can include fats and oils originating from plant or animal sources and/or tissues, and/or synthetically produced based on the structures of fats and oils originating from plant or animal sources. Lipid material can be refined, extracted or purified by known chemical or mechanical processes. Certain fatty acids present in lipids, termed essential fatty acids, must be present in the mammalian diet. The lipid can, in some embodiments, comprise a Type I U.S.P.-National Formulary vegetable oil.

The digestive enzymes used in the describe methods can be any combination of digestive enzymes of a type produced by the pancreas, including, but not limited to digestive enzymes from a pancreatic source or other sources. The enzymes are not limited to pancreatic enzymes of porcine origin, but can be of other animal or plant origin as well as those which are synthetically derived. The digestive enzymes can be derived from mammalian sources such as porcine-derived digestive enzymes. The enzymes can include one or more enzymes, and can also be plant derived, synthetically derived, recombinantly produced in microbial, yeast, or mammalian cells, and can include a mixture of enzymes from one or more sources. Digestive enzymes can include, for example, one or more enzymes from more or more sources mixed together. This includes, for example, the addition of single digestive enzymes to digestive enzymes derived from pancreatic sources in order to provide appropriate levels of specific enzymes that provide more effective treatment for a selected disease or condition. One source of digestive enzymes can be obtained, for example, from Scientific Protein Laboratories. The digestive enzyme can be, for example a pancreatic extract complex composition. In one embodiment, the digestive enzymes will comprise or consist essentially of 25 U.S.P. units/mg protease, 2 U.S.P. Unit/mg lipase, and 25 U.S.P. Units/mg amylase. The term digestive enzyme can refer to one or more enzymes of a type produced by the pancreas.

The digestive enzyme particles used as cores in the present disclosure include digestive enzyme particles where about 90% of the particles are between about #40 (425µm) and #140 (105µm) USSS mesh in size, or between about 105 to 425 µm, or where at least about 75% of the particles are between about #40 USSS (425µm) and #80 USSS (180µm) mesh, or about 180 to 425 µm in size. Particles between #40 USSS (425µm) and #140 (105µm) mesh in size pass through #40 USSS (425µm) mesh but do not pass through #140 USSS (105µm) mesh. The coated or encapsulated digestive enzyme particles in one embodiment can comprise less than about 35, 30, 25, 20, 15, or 10% of the particles which can be sieved through #100 USSS mesh (150 µm). In some embodiments, the term "non-aerosolizable" refers to a coated or encapsulated enzyme preparation where less than about 20% or less than about 15% of the particles can be sieved through #100 USSS mesh (150 µm). The encapsulated digestive enzyme preparation can be an encapsulated digestive enzyme composite where the digestive enzyme particles contain two or more enzymes.

The minimum amount of pancreatic enzyme present in the core is at least about 5% active enzymes by weight of the coated enzyme preparation, but in other embodiments can be at least about 30% or at least about 50% by weight. The maximum amount of pancreatic/digestive enzyme present in the composite is at most about 95% by weight, and in other embodiments at most about 90%, 85%, 80%, 75%, or 70% of the coated enzyme preparation. In other embodiments, the amount of pancreatic enzyme present in the composite is about 10%, 15%, 20%, 25%, 35%, 40%, 45%, 55%, 60%, 65%, 70%, 72.5%, 75%, 77.5%, 80%, 82.5%, 87.5%, or 92.5% by weight or anywhere in between.

"At least about" or "at most about" a percentage (%) of enzyme can include equal to or about that % of enzyme. The term "about" includes equal to, and a range that takes into account experimental error in a given measurement. As used in connection with particle sizes, the term "about" can refer to plus or minus 10, 9, 8, 7, 6, 5, 4, 3, 2, or 1% or anywhere in-between. As used in connection with % particles that can be sieved, the term "about" can refer to plus or minus 10, 9, 8, 7, 6, 5, 4, 3, 2, or 1% or anywhere in-between.

The composition which contains the encapsulated digestive enzyme preparation or composite can be delivered as a sprinkle, powder, capsule, tablet, pellet, caplet or other form. Packaging the encapsulated enzyme preparations in an enzyme delivery system that further comprises single dose sachet-housed sprinkle preparations allows for ease of delivery, and accurate dosing of the enzyme, by allowing a specific amount of enzyme to be delivered in each dosing. Allowing for specific unit dosing of an enzyme preparation which maintains the enzyme activity within specific stability parameters in an enhancement over other sprinkle formulations, which are housed, in a multi-unit dosing form that allows for air, moisture and heat to depredate and denature the enzyme preparation. In a preferred embodiment, the powder or sachet is housed in a trilaminar foil pouch, or similar barrier to keep out moisture and to protect the enzyme preparation from adverse environmental factors. Also, the disclosure relates to an improvement in stability due to a reduction in hydrolysis due to the lipid encapsulation.

Further, the lipid encapsulation methodology reduces the aerosolization of the enzyme preparation that can be caustic to the child if inhaled through the lungs or the nose. The disclosure includes delivery of digestive enzymes with improved safety of administration, by reducing the amount of aerosolization of the enzyme. The lipid encapsulation reduces aerosolization and the potential for caustic burn, aspiration, and/or aspiration pneumonias in subjects and administrators of the enzyme preparation, thereby reducing the potential for illness in already compromised subjects, and leading to safer administration.

As used herein, the term "non-aerosolizable" will be used to refer to a coated or encapsulated enzyme preparation where substantially all of the particles are large enough to eliminate or reduce aerosolization upon pouring of the coated enzyme preparation compared to uncoated enzyme particles. For example, the term "non-aerosolizable" can refer to a coated or encapsulated enzyme preparation where at least about 90% of the particles are between about #40 USSS (425µm) and #140 USSS (105µm) mesh in size, or between about 105 to 425 µm, or where at least about 75% of the particles are between about #40 USSS (425µm) and #80 USSS (180µm) mesh, or about 180 to 425 µm. The term "non aerosolizable" can also refer to a coated or encapsulated enzyme preparation where less than about 35, 30, 25, 20, 15 or 10% of the particles can be sieved through #100 USSS mesh (150 µm). In some embodiments, the term "non-aerosolizable" refers to a coated or encapsulated enzyme preparation where less than about 20% or less than about 15% of the particles can be sieved through #100 USSS mesh (150 µm).

As described and referred to herein, suitable pancreatic/digestive enzymes and suitable coatings can be used in the compositions and methods of this disclosure. The choice of suitable enzymes and of suitable lipid coatings, including choice of the type or amount of enzymes or coating, are guided by the specific enzyme needs of a subjects, and the selected diseases to be treated. The encapsulated enzyme preparations that are disclosed have not been previously described.

Some examples relate to specific blends of enzymes and lipids selected for delivery in subjects with ADD, ADHD, autism, and other neurological and behavioral disorders susceptible to treatment with digestive/pancreatic enzymes based on the transit times in the human gastrointestinal tract. It can further be based upon the need of a subject to be treated for various components of the digestive enzymes. Further, disclosed herein are examples that relate to improvement of the delivery of digestive enzymes to humans based specifically upon required delivery times, and dissolution profiles.

While general methods for coating certain sensitive biologic substances have been described, see, *e.g.*, US Patent No. 6,251,478, the encapsulated bioactive substance of this disclosure is an enzyme preparation comprising a core containing digestive enzymes comprising or consisting of multiple proteases, lipases, and amylases, and a coating which comprises or consists essentially of an emulsifiable lipid.

Additives can be blended with the emulsifiable lipid. Selection of the lipid(s) and additives will control the rate of release of the bioactive substance. In the case of the digestive and or pancreatic enzymes, the lipid coat must be uniquely chosen to release the bioactive substance in the area of the digestive tract selected for release to optimize treatment.

The disclosure further relates to the administering of the coated and/or encapsulated enzyme preparation in a sachet or pouch preparation for ease of delivery to subjects and adults. Also disclosed but not claimed is the administration of a coated enzyme particle preparation, housed in a sachet or pouch. This facilitates administration, including but not limited to, administration in food or drink, direct administration into the oral cavity, or administration directly into the GI system through an NG-tube, G-tube or other GI entrances or deliveries.

Each dose may contain about 100 to 1500 mg of coated or encapsulated enzyme preparation, and each dose can contain about 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, or 1500 mg of coated or encapsulated enzyme preparation. "About" can include 80 to 125% of the recited preparation. Each dose can also be plus or minus 10% of the recited weight. In one embodiment each does will have a protease activity of not less than about 156 U.S.P. units/mg ± 10%. The protease activity can also be not less than about 100, 105, 110, 115, 120, 125, 130, 135, 140, 145, 150, 155, 160, 165, 170, 175, 180, 185, 190, 195, or 200 U.S.P. units/mg.

At least two doses of a composition comprising a therapeutically effective amount of the coated digestive enzyme preparations can be administered in the methods described herein. About 80% of the enzyme may be released by about 30 minutes in a dissolution test performed at pH 6.0. About 80% of the enzyme may be released by about 30 minutes after the coated digestive enzyme preparations reach the small intestine.

Delivery of enzymes to humans can be improved by reducing the use of excipients, extenders and solvents currently used in the preparations for delivery of digestive enzymes to humans. For example, the encapsulated digestive enzyme preparation can contain only one excipient, which increases the safety of administration by decreasing the chance of an allergic response. In one embodiment, the excipient is hydrogenated soy oil.

Because, the lipid encapsulation method does not require the enzyme preparation to be treated with solvents, extenders and excipients to facilitate flow or improve stability, also disclosed is a "clean" preparation of GRAS substances (generally regarded as safe) to be administered. The reduction in the use of solvents, extenders excipients and other additives permitted by the methods of this disclosure reduces the exposure of subjects administered the enzyme replacement, to potential allergens, thereby producing a hypoallergenic enzyme preparation that further enhances its potential uses in the treatment of subjects who might otherwise develop an allergic response to treatment. Administration of the coated enzyme preparations of this disclosure can thus reduce exposure to potentially toxic substances and will also reduce the possibility of allergy formation. Accordingly, in some embodiments, the encapsulated digestive enzyme preparation is hypoallergenic.

Digestive enzymes can be safely administered. The lipid coat adds weight to the enzyme preparation, which reduces the potential for aerosolization. Previous uncoated enzymes have been shown to become aerosolized, and can therefore be inhaled and contact the nasal cavity or the lungs, causing injury to the mucosa of those administered and those administering the enzyme preparation.

Sachet preparations can be improved for delivery to subjects. Disclosed herein but not claimed are means for administration of a coated digestive enzyme preparation, housed in a sachet which allows for particular types of administration including but not limited to administration in food, drink, or direct administration into the oral cavity or directly into the GI system through a NG-tube, G-tube or other GI entrances. The sachet, which represents a unit dosage or multiple doses for a day, represents a single unit dose. The sachet of a trilaminar foil allows the enzyme /lipid powder to remain stable and allows for ease of administration.

In another embodiment, the rate of release of the pancreatic/digestive enzyme from an encapsulated enzyme preparation can be controlled upon exposure to a solvent. As disclosed herein but not claimed the method may comprise blending an emulsifiable lipid with an amount of one or more additives to obtain a lipid blend; and coating the digestive enzyme particle with the blend to form an encapsulated digestive enzyme preparation containing particles comprising a core which contains the enzyme, and a coating which contains the lipid. In some embodiments, the emulsifiable lipid is a blend where the emulsifiable lipid and additive are not the same, and where the rate of release of the enzyme from the encapsulated composite upon exposure to a solvent is decreased as the amount of additive is increased. In the alternative, the rate of release of the enzyme from the encapsulated composite upon exposure to a solvent is increased as the amount of additive is decreased.

The lipid coating surprisingly does not appear to be reduced or destroyed by HC1 (hydrochloric acid) present in the stomach, thereby protecting the enzyme from degradation following administration until the enzyme preparation reaches its target region in the GI tract. Further the lipid coat reduces the exposure of the enzyme to attack by water, thereby reducing hydrolysis, and further protecting the digestive enzymes from degradation. An excipient containing only lipid can be used to coat or encapsulate digestive enzyme particles containing lipase.

The use of digestive enzymes for the treatment of specific disease targets can be made possible by preparing encapsulated digestive enzyme composite having differing release characteristics. Since various neurological and behavioral diseases can impact the gastrointestinal systems in humans in various ways, the use of specific enzyme preparations and the ensuing encapsulation can make the difference as to where and for what duration of time the enzyme preparation is delivered.

In cases of administration to subjects where delivery of lipases is required for effective treatment, the dissolution profile of the enterically-coated digestive enzymes needs to favor a longer delay in the release of the enzymes, as well as the delivery of a high lipase formulation.

Treatment of subjects with autism who require delivery of protease enzymes for effective treatment, the lipid encapsulate can be modified to deliver the protease during an earlier transit time window, in the proximal small intestine, to optimize protein digestion. In another example, for subjects who have slow GI transit times due to the dysautonomic nature of their neurological condition, still another release profile is required to deliver enzymes for effective treatment. The lipid and/or additive selection will be made to obtain enzyme release at later times after administration.

Transit times for digestive enzymes through the digestive system can be controlled by layering lipids, or through encapsulation with specific lipid types. Also disclosed herein is the use of a composition containing a selected blend of enzymes and lipids for delivery in subjects susceptible to treatment with pancreatic/digestive enzymes, based upon the transit times in the gastrointestinal systems of humans.

The improved flow qualities can facilitate packaging of the coated digestive enzyme preparations into, for example, pouches or sachets.

Lipid encapsulation can be used to make a coated digestive enzyme preparation for specific delivery times within the human gastrointestinal (GI) tract targeted for use in the treatment of a specific disease or condition. This disease or condition can be caused by or characterized by a digestive deficit that can be treated by the administration of digestive enzymes to the appropriate region of the GI tract. The neurological or behavioral disease or condition is one not traditionally associated with the digestive system, where one or more symptoms can be treated by administering an effective amount of a pancreatic and/or digestive enzyme preparation.

Thus, disclosed herein is a lipid encapsulation of specific enzymes targeted for use in the treatment of specific diseases, and the encapsulation method includes the amount and type of lipids used in the methods of described herein for the preparation of the encapsulated digestive enzyme composite. The present disclosure also relate to methods of making the enzyme preparations by lipid coating and/or encapsulation of pancreatic and/or digestive enzymes. The methods comprise providing an emulsifiable lipid, and coating pancreatic/digestive enzyme particles with the lipid, where the pancreatic/digestive enzymes comprise 5-90% of the coated enzyme preparations by weight. The uncoated pancreatic/digestive enzyme particles may have a size range of about 105-425 µm.

The method described herein is for preparing an encapsulated digestive enzyme preparation, the method comprising a) screening uncoated digestive enzyme particles to obtain particles of a suitable size for encapsulation; and b) coating the screened digestive enzyme particles with an emulsifiable lipid to form coated or encapsulated digestive enzymes containing a core which contains the pancreatic/digestive enzyme and a coating which contains the emulsifiable lipid. The encapsulated digestive enzyme preparation may be a controlled release digestive enzyme preparation, which can have enhanced flow properties.

Screening of the particles can include quality control steps to improve the activity, appearance or particle size of the digestive enzyme. For example, the particles can be analyzed to determine enzyme activity content, and/or visualized using chromatographic, microscopic or other analytical methods. The particles can also be screened to obtain particles of a suitable size for encapsulation by removing particles that are too fine or too large. For example, the particles can be sieved to obtain particles of a suitable size or more uniform size range for encapsulation. As a further example, the particles can be sieved through USSS #40 mesh (425µm) and through USSS #140 (105µm) mesh. Particles that pass through the #40 USSS mesh (425µm) but are retained by the #140 USSS mesh (105µm) are of an appropriate size range for coating or encapsulation Particles can also be screened by sieving through USSS #140, #120, #100, #80, #70, #60, #50, #45, or #40 mesh, (i.e. 105µm, 125µm, 150µm, 180µm, 212µm, 250µm, 300µm, 355µm, or 425µm) or any combination thereof.

Enzyme preparations supplied by the API supplier can be provided as irregular shaped, and multi-sized particles, with uneven edges, and much clumping, and containing some crystalline salt particles (data not shown). Uneven particle size and shape reduces flow properties and interferes with packaging. In addition, pouring uncoated enzyme into the mouth of a subject would be difficult, and potentially can cause too much or too little of the enzyme to be delivered. Processing the digestive enzyme particles according to methods described herein yields a non-dusty, free-flowing particulate preparation suitable for sachet packaging and for pouring onto food or drink. In addition, as discussed throughout, the use of lipid encapsulation to prevent aerosolization, and therefore increase safety, to increase flow properties which enhance manufacturing of a pharmaceutical is disclosed herein.

The size distribution of particles in an exemplary raw enzyme preparation may be determined (data not shown). Large particles (>40 USSS mesh, 425µm) and very small particles (<140 USSS mesh, 105µm) are generally not suitable for proper encapsulation and can be removed by screening. In order to increase the flow properties of the encapsulated pancreatic enzyme preparation, digestive enzyme particles can be sieved to remove fines and overly large particles, for example by including only particles of sizes 40-140 USSS mesh (425µm to 105µm), or about 105 to 425 microns. The coated digestive enzyme preparation containing 80% digestive enzyme by weight may be made by coating sieved pancreatic enzyme particles with a hydrogenated vegetable oil using 20 lbs. of enzyme particles and 5 lbs of hydrogenated vegetable oil.

The temperature of the lipid or lipid blend may be maintained between 37.7°C (100° F) and 48.8 °C (120° F) before application to the digestive enzymes, which are not heated.

The lipid should be present in the preparation at a minimum amount of about 5% by weight of the encapsulated composite, preferably about 30%, and more preferably about 50% by weight of the encapsulated composite. The maximum amount of pancreatic/digestive enzyme present in the encapsulated composite is about 95% by weight of the composite, preferably about 90%, and more preferably about 85% of the encapsulated composite. The emulsifiable lipid can be any lipid or lipid-derived material that emulsifies or creates an emulsion yet has a melting point which allows the emulsifiable lipid to be a solid at typical storage temperatures, for example, 23° C.

"Emulsifiable lipids" as used herein means those lipids which contain at least one hydrophilic group and at least one hydrophobic group and have a structure capable of forming a hydrophilic and hydrophobic interface. These chemical and/or physical properties, mentioned above, of an emulsifiable lipid permit emulsification. Examples of interfaces include, for example, micelles and bilayers. The hydrophilic group can be a polar group and can be charged or uncharged.

The emulsifiable lipid can be derived from animal or vegetable origins, such as, for example, palm kernel oil, soybean oil, cottonseed oil, canola oil, and poultry fat, including hydrogenated type I vegetable oils. The lipid may be hydrogenated. The lipid can also be saturated or partially saturated. Examples of emulsifiable lipids include, but are not limited to, monoglycerides, diglycerides, fatty acids, esters of fatty acids, phospholipids, salts thereof, and combinations thereof.

The emulsifiable lipid is preferably a food grade emulsifiable lipid. Some examples of food grade emulsifiable lipids include sorbitan monostearates, sorbitan tristearates, and calcium stearoyl lactylates. Examples of food grade fatty acid esters which are emulsifiable lipids include acetic acid esters of mono- and diglycerides, citric acid esters of mono- and di-glycerides, lactic acid esters of mono- and di-gylcerides, polyglycerol esters of fatty acids, propylene glycol esters of fatty acids, and diacetyl tartaric acid esters of mono- and diglycerides. Lipids can include, for example, hydrogenated soy oil.

Any emulsifiable lipid can be used in the methods and products disclosed herein. The emulsifiable lipid used may produce non-agglomerating, non-aerosolizing enzyme preparation particles.

The method disclosed herein relates to preparation of an encapsulated, controlled release digestive enzyme preparation with enhanced flow properties, the method comprising: a) blending an emulsifiable lipid with one or more additives to obtain a blend; and b) coating screened digestive enzyme with the blend to form an encapsulated digestive enzyme containing a core which contains the digestive enzyme and a coating which contains the blend of emulsifiable lipid.

The coating of the enzyme with the lipid allows for the enzyme to become more uniform in size and shape, but reduces the jagged edges associated with the raw enzyme, and allows for ease of administration and ease of manufacturing, as the flow properties associated with the covered enzyme will allow for the manufacturing machinery to easily fill the sachet/pouch with the enzyme and reduces overfilling or under filing of the sachet. The unit dose packaging reduces the ability of the child to open the multi dose can/box/ or other container. The trilaminar foil pouch or sachet further reduces the ability of a subject to open the sachet/pouch, and over utilize the enzyme.

Disclosed herein but not claimed is a method of controlling the rate of release of a digestive enzyme from the encapsulated preparation by using a lipid blend to coat the digestive enzyme. The method includes blending an emulsifiable lipid with one or more additives to obtain a blend, and coating the digestive enzyme with the blend to form an encapsulated digestive enzyme containing a core which contains the digestive enzyme and a coating which contains the blend of emulsifiable lipid. The rate of release of the enzyme from the encapsulated preparation upon exposure with a solvent is decreased as the amount of additive is increased. In the alternative, the rate of release of the enzyme from the encapsulated composite upon exposure with a solvent is increased as the amount of additive is decreased. Thus, the nature of the coating allows for controlled release of the enzyme from the encapsulate.

Non-emulsifiable lipids do not possess the chemical and/or physical properties related to emulsification as described above and include any lipid, lipid derived material, waxes, organic esters, or combinations thereof. Non-emulsifiable lipids generally do not emulsify by themselves. Non-emulsifiable lipids can be used as additives so long as the properties of the coating, and constituent lipids, permit emulsification. Non-emulsifiable lipids, such as, for example, triglycerides, can be blended with an emulsifiable lipid disclosed herein. The non-emulsifiable lipid can be derived from animals, vegetables, mineral, or synthetic origins. The non-emulsifiable lipid is preferably hydrogenated, and can be saturated or partially saturated, and includes, but is not limited to triglycerides. The coating may contain a blend of monoglycerides and triglycerides applied to a pancreatic/digestive enzyme.

The inclusion of one or more additives with an emulsifiable lipid disclosed herein is used to control emulsification of the coating and release of the enzyme. For example, the additive, triglyceride, can be blended with monoglycerides (e.g., an emulsifiable lipid), to control emulsification of the coating and thus control (e.g., decrease) the rate of release of the enzyme from the composite. As a further example, one or more additives, such as a diglyceride and a triglyceride can be blended with the emulsifiable lipid to control the rate of release of the enzyme. Hydrogenated vegetable oils can contain emulsifying agents, such as soy lecithin or other components.

Properties including mechanical strength, melting point, and hydrophobicity can be considered when choosing a suitable lipid coating for the digestive enzyme. Lipids having lower melting points or more polar, hydrophilic properties were generally less suitable for encapsulation because they resulted in product that would cake under accelerated storage stability conditions. Enzyme preparations made using, for example, hydrogenated soy oil, hydrogenated castor wax, and carnauba wax all demonstrated good pouring and no caking.

The wax can be paraffin wax; a petroleum wax; a mineral wax such as ozokerite, ceresin, or montan wax; a vegetable wax such as, for example, carnauba wax, bayberry wax or flax wax; an animal wax such as, for example, spermaceti; or an insect wax such as beeswax.

Additionally, the wax material can be an ester of a fatty acid having 12 to 31 carbon atoms and a fatty alcohol having 12 to 31 carbon atoms, the ester having from a carbon atom content of from 24 to 62, or a mixture thereof. Examples include myricyl palmitate, cetyl palmitate, myricyl cerotate, cetyl myristate, ceryl palmitate, ceryl certate, myricyl melissate, stearyl palmitate, stearyl myristate, and lauryl laurate.

A method is disclosed herein but not claimed for controlling rate of release of a pancreatic/digestive enzyme from an encapsulated composite upon exposure to a solvent. The method includes coating the enzyme with an amount of an emulsifiable lipid to form an encapsulated pancreatic enzyme substance composite, wherein the rate of release of the enzyme from the encapsulated composite is decreased as the amount of emulsifiable lipid based on total weight of the encapsulated composite is increased. In the alternative, the rate of release of the pancreatic enzyme from the encapsulated composite is increased as the amount of emulsifiable lipid based on total weight of the encapsulated composite is decreased. The emulsifiable lipid useful in this example can consist essentially of one or more monoglycerides.

The solvent in which a lipid emulsifies can be an aqueous solvent. The aqueous solvent interacts with the hydrophilic groups present in the emulsifiable lipid and disrupts the continuity of the coating, resulting in an emulsion between the aqueous solvent and the lipids in the coating, thus releasing the bioactive substance from the composites.

Disclosed but not claimed are methods of using encapsulated pancreatic or digestive enzyme cores for treatment of neurological conditions or disorders to achieve specific ends. Disclosed herein but not claimed are methods for lipid encapsulation of medications for human consumption which have the characteristics of a time-released medication, and which utilize the lipid encapsulation for stability. Described below are methods for the preparation of an encapsulated enzyme preparation comprising a coating of emulsifiable lipid and a digestive enzyme suitable for the time-specific arid/or site-specific targeted release along the GI tract.

Parts of this disclosure stem from the surprising and unexpected discovery that certain pharmaceutical dosage preparations comprising a coating of emulsifiable lipid and a digestive enzyme can have novel potentiated activity and unexpected favorable release and dissolution profiles and absorption kinetic parameters along the various portion of the GI tract. These characteristics are useful for formulating a specific bioactive enzyme for site specific targeted release along the GI tract.

In some cases, determination of whether a subject is in need of treatment with an effective amount of digestive enzymes can be based on a determination that a subject has an enzyme deficiency. In other cases, determination of whether a subject is in need of treatment with an effective amount of digestive enzymes can be based on a determination that a subject has an abnormal chymotrypsin level as measured in the GI tract, directly or at the end of the GI tract as a measure of fecal chymotrypsin. In yet other cases, determination of whether a subject is in need of treatment with an effective amount of digestive enzymes can be based on a determination that a subject has an abnormal stool pH level. In yet other cases determination of whether a subject is in need of treatment with an effective amount of digestive enzymes can be based on a determination that a subject has abnormal FCT and stool pH levels.

Levels of amino acids which are the breakdown products of protease digestion can be measured as well to determine if there is a need for enzyme replacement. Low and absent proteases will leave a dearth of amino acids, and amino acid pool in the body will be altered and subsequent determination of the need for enzymes to break down proteins can be determined by the measurement of amino acids in the blood.

As enzyme replacement may be necessary as a result of enzymes (such as the proteases) either being secreted in insufficient amount, in normal amounts which are then degraded through an unsuitable environment in the small intestine, or if the enzyme (for example, the proteases) are secreted in a defective or inactive form will all necessitate the need for exogenous enzyme replacement.

Further stool pH will be low or abnormally low in the absence of protein breakdown and may signify the need for proteases replacement. Therefore, an abnormal blood or stool pH may indicate the need for enzyme replacement.

Further in the examination of nutrient uptake and nutrient digestion may also signal the need for enzyme replacement and/or an abnormal vitamin intake such as vitamin K could be potentially indicative of autism or the formation of autistic symptomotology, or other co-morbid condition.

Method disclosed herein may comprise using the enzyme formulations disclosed herein to treat subjects and other subjects with autism who also have an enzyme deficiency. The enzyme deficiency could be determined by any method used in determining or diagnosing an enzyme deficiency. The determination or diagnosis can be made by evaluating symptoms, including eating habits, self-imposed dietary restrictions, and symptoms of eating disorders and/or gastrointestinal disorders. The determination can be made on the basis of a biochemical test to detect, for example, levels or activities of enzymes secreted, excreted or present in the GI tract, and/or by determining the presence of a mutation in a gene or aberrant expression of a gene encoding one or more digestive enzymes. The enzyme deficiency can also be determined, for example, by detecting a mutation or aberrant expression of a gene encoding a product regulating or otherwise affecting expression or activity of one or more digestive enzymes.

The determination of behavioral symptoms and symptom improvement can be examined by the administration of enzymes and especially proteases to improve an aspect of the behavior. The behaviors include but are not limited to: irritability, agitation, aggression, crying, lethargy, social withdrawal; social isolation, stereotypic behavior including neuro stimming (autistic stereopathy) and obsessive compulsive behaviors, hyperactivity, noncompliance; inappropriate speech. Further, the behaviors can encompass, a lack of expressive or receptive language, limited vocabulary, lack or low levels of executive function, as well as restricted and repetitive movements and other proprioceptive issues which manifest in autism as well as other neurological conditions.

A subject to be treated can either have symptoms of autism or other co-morbid neurological or behavioral manifestations or have a genetic based co-morbidity. Further the assessment of the need for enzyme replacement could also determine the need for treatment with the enzyme delivery systems described herein. Subjects who are determined to have autism based on clinical symptoms but not a co-morbidity such as a genetic co-morbidity, are treated with the enzyme delivery systems described herein. However, subjects who are determined to have autism based on clinical symptoms and a co-morbidity, who nevertheless also test abnormally low for FCT level or positive using another indicator of GI pathogens and/or low digestive enzyme activity or expression can also be treated with the enzyme delivery systems disclosed herein.

The need for enzyme replacement based upon symptomology has utility as the degradation of certain enzymes makes testing difficult. A direct marker such as the measurement of fecal chymotrypsin, as well as a secondary or surrogate marker such as that seen with low circulating amino acids could be utilized for example to determine the need for the enzyme preparations described herein.

The determination of an enzyme deficiency can be made using a test for fecal chymotrypsin levels. Methods such as PCR or other amplification, SNP detection, sequencing, and/or DNA combing can be used to detect the presence of a mutation or presence of short RNA sequences which interfere with expression of one or more genes encoding a digestive enzyme. For example, the mutation can in a gene encoding a digestive enzyme which decreases or eliminates the activity of the enzyme. As another example, the mutation can be mutation in the MET gene, a gene encoding the pleiotropic MET receptor tyrosine kinase *See* Campbell et al., PNAS 103(46), 16834-39 (2006). These mutations can include, for example, the MET promoter variant rs1858830 C allele, and or mutations in the MET signaling pathway such as a haplotype of the SERPINE1 gene, or the rs344781 PLAUR promoter variant T allele.

The enzyme formulations disclosed herein are suited for use in delivering digestive enzymes to subjects with autism, autism spectrum disorders, ADD, ADHD, and other neurological diseases or conditions in need of enzyme treatment. The co-morbid symptoms of other neurological or behavioral conditions may also be amenable to treatment with the said enzyme preparation described herein.

Fallon has described certain methods and enzyme compositions for use in treating subjects and other subjects, with autism, for example, U.S. Patent Nos. 7,138,123, 6,660,831, 6,632,429, and 6,534,063.

In the experiments described herein, several factors were discovered that allowed for the unexpected enhanced / potentiated efficacy and property. For example, it was discovered that certain encapsulation enzymatic preparations comprising soy oil exhibited certain surprising characteristics that led to improvements in the site-specific activity, release/dissolution profile, and ease of manufacturing, packaging and storage. Without being bound to a particular theory of operation, the skilled artisans will appreciate that other methods of sample preparation and/or formulation that can also yield these advantageous parameters are also contemplated herein.

The encapsulation of an enzyme as described herein did not by definition anticipate the properties exhibited. For example, the stability of the product over 36 months under standard and nonstandard conditions was not obviated by the known materiality of the encapsulation process. The properties of the enzyme as formulated by ratio described herein could not have anticipated the protective qualities of the combined enzyme-encapsulation material complex.

The concentration of digestive enzymes in the pharmaceutical composition will depend on the degradation, inactivation and excretion rates of the enzymes, the physicochemical characteristics of the enzymes, the dosage schedule, the dosage form, and amount administered as well as other factors known to those of skill in the art.

The pharmaceutical composition can be administered at once or can be divided into a number of smaller doses to be administered at intervals of time. It is understood that the precise dosage and duration of treatment is a function of the wound and can be determined empirically using known testing protocols or by extrapolation from *in vivo* or *in vitro* test data. It is to be further understood that for any particular subject, specific dosage regimens should be adjusted over time according to a subject need and the professional judgment of the person administering or supervising the administration of the compositions, and that the concentration ranges set forth herein are exemplary only and are not intended to limit the scope or practice of the claimed compositions. In some embodiments, the compositions are provided in unit dosage forms suitable for single administration, or multi-dose administration, of a precise dose.

Dosing should be given with food to aid in the absorption of the nutrients and to facilitate breakdown. Also anticipated is release of the enzyme over time, and this can be expanded to a time release formulation whereby once a day or other long acting affects can come from the administration of the enzyme.

The compositions can be administered either alone or more typically in combination with a conventional pharmaceutical carrier, excipient or the like. The term "excipient" is used herein to describe any ingredient other than the compound(s) (enzymes) used in the composition as described herein and known in the art. Lipid encapsulation is one preferred embodiment, but other carriers and excipients can be utilized to deliver the enzyme preparation.

Methods of preparing such dosage forms are known, or will be apparent, to those skilled in this art; for example, see Remington: The Science and Practice of Pharmacy, 21st Edition (Lippincott Williams & Wilkins. 2005).

Appropriate dosages will depend on a subject (species, age, weight, health, *etc.),* the severity of the condition, the type of formulation and other factors known to those having ordinary skill in the art. It is to be noted that concentrations and dosage values can vary with the severity of the condition, weight of a subject, the amount and types of food eaten and other factors as determined by the administering practitioner. It is to be further understood that for any particular subject, specific dosage regimens should be adjusted over time according to a subject need and the professional judgment of the person administering or supervising the administration of the compositions.

In one embodiment a composition can be administered 1 or more times a day, such as 1, 2, 3, 4, 5, 6, 7, 8, 9, or 10 times a day preferably with food. Specific dosage forms or time release applications can be administered without food. In another embodiment, a composition can be administered orally 3 times a day with or without food, or with each substantial meal. The composition can be in tablet, capsule, granular, in sprinkle form, and have taste maskers present for ease of delivery to subjects. The compositions can be packaged in a unit dose package with the drug remaining stable for over 30 months.

Experiment 1 examined the changes seen in subjects administered Formulation 1 described herein when compared to subjects who received a placebo. Formulation 1 is a granulated pancreatin soy lipid-encapsulated drug product that has a protease activity of not less than 156 U.S.P. units/mg. A single dose of Formulation 1 is provided in a pouch or sachet at about 900 mg. Subjects aged 3-8 diagnosed with autism were administered a composition of formula 1 or a placebo. While females are typically known to have more severe cases of autism, one surprising discovery made by the present inventors is that girls were found to improve more than boys after treatment with compositions described herein. Boys were also identified as exhibiting improvement in one or more symptoms of autism. Furthermore, subjects with more severe cases of autism as a whole had greater improvements as compared to subjects with less severe cases of autism when treated with compositions described herein. Disclosed herein but not claimed is a method of treating an autistic child, comprising administering to a subject in need thereof a composition described herein. A subject to be treated with such methods may be autistic. A subject to be treated may be female. A subject to be treated may be male. A subject can be administered a single dose or multiple doses of the compositions. A subject treated with such methods may exhibit an improvement in autism of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

The factors (subscales) of the Aberrant Behavior Checklist (ABC) scale are as follows: (I) irritability, agitation, crying; (II) lethargy, social withdrawal; (III) stereotypic behavior; (IV) hyperactivity, noncompliance; and (V) inappropriate speech.

At a terminal FCT level of 6.0 Units/gram (fresh) or 9.0 (frozen) or greater of feces, additional improvements observed following treatment with compositions described herein include body weight, ABC stereotypical behavior, ABC hyperactivity, ABC irritability, ABC lethargy / social withdrawal.

At a terminal FCT level of 6.0 Units/gram (fresh) or 9.0 (frozen) or greater of feces, along with an FCT 3.5 or greater from baseline, the present inventors determined that there was statistically significant improvement in all 4 ABC scales - irritability, hyperactivity, lethargy / social withdrawal, and stereotypical behavior.

The present inventors have found that subjects treated with compositions described herein demonstrated overall better improvement as compared to treatment with aripiprazole (ABILIFY^{®}) on the ABC irritability scale. In general, females who experience an increase in FCT have an improvement is seen in four ABC scales: ABC stereotypical behavior, ABC hyperactivity, ABC irritability, ABC lethargy / social withdrawal.

Disclosed herein but not claimed is a method of improving all four subscales of the ABC scale in a subject, comprising administering to a subject in need thereof a composition described herein. A subject to be treated with such methods may be autistic. A subject to be treated may be female. A subject to be treated may be male. A subject treated with such methods may exhibit an improvement in all four subscales of the ABC scale of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95% or about 100% compared to a subject treated with a placebo.

Subjects treated with a composition described herein may exhibit an improvement in irritability and/or agitation according to the ABC scale. Disclosed herein but not claimed is a method of treating irritability and/or agitation in a subject, comprising administering to a subject in need thereof a composition described herein. A subject can be administered a single dose or multiple doses of the compositions. A subject treated with such methods may exhibit an improvement in irritability and/or agitation of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95% or about 100% compared to a subject treated with a placebo.

In another embodiment, subjects treated with a composition described herein exhibit a significant improvement in social withdrawal and/or lethargy based on the ABC scale. Disclosed herein but not claimed is a method of improving social withdrawal and/or lethargy in a subject, comprising administering to a subject in need thereof a composition described herein. A subject can be administered a single dose or multiple doses of the compositions. A subject treated with such methods may exhibit an improvement in withdrawal and/or lethargy of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

In one embodiment, subjects treated with a composition described herein exhibit a significant improvement in hyperactivity based on the ABC scale. Disclosed herein but not claimed is a method of decreasing hyperactivity in a subject, comprising administering to a subject in need thereof a composition described herein. A subject can be administered a single dose or multiple doses of the compositions. In a subject treated with such methods may exhibit an improvement (i.e., a decrease) in hyperactivity of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

Disclosed herein but not claimed is a method of improving stereotypic behavior in a subject, comprising administering to a subject in need thereof a composition described herein. A subject to be treated with such methods may be autistic. A subject can be administered a single dose or multiple doses of the compositions. A subject treated with such methods may exhibit an improvement in stereotypic behavior of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

The present inventors identified that all of the behavioral measurements that improved after treatment with the present methods, did so in correlation with improved FCT levels.

Disclosed herein but not claimed is a method of improving one or more behaviors in a subject, comprising administering to a subject in need thereof a composition described herein. A subject to be treated with such methods may be autistic. A subject can be administered a single dose or multiple doses of the compositions. A subject treated with such methods may exhibit an improvement in one or more behaviors of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

Disclosed herein but not claimed is a method of improving inappropriate speech in a subject, comprising administering to a subject in need thereof a composition described herein. A subject can be administered a single dose or multiple doses of the compositions. A subject treated with such methods may exhibit an improvement (i.e., decrease) in inappropriate speech of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

In yet another embodiment, subjects treated with a composition described herein exhibit an improvement in the overall 5 subscales, which include the three described above, as well as stereotypy and inappropriate speech.

Disclosed herein but not claimed is a method of improving one, two, three, four, or five of the subscales of the ABC scale in a subject, comprising administering to a subject in need thereof a composition described herein. A subject can be administered a single dose or multiple doses of the compositions. A subject treated with such methods may exhibit an improvement of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% of one, two, three, four, or five subscales of the ABC scale compared to a subject treated with a placebo.

In one embodiment the lethargy and hyperactivity scales were both reduced whereas, typically, they are reciprocal scales - *i.e.*, an improvement in lethargy also results in an increase in hyperactivity and vice versa.

Disclosed herein but not claimed is a method of improving lethargy and hyperactivity in a subject, comprising administering to a subject in need thereof a composition described herein. A subject can be administered a single dose or multiple doses of the compositions. A subject treated with such methods may exhibit an improvement in lethargy and hyperactivity of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

The present methods are efficacious in subjects that hold mild and or moderate levels of lethargy, hyperactivity, social withdrawal, and irritability. The present methods are even more efficacious in subjects with increased levels of lethargy, hyperactivity, social withdrawal, and irritability.

The compositions described herein, in certain embodiments, behave like a partial dopamine agonist.

In one embodiment the compositions described herein accomplish this effect without a sedating effect or an increase in neurological symptoms (such as dizziness, Parkinsonisms, dystonia, akathisia, somnolence, fatigue, extrapyramidal disorders, tremor, and drooling), as compared to other approved drugs for autism. Treatment of a subject with such methods does not cause a sedating effect. Treatment of a subject with such methods does not cause an increase in one or more neurological symptoms, wherein the neurological symptoms are dizziness, Parkinsonisms, dystonia, akathisia, somnolence, fatigue, extrapyramidal disorders, tremor, and drooling.

In one embodiment, improvement in one or more symptoms in subjects after treatment with a composition described herein is accomplished without weight gain.

Furthermore, these positive changes in the subscales can be accomplished/met without any side effects in accordance with FDA reporting standards (a rate greater than 5%).

Disclosed herein but not claimed is a method of treating a subject exhibiting one or more of the ABC subscales: (I) irritability, agitation, crying; (II) lethargy, social withdrawal; (III) stereotypic behavior; (IV) hyperactivity, noncompliance; and (V) inappropriate speech, comprising administering to a subject in need thereof a composition described herein, wherein the side effects of treatment are without any side effects in accordance with FDA reporting standards (a rate greater than 5%).

Changes that occur in the subscales may occur at week 4 and continue to improve thereafter. Within subjects of ages 3 to 8, there is no age effect, that is, the compositions are equally efficacious amongst all age groups. No geographical correlation was observed in subjects treated with compositions described herein.

Improvements were observed in both receptive and expressive language as measured by the Expressive Vocabulary Test (EVT) and the Peabody Picture Vocabulary Test. The Expressive Vocabulary Test (EVT) is a subjectively administered, norm-referenced test of expressive vocabulary and word retrieval. For 38 labeling items, the examiner points to a picture or a part of the body and asks a question. On 152 synonym items, the examiner presents a picture and stimulus word(s) within a carrier phrase. The examinee responds to each item with a one-word answer. All stimulus pictures are in full color, carefully balanced for gender and ethnic representation. Results are assessed based on Age- and Grade-Based Standard Scores, Percentiles, Normal Curve Equivalents (NCEs), Stanines, Age and Grade Equivalents, Growth Scale Value (GSV).

The Peabody Picture Vocabulary Test measures a subject's receptive (hearing) vocabulary for Standard American English and provides a quick estimate of their verbal ability or scholastic aptitude. An examiner presents a series of pictures to each person. There are four pictures to a page, and each is numbered. The examiner states a word describing one of the pictures and asks a subject to point to or say the number of the picture that the word describes. Item responses can also be made by multiple choice selection depending upon subject's age. The total score can be converted to a percentile rank, mental age, or a standard deviation IQ score.

Disclosed herein but not claimed is a method of improving receptive and expressive language in a subject, comprising administering to a subject in need thereof a composition described herein. A subject can be administered a single dose or multiple doses of the compositions. A subject treated with such methods may exhibit an improvement in receptive and expressive language of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

Subjects with autism that had aphasia and other potential unknown causes that present as a lack of expressive language manifest speech improvements surprisingly with age appropriate grammatical structure and vocabulary without a learning curve. Disclosed herein but not claimed is a method of improving grammatical structure and vocabulary, comprising administering to a subject in need thereof a composition described herein. A subject can be administered a single dose or multiple doses of the compositions. A subject treated with such methods ay exhibit an improvement in grammatical structure and vocabulary of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

Disclosed herein but not claimed is a method of improving overall growth scales as well as increased question ceiling levels and reduction in error rates in a subject, comprising administering to a subject in need thereof a composition described herein. A subject can be administered a single dose or multiple doses of the compositions. A subject treated with such methods may exhibit an improvement in overall growth scales of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo. A subject treated with such methods may exhibit an increased question ceiling level and reduction in error rate of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

Changes in hyperactivity were also observed in the Conners' 3-teacher report (3-TR) which is the gold standard for diagnosing as well as adjusting established medications for Attention Deficit and Attention Deficit Hyperactivity Disorders. Connors' 3-TR can be administered according to conventional methods and scored according the T-score guidelines. Attributes that are assessed include: restless or overactive behavior; excitability and impulsiveness; failing to finish tasks; inattentiveness and ease of distraction; temper outbursts; fidgeting; disturbances of other subjects; demands to be met immediately and ease of frustration; ease and frequency of crying; and rapid and drastic mood changes. Each attribute is scored on a scale of 0-3 where 0 = never, seldom; 1 = occasionally; 2 = often, quite a bit; and 3 = very often; very frequent.

Administration of the compositions described herein resulted in a significant reduction in hyperactivity and improvement in the Global Index of the Conners' Test.

Disclosed herein but not claimed is a method of treating a subject, comprising administering to a subject in need thereof a composition described herein, wherein treatment reduces hyperactivity results in an improvement in inattention, learning problems, executive functioning, aggression, and peer relations. A subject can be administered a single dose or multiple doses of the compositions. A subject treated with such methods may exhibit an improvement in inattention, learning problems, executive functioning, aggression, and peer relations of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

Persuasive development disorders can be classified under the DSM-IV-TR classification as an autistic disorder, Asperger's Disorder, PDD-NOS (including atypical autism), Rett's Disorder, and Childhood Disintegrative Disorder. Treatment may improve one or more of the attributes of the Conners' DSM-IV Scale according to conventional scoring. The Conners' ADHD/DSM-IV Scales (CADS) consist of the items of the CRS-R that best differentiate subjects with Attention-Deficit/Hyperactivity Disorder from nonclinical subjects. The DSM-IV^{™} Symptom Scales directly correspond to DSM-IV criteria for ADHD diagnosis with parent (CAD-P), teacher (CADS-T) and self-report (CADS-A) forms. Separate measurements for ADHD inattention, hyperactivity impulsivity, conduct disorder, oppositional defiant, and the global impression scale have been made after treatment with compositions described herein with the same results demonstrating improvement observed. A subject treated with such methods may exhibit an improvement in one or more of the attributes of the Conners' DSM-IV Scale according to conventional scoring of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

The compositions disclosed herein reduce the occurrence of diarrhea in at least the population of autistic subjects and most likely for subjects overall for certain types of gastrointestinal disturbances. Disclosed herein but not claimed is a method for reducing the occurrence of diarrhea in subjects, comprising administering to a subject in need thereof a composition described herein. The child may be autistic. The child may not be autistic Administration of the present compositions may reduce the occurrence of diarrhea by at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

The majority of subjects with autism universally suffers from a protein mal-absorption syndrome, and thus undernourished. The present inventors have identified that a pretreatment FCT level of 15.5 mean (frozen) across the entire study population indicated that subjects are universally suffering from a protein mal-absorption syndrome and, thus, are under-nourished. This includes all subjects that are above the 12.6 units/gram (frozen) of chymotrypsin cutoff for the study.

The compositions described herein also were observed to improve diet and weight in subjects. As subjects approached reasonably normal levels of chymotrypsin, the stronger the increases in balanced food consumption, protein intake, vegetable intake, meat intake, cholesterol, vitamin K, calcium, improvements in glycemic load, and improvements in ABC irritability, social withdrawal / lethargy and hyperactivity.

Disclosed herein but not claimed is a method of normalizing chymotrypsin levels in a subject, comprising administering to a subject in need thereof a composition described herein. Such treatment may increase balanced food consumption, protein intake, vegetable intake, meat intake, cholesterol, vitamin K, calcium, improvements in glycemic load. Such treatment may improve ABC irritability, social withdrawal / lethargy and hyperactivity.

At a terminal FCT level of 6.0 Units/gram (fresh) or 9.0 (frozen) of feces or greater, the present inventors identified that numerous minerals and vitamins improve following treatment: plant-based vitamin A, Carotenoids (alpha and beta carotene), vitamin K, vitamin E, vitamin C, Selenium, copper, food folate, lutein, lycopene, magnesium, moisture content of foods, potassium, phosphorus, sodium, polyunsaturated fatty acids, monounsaturated fatty acids, saturated fats, cholesterol, along with a beneficial decrease in Theobromine. Calcium and Iron (both a positive 2 charge ions), and animal sources of vitamin A, were not observed to improve.

In general, subjects treated with a composition described herein and which subjects had a level of 40 milligrams or greater of daily copper daily intake and at the same time an FCT level of 6.0 Units/gram (fresh) or 9.0 (frozen) or greater of feces were observed to have an improvement in four ABC scales: ABC stereotypical behavior, ABC hyperactivity, ABC irritability, ABC lethargy / social withdrawal.

In general, subjects treated with a composition described herein and which had a level of 2000 micrograms or greater daily intake of lycopene, daily intake and at the same time a lutein daily intake level of 500 micrograms or greater improvement is seen in four ABC scales: ABC stereotypical behavior, ABC hyperactivity, ABC irritability, ABC lethargy / social withdrawal.

In general, subjects treated with a composition described herein and which had a level of 40 micrograms or greater daily intake of selenium have daily intake improvement in four ABC scales: ABC stereotypical behavior, ABC hyperactivity, ABC irritability, ABC lethargy / social withdrawal.

In general, with an increase in alpha and simultaneously beta carotene improvement is seen in four ABC scales: ABC stereotypical behavior, ABC hyperactivity, ABC irritability, ABC lethargy / social withdrawal after treatment with a composition herein (data not shown).

The glycemic index (GI) is a measure of the effects of carbohydrates on blood sugar levels. Carbohydrates that break down quickly during digestion and release glucose rapidly into the bloodstream have a high GI; carbohydrates that break down more slowly, releasing glucose more gradually into the bloodstream, have a low GI. A lower glycemic index suggests slower rates of digestion and absorption of the foods' carbohydrates and may also indicate greater extraction from the liver and periphery of the products of carbohydrate digestion. A lower glycemic response may equate to a lower insulin demand in some cases and may improve long-term blood glucose control and blood lipids. The insulin index is also useful for providing a direct measure of the insulin response to a food.

The glycemic index of a food is defined as the area under the two-hour blood glucose response curve (AUC) following the ingestion of a fixed portion of carbohydrate (usually 50 g). The AUC of the test food is divided by the AUC of the standard (either glucose or white bread, giving two different definitions) and multiplied by 100. The average GI value is calculated from data collected in 10 human subjects. Both the standard and test food must contain an equal amount of available carbohydrate. The result gives a relative ranking for each tested food.

Conventional methods use glucose as the reference food, giving it a glycemic index value of 100 by definition. This has the advantages of being universal and producing maximum GI values of approximately 100. White bread can also be used as a reference food, giving a different set of GI values (if white bread = 100, then glucose ≈ 140).

GI values can be interpreted intuitively as percentages on an absolute scale and are commonly interpreted as follows:

| **Classification** | **GI range** | **Examples** |
|---|---|---|
| Low GI | 55 or less | most fruits and vegetables, legumes/pulses, whole grains, nuts, fructose |
| Medium GI | 56-69 | whole wheat products, basmati rice, sweet potato, sucrose, baked potatoes |
| High GI | 70 and above | white bread, most white rices, corn flakes, extruded breakfast cereals, glucose, maltose |

The present inventors have identified that, in general, in subjects treated with a composition herein and having a glycemic index of less than 55 and an FCT level of 6.0 Units/gram (fresh) or 9.0 (frozen) of feces or greater, an improvement is seen in four ABC scales after treatment: ABC stereotypical behavior, ABC hyperactivity, ABC irritability, ABC lethargy / social withdrawal (data not shown).

At a terminal FCT level of 6.0 Units/gram (fresh) or 9.0 (frozen) or greater in feces after treatment, there are increases in calorie intake, protein intake, meat, poultry, fish, fruit consumption, vegetable consumption, legume consumption, carbohydrate consumption along with a beneficial decrease in the glycemic index.

Decreases in glycemic index are highly correlated with improvements in lethargy as measured on the ABC scale. The glycemic index of a subject treated with a composition described herein may decrease by at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo. Furthermore, in a subject may experience a decrease in lethargy as measured on the ABC scale. Such a decrease may be about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95% ,or about 100% compared to a subject treated with a placebo.

Disclosed herein but not claimed is a method of improving the uptake of one or more vitamins and/or minerals in an autistic child, comprising administering to a subject in need thereof a composition described herein. A subject to be treated with such methods may be autistic. A subject to be treated may be female. A subject to be treated may be male. Uptake of one or more of plant-based vitamin A, Carotenoids (alpha and beta carotene), vitamin K, vitamin E, vitamin C, Selenium, copper, food folate, lutein, lycopene, magnesium, moisture content of foods, potassium, phosphorus, sodium, polyunsaturated fatty acids, monounsaturated fatty acids, saturated fats, cholesterol, and Theobromine may be improved by at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95% or about 100% compared to a subject treated with a placebo.

Disclosed herein but not claimed is improving the quality of diet of an autistic child by increasing thirteen essential vitamins and minerals: vitamins B6, B 12, A (plant sources), C, E, K, along with Copper, Iron, Cholesterol, Niacin, Riboflavin, Thiamin, and Zinc, by administering to the child a composition described herein. In studies conducted by the present inventors, the increases in these vitamins and minerals were statistically significant (data not shown).

The chymotrypsin biomarker can also be a good indicator of subjects with physiological malnutrition. Disclosed herein is but not claimed a method of monitoring physiological malnutrition in a subject comprising measuring chymotrypsin concentrations in one or more body fluids or tissues and comparing those levels to a baseline concentration or to levels in one or more healthy subjects.

All autistic subjects at baseline were observed to have a lower overall caloric intake compared to subjects age-matched either subjects with a sedentary life style or an active life style. Disclosed herein but not claimed is a method of improving caloric intake in an autistic subject, comprising administering to a subject in need thereof a composition described herein. A subject treated with such methods may exhibit improved caloric intake of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

Compositions described herein can be used to prevent or reduce the number of fractures in subjects with abnormal flora in their guts (such as autistic subjects), and in other subject populations such as, for example, subjects who take antibiotics, who have Crohn's disease, and who have a small and large intestinal disorder that results from damage inflammation necrosis or disease. Prevention or reduction of the number of fractures can be at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

Increased clotting disorders in autistic and other subjects has been observed due to deficiency in vitamin K and deficiency in serotonin transport from platelets. Disclosed herein but not claimed is a method of treating a clotting disorder in a subject, comprising administering to a subject in need thereof a composition described herein. Clotting may be modified by at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

Potential measuring levels of gamma carboxylated proteins in the blood as a measure of vitamin K deficiency can be a novel biomarker for autism and other mal-nutritional deficiencies. As subjects with autism demonstrated increased vitamin K intake, the carboxylation of certain glutamate residues can be essential to autism. Disclosed herein but not claimed is a method of decreasing vitamin K intake in autistic subjects, comprising administering to a subject in need thereof a composition described herein. A subject treated with such methods may exhibit decreased vitamin K intake of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

Also disclosed herein but not claimed is a method of decreasing carboxylation of glutamate residues, comprising administering to a subject in need thereof a composition described herein. A subject treated with such methods may exhibit decreased carboxylation of glutamate residues of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

It appears that subjects with autism possess an ongoing loss of overall caloric intake as well as the caloric intake of fat, protein, and carbohydrates. Enzyme replacement therapy reverses the loss of protein and fat caloric intake.

Disclosed herein but not claimed is a method of reversing protein loss and fat caloric intake in a subject, comprising administering to a subject in need thereof a composition described herein. A subject treated with such methods may exhibit decreased protein loss of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

In general, female subjects who are treated with a composition described herein who experience an increase in protein consumption, have an improvement in four ABC scales: ABC stereotypical behavior, ABC hyperactivity, ABC irritability, ABC lethargy / social withdrawal. Disclosed herein but not claimed is a method of increasing protein consumption in an autistic female child, comprising administering to a female individual in need thereof a composition described herein. A female individual treated with such methods may exhibit an improvement in four ABC scales of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a female individual treated with a placebo.

The present inventors identified that overall consumption of polyunsaturated fatty acids improved in a subject population treated with a composition described herein when study participants achieved a study level of FCT greater than 6.0 grams (unfrozen) or 9.0 units/ gram (frozen) in their feces (data not shown). Disclosed herein but not claimed is a method of increasing consumption of polyunsaturated fatty acids in a subject in need thereof, comprising administering to a subject a composition described herein. Consumption of polyunsaturated fatty acids may increase by at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

The present inventors also identified that subjects treated with compositions described herein exhibited an increased consumption of protein and fats, which seemed to correlate more favorably to improvements in irritability and hyperactivity on the ABC scale. Disclosed herein but not claimed is a method of increasing consumption of polyunsaturated fatty acids in a subject in need thereof, comprising administering to a subject a composition described herein. Consumption of protein and fats may increase by at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

It appears that Asians improved to an even greater extent for ABC, caloric, essentially everything overall. Disclosed herein but not claimed is a method of treating an Asia subject for improving one or more subscales of the ABC scale, improving caloric intake, preventing or reducing the number of fractures in subjects with abnormal flora in their guts (such as autistic subjects), and in other subject populations such as, for example, subjects who take antibiotics, who have Crohn's disease, and who have a small and large intestinal disorder that results from damage inflammation necrosis or disease, improve diet and weight, improve one or more of Connor's DSM-IV attributes, or any of the other conditions/attributes described herein, comprising administering to an Asia subject in need thereof a composition described herein.

Girls respond better to enzyme replacement therapy, as they tend to have more severe cases of autism. Disclosed herein but not claimed is a method of treating autism in a female individual, comprising administering to a female individual in need thereof a composition described herein. A female individual treated with such methods may exhibit a reduction in one or more symptoms of autism of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95% or about 100% compared to a female individual treated with a placebo.

In one embodiment, there can be a condition of protein calorie malnutrition in autistic subjects. The form of protein energy malnutrition in the autistic population is due to the selection of improperly balanced diet due to the self-restriction that results from inability to digest protein. Indicators for malnutrition in the autistic population that can serve as a partial marker for autism (which would be confirmed by additional confirmation by the ABC test or other markers) include, for example: one or more of Anthropometry (for example: height for age (*e.g.*, chronic malnutrition , stunting, *etc.*), weight for age (*e.g.*, protein energy malnutrition), weight for height (*e.g.*, acute malnutrition, wasting, *etc.*), middle upper arm circumference, demi or arm span, knee height, sitting height, skin fold thickness, head circumference, edema, and body mass index); deficiencies in essential vitamins and minerals (other than vitamin K); micronutrients; biochemical testing (e.g., Albumin, Prealbumin and/or Cholesterol); monitoring oral intake; other enzyme deficiencies such as elastase and trypsin along with other enzymes.

Subjects with autism that have enzyme replacement therapy have statistically significant reductions in whole grains as well as approximately a 20% reduced intake in overall carbohydrates as a result of the enzyme replacement therapy over a twelve week period. Disclosed herein but not claimed is a method of reducing whole grains and/or intake of carbohydrates, in an autistic child, comprising administering to a subject in need thereof a composition described herein. A subject treated with such methods may exhibit a reduction in one or more symptoms of autism of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

The compositions described herein can be used to treat autistic subjects as it has been newly identified that many autistic subjects avoid eating protein. Overall protein intake doubles with enzyme replacement therapy and an approximate 15% increase in fat consumption compared to subjects not treated with an enzyme over a 12 week period. Disclosed herein but not claimed is a method of increasing protein intake in a subject, comprising administering to a subject in need thereof a composition described herein. A subject treated with such methods may exhibit a reduction in one or more symptoms of autism of at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo.

In one embodiment, subjects having a history of allergies and treated with enzyme replacement therapy had a larger intake of fiber, calories, fat, protein, and carbohydrates than autistic subjects treated with enzyme replacement therapy that did not have allergies. The methods described herein can be modified as needed depending upon whether or not a subject to be treated has allergies.

In autistic subject given enzyme replacement therapy, the increased caloric intake did not result in an abnormal increase in weight due to the overall improvement in the quality of their diets. The increase in quality of their diets had statistically significant changes in the thirteen essential vitamins and minerals which are vitamins B6, B12, A, C, E, and K, along with Copper, Iron, Cholesterol, Niacin, Riboflavin, Thiamin, and Zinc. Thus, a subject treated with any of methods described herein may exhibit an improvement in caloric intake without causing an abnormal increase in weight or obesity.

Compositions described herein can be administered with one or more additional agents. For example, disclosed herein but not claimed is a method of treating autism in a subject, comprising administering to a subject in need thereof a composition described herein with glutamate enhancing therapy.

A subject to be treated with a method described herein includes a subject that is between about 1 and about 25 years of age. A subject may be from about 5 years to about 20 years of age, from about 2 years to about 10 years of age, from about 3 years to about 8 years of age, from about 10 years to about 15 years of age, from about 10 to about 20 years of age, or any range therebetween.

Disclosed herein but not claimed is a method for monitoring the fecal chymotrypsin level to select dosing of a composition herein for treatment of subjects with Autism. The concentration of fecal chymotrypsin may be measured using any conventional assay. The method may include measuring the concentration of chymotrypsin in a sample obtained from an autistic child, comparing the concentration to one or more control values, and determining if additional treatment is needed. In one embodiment, a control concentration may be that of a subject known to not be autistic. In another embodiment, a control concentration may be that of a subject known to be autistic.

Fecal chymotrypsin level may be used to titrate dosage in order to tailor dosing for an optimal subject response by assessing the values of fecal chymotrypsin and the deltas between the concentration identified in a subject compared to the one or more controls (*i*.*e*., difference between baseline and termination).

Dosing may be adjusted based on one or more of the following: weight, baseline chymotrypsin level, instantaneous chymotrypsin level, time averaged chymotrypsin level, change in chymotrypsin level, change in chymotrypsin level per unit time, rate of change of chymotrypsin level per unit time (2nd derivative), cumulative dose to date, time averaged dosing over a given time period, rate of change of dosing against rate of change in chymotrypsin level, derivative of rate of change of dosing against rate of change in chymotrypsin level.

Fecal chymotrypsin levels may be determined on an hourly, daily, weekly, biweekly, monthly, bi-monthly or yearly basis.

Other enzymes secreted in stools that may be equivalent or even less efficacious markers may be also be measured in order to assess a subject's state and determine the best course of treatment.

The present inventors have identified that, the more severe the autism diagnosed, the lower the baseline fecal chymotrypsin level is found.

Furthermore, the lower the baseline fecal chymotrypsin level, the greater improvement is observed in the fecal chymotrypsin level and a corresponding greater improvement in the disease following treatment with a composition described herein (data not shown).

In one embodiment, a terminal FCT level of 6.0 Units/gram (fresh) or 9.0 (frozen) or greater of feces along with an FCT 3.5 or greater from baseline determination indicates an improvement in the subject being treated.

In one embodiment, the level of hyperactivity and other symptoms of ADHD can be determined by the Conners-3 test.

In yet another embodiment, the Conners-3 subscales for hyperactivity can be compared to those on the ABC. In yet another embodiment, the Conners scale can be utilized to determine if a change in behavior has occurred in subjects with ADHD and autism as hyperactivity is a co-morbid symptom found in autism.

In another embodiment, the attributes that can be measured utilizing the Conners-3 in subjects with autism are: restless or overactive behavior; hyperactivity; excitability and impulsiveness; failing to finish tasks; inattentiveness and ease of distraction; temper outbursts; fidgeting; disturbances of other subjects; demands to be met immediately and ease of frustration; ease and frequency of crying; and rapid and drastic mood changes. Each attribute is scored on a scale of 0-3 where 0 = never, seldom; 1 = occasionally; 2 = often, quite a bit; and 3 = very often; very frequent.

In one embodiment, the comparison of change in intake of carbohydrates as measured by the use of the block food screener and the Conners test for change in hyperactivity demonstrate that at all levels of carbohydrate intake change the subjects administered a composition described herein improved in their Conners scores over subjects administered the placebo over the course of 12 weeks.

In yet another embodiment, the changes the improvement in hyperactivity in subjects administered the enzyme composition may be due to the improvement in carbohydrate digestion.

In yet another embodiment, the changes the improvement in hyperactivity in subjects administered the enzyme preparation may be due to the improvement in protein digestion as evidenced by an improvement in the protein components of the carbohydrates.

In yet another embodiment, the enzyme preparation contains proteases, amylases, and lipases, thus, subjects administered the enzyme composition demonstrate improvement over subjects who are not administered the enzyme composition.

In one embodiment, the comparison of change in intake of carbohydrates as measured by the use of the block food screener and the Conners test for change in inattention demonstrate that at all levels of carbohydrate intake change the subjects administered a composition described herein improved in their Conners scores over subjects administered the placebo over the course of 12 weeks. In yet another embodiment, the changes the improvement in inattention in subjects administered the enzyme preparation may be due to the improvement in carbohydrate digestion. In yet another embodiment, the changes the improvement in inattention in subjects administered the enzyme preparation may be due to the improvement in protein digestion as evidenced by an improvement in the protein components of the carbohydrates.

In yet another embodiment, the enzyme preparation contains proteases, amylases, and lipases, thus those administered the enzyme demonstrated improvement over subjects who are not administered the enzyme. In yet another embodiment, a decrease in inattention was seen in subjects not administered the enzyme as their carbohydrate intake increases. In subjects administered a placebo, the inattention increases as the carbohydrate intake increases. In yet another embodiment, the increase in carbohydrate intake without the concomitant replacement of enzyme, allows for the worsening of the inattention, most likely due to the inability to digest the protein in the carbohydrates (gliadin). In yet another embodiment, as carbohydrate consumption increases, it was observed that inattention decreases in those administered Formulation 1, and was observed to increase in subjects not administered the medication (e.g., a composition of Formulation 1). In yet another embodiment, the increase in carbohydrate intake without the concomitant replacement of enzyme, allows for the worsening of the inattention, most likely due to the inability to digest the protein in the carbohydrates (gliadan).

In yet another embodiment, the PPVT and the EVT tests were both affected by the amount of protein intake following administration of a composition described herein. The PPVT and the EVT growth score changes are the measure of the amount of overall change in receptive and expressive language adjusted for the age of the child.In one embodiment, the growth scores for both the EVT and the PPVT increased, demonstrating improvement in subjects administered the enzyme preparation. In yet another embodiment, at the end 12 weeks the measure of both the PPVT and the EVT growth scores of those administered a composition described herein remain significantly better than subjects administered the placebo. In yet another embodiment, in both the EVT and the PPVT, subjects administered the placebo exhibit a worsening in their scores as the amount of protein subjects ingest per day at week 12 increases. In yet another embodiment, an improvement in scores is seen in the PPVT in subjects administered a composition described herein as the amount of protein that is ingested per day at week 12 increases. In yet another embodiment, the EVT the scores remain constant demonstrate large improvements across all levels of protein intake per day at week 12. In yet another embodiment, the instant findings represent the administration of a high protease formulation where at week 12 a significant improvement in growth scores in both the PPVT to the EVT can be demonstrated. Improvement in any one of the symptoms or outcomes may be measured according to any of the tests described herein or conventionally accepted in the art. For example, improvement may be at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% compared to a subject treated with a placebo (placebo). In another example, improvement may be at least about 2-fold, about 3-fold, about 5-fold, about 10-fold, about 15-fold, about 20-fold, about 25-fold, about 30-fold, about 35-fold, about 40-fold, about 45-fold, about 50-fold, about 55-fold, about 60-fold, about 65-fold, about 70-fold, about 75-fold, about 80-fold, about 85-fold, about 90-fold, about 95-fold, or about 100-fold compared to a subject treated with a placebo (placebo).

### EXAMPLES

The invention is defined by the claims. Any of the following examples that do not fall within the scope of the claims do not form part of the invention but are provided for comparative or informative purposes only.

The following experiments describe exemplary procedures in accordance with the invention. It is to be understood that these experiments and corresponding results are set forth by way of illustration only. By way of example, these studies demonstrate some of the unexpected improvements realized by the exemplary encapsulated enzyme preparations of the present disclosure.

### EXAMPLE 1: Increased flow properties and pourability of an exemplary encapsulated digestive enzyme preparation

Before the exemplary methods and preparations of the present disclosure is applied, examination of an unprocessed, raw enzyme preparation (Scientific Protein Laboratories (SPL) of Wanakee, WI) revealed that it contained significant variability in particle size and 20 irregular morphology, as shown in an electron micrograph of the particles (data not shown). Some crystalline salt particles are also visible. The raw enzyme does not pour as it clumps and is difficult to measure due to the uneven surfaces, and jagged edges. The raw preparation is also not suitable for lipid encapsulation without further processing because the raw product contains particles both too large and too small for proper encapsulation. The sieved enzyme while more uniform in size, continues to exhibit uneven surfaces and clumps while pouring.

The coated enzyme preparation may be produced following sieving and lipid coating of the raw material (data not shown). In this example, the morphology of particles is significantly improved, with rounder surfaces. This leads to a non-dusty product with good flow and organoleptic properties.

The morphology of the enzyme is now greatly improved due to the rounding of the surfaces, which leads to a product which is less dusty, does not aerosolize and has good flow and improved organoleptic properties.

The size distribution of particles in the raw enzyme preparation is determined. In general, large particles (>40 USSS mesh, 425µm) and very small particles (<140 USSS mesh, 105µm) are not suitable for proper encapsulation. In order to increase the flow properties of the encapsulated pancreatic enzyme preparation, the raw enzyme particles were sieved to include only particles of sizes 40-140 USSS mesh (425µm to 105µm), or about 105 to 425 microns.

### EXAMPLE 2: Stability of an exemplary encapsulated digestive enzyme preparation: temperature storage

In a further example, multiple types and weight percentages of lipids were used to coat the sieved enzyme cores. Properties including mechanical strength, melting point, and hydrophobicity were taken into consideration in choosing a suitable lipid coating for the pancreatic enzyme. Multiple examples of lipid coatings were examined below and their physical appearances were examined under 25° C and at 40° C. Accordingly, lipids with a range of physical properties such as mechanical strength, melting point and hydrophobicity were evaluated for coating of the pancreatic enzymes. In this example, it was found that the decreasing the melting point or increasing the hydrophilicity of the coatings were not suitable for encapsulation because they resulted in product that would cake under accelerated storage stability conditions. The sieved and encapsulated enzyme preparations made using hydrogenated soy oil, hydrogenated castor wax, and carnauba wax all demonstrated good pouring and no caking.

Both the hydrogenated monoglycerides and the soy oil / monoglyceride blends demonstrated caking at the higher temperature. Therefore, it is clear that the lower melting or more hydrophilic coatings were not suitable for encapsulation because they resulted in a product that would cake under extended storage conditions as evidenced by our accelerated storage condition test at 40 degrees Centigrade.

Both the hydrogenated monoglycerides and the monoglyceride blends demonstrated caking at the higher temperature. Therefore, it is clear that decreasing the melting point or increasing the hydrophilicity of the coatings were not suitable for encapsulation because they resulted in a product that would cake under extended storage conditions as evidenced by our accelerated storage condition test at 40 degrees Centigrade.

### EXAMPLE 3: An exemplary encapsulated digestive enzyme preparation suitable for pancreatic enzymes: enzyme activity measured as a function of stability.

In a further example, enzyme stability was determine according to the following method: for the accelerated test, standard ICH guidelines were used: the coated preparations were placed in a plastic container, which was stored in a controlled humidity cabinet at 40°C and 75% relative humidity. Enzymatic activity was measured by grinding the coated enzyme preparations, dispersing in appropriate buffers, and testing for lipase activity.

The soy oil 80% appeared to impart the greatest amount of stability of all the lipids, an effect that surprisingly was greater for enzyme preparations stored in capped containers than in uncapped containers. Tests of stability for 75% relative humidity enzyme preparations stored at 40°C in open pans did not show significant differences in stability between coated and uncoated preparations.

### EXAMPLE 4: An exemplary encapsulated digestive enzyme preparation suitable for pancreatic enzymes: enzyme activity and rate of release of multiple soy encapsulated pancreatic enzyme

In a further example, encapsulates were prepared according to the methods described below. The raw enzyme material was sieved to obtain particles smaller than 40 USSS mesh (425µm) but larger than 140 USSS mesh (105µm), to remove fines, and to obtain a more uniform mixture more suitable for enteric coating.

The following preparations were made: (1) 70% active enzyme by weight, with a standard stable soy coating; (2) 80% active enzyme by weight, with a standard stable soy coating; and (3) 90% active enzyme by weight, with a standard stable soy coating.

Activity in each encapsulated enzyme preparation was measured by grinding the encapsulates, dispersing the ground material in appropriate buffers, and testing for lipase activity.

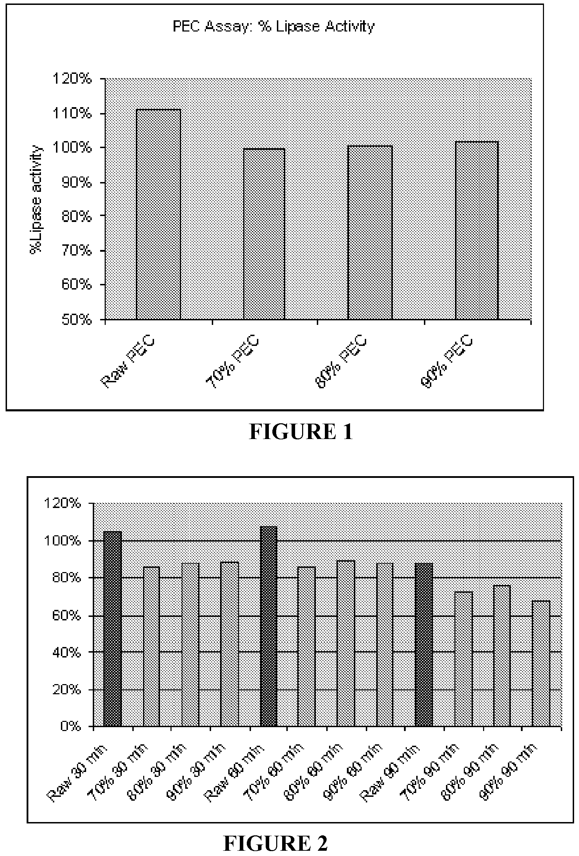

As shown in Figure 1, the enzyme activity in the coated preparations does not show any significant loss of activity upon coating (decrease from 110 to 100% activity, normalized to stated enzyme activity of the raw enzyme material).

Enzyme release was measured by suspending each encapsulate in a dissolution apparatus at pH 6.0 buffer for 30, 60, and 90 minutes (100 rpm, as per U.S.P. guidelines). As shown in Figure 2, all encapsulates show between 80-90% release at 30 and 60 minutes. At 90 minutes, the measured enzyme activity obtained with these preparations decreases.

### EXAMPLE 5: An exemplary encapsulated digestive enzyme preparation suitable for pancreatic enzymes: particle size of multiple soy oil encapsulated pancreatic enzyme

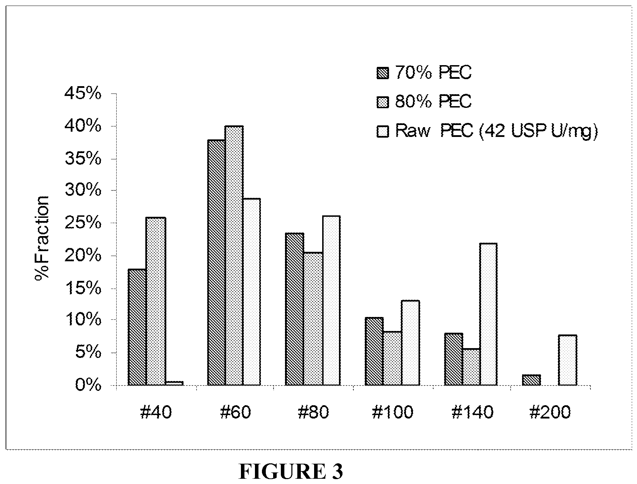

In a further example, preparations containing 70% or 80% active pancreatic enzyme by weight, encapsulated with soy oil were compared to raw pancreatic enzyme material with respect to particle size, as shown in Figure 3.

All levels of lipid demonstrate an impact of particle size. The 80% PEC demonstrates the most uniform as none appear at the 200 USSS mesh (75µm) level.

### EXAMPLE 6: An exemplary encapsulated digestive enzyme preparation suitable for pancreatic enzymes: smell and taste

Examination of exemplary encapsulated enzyme preparations containing 70%, 80% and 90% enzyme by weight was performed to determine their taste and smell when compared to SUCANAT^{®} and brown sugar, as well as compared to the raw enzyme. The results are shown in the table below. SUCANAT^{®} is an organic whole food sweetener.

| **SUBSTANCE** | **ODOR** | **TASTE** |
|---|---|---|
| Brown sugar | Yes | Sweet |
| SUCANAT^{®} | No | Sweet |
| Raw Enzyme | Meaty/smoky | N/A |
| 70% | No | No |
| 80% | No | No |
| 90% | Slight | Salty |

### EXAMPLE 7: An exemplary encapsulated digestive enzyme preparation suitable for pancreatic enzymes: manufacturing

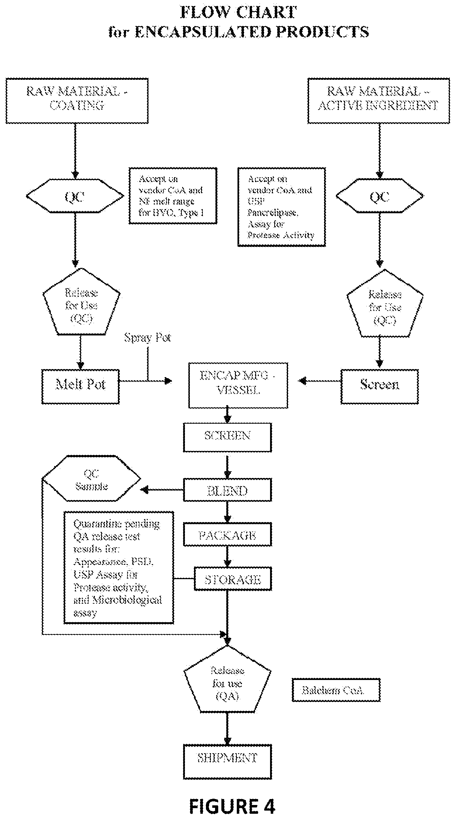

The flow chart outlining a manufacturing process useful in making an enzyme preparation is shown in Figure 4.

Ingredients used in making a batch of an exemplary encapsulated pancreatic enzyme preparation included 20.0 lbs of sieved pancreatic enzyme and 5.0 lb. of hydrogenated vegetable oil, for example, soy oil.

The pancreatic enzyme concentrate was first sieved through a #40 USSS mesh (425µm), screen and the material which passed through the mesh was retained. The retained material was then screened through a #140 USSS mesh (105µm) screen (or the equivalent), and the material which did not pass through the mesh was retained as the sieved pancreatic enzyme material or particles.

In the encapsulation process, the appropriate coating material is charged to the melt pot and brought to and maintained at 43.3°C (110° F) for the spraying process. Any temperature that will provide appropriate consistency during the spraying process can be used. In some example, the temperature is further selected based on the melting points of the lipids used in the coating, and/or so that after contact of the sieved pancreatic enzyme material or particles with the coating, the activity of the enzyme preparation remains about the same.

The liquefied coating material is weighed and transferred to the spray pot. The sieved pancreatic enzyme was added to the encapsulation manufacture vessel. The pancreatic enzyme particles are encapsulated with coating material to the selected coating level.

The encapsulated material is screened with a #14 USSS mesh (1400µm) screen (or equivalent), and the material that passes through the screen is retained. Following sieving, the material is collected and samples are removed for quality control (QC).

If two sub-batches are to be blended, the loaded screened material is added to a suitable blender and blended for 7 to 10 minutes. Samples are obtained for finished product testing. The encapsulated material is bulk packaged and placed in quarantine pending test results. Upon achieving acceptance criteria, the finished product is released by the Quality group. Afterwards, the product can be shipped as directed.

Samples are collected for finished product testing, including analytical testing and microbial assays, which can be tested over time.

### EXAMPLE 8: An exemplary encapsulated digestive enzyme preparation suitable for pancreatic enzymes: packaging

In yet another further example, the stability of the enzyme is due in part to the encapsulation and in part to the trilaminar foil packaging. The following demonstrates the packaging process for the single dose sachets/pouches.

First, following manufacture the product is dispensed into clean, drums double lined with food-grade polyethylene bags, and the drums are sealed. If specification criteria are met, the lot is then released from quarantine, and the material is then shipped to a suitable packager for placement into sachets for individual dosing to a subject.

For example a PD-73272 Printed Child Resistant (CR) Pouch consisting of 26# C1S Paper/ 7.5# LDPE/.0007" Aluminum Foil/15# with a Surlyn liner is utilized for packaging. Preferably, pre-printed film/foil, exterior printing will be with 1 color eye-mark on white background while in-line printing of lot number, expiration date and product code will also be in 1 color, black. Overall sachet dimension are: W 2.50" x H 3.50". The sachet is sized to hold 900 mg of granules of Pancreatin lipid-encapsulated drug product with a tolerance of ±10% into a unit dose pouch/sachet. The final product will have a protease activity of not less than 156 U.S.P. units/mg.

### EXAMPLE 9: An exemplary encapsulated digestive enzyme preparation suitable for pancreatic enzymes: dissolution

The effect of the release of pancreatic extract complex from lipid encapsulated particles with soy oil was studied using particles with varying levels of lipid coating (expressed as % lipid coating per total particle weight. The coating level was varied from 10% to 30%. There was no significant effect of lipid coating in this range on the release of pancreatic extract complex in an aqueous environment from the particles over a 60-minute period. All formulations release over 80% of the enzyme within the first 30 minutes following the initiation of dissolution. Maximum release for the 90%, 80% and 70% particles was 85%, 88%, and 83% respectively by 60 minutes.

The choice of 70% -90% encapsulated pancreatic enzyme preparation (active enzyme by weight) was selected on the basis of its release profile, as suitable for release of the enzyme in the proximal small intestines where protein digestion by the protease component will take place.

Soy oil was selected as the lipid coating, for its lack of protein components, and corresponding lack of antigenic properties, to minimize or eliminate the possibility of an allergic reaction to the lipid coating in treated subjects and subjects with autism.

The use of the 70-90% preparation increases pourability and flow properties while decreases aerosolization, which permits use of a sachet or pouch delivery system.

The addition of the trilamminar foil housing ensures that the sprinkle formulation will be stable, transportable, and will be delivered by a single unit dose mechanism.

The low lipase formulation allows also for the safety by reducing the potential for colonic strictures, and enhances the utilization of the protease portion of the formulation.

### EXAMPLE 10: Biochemical biomarkers, and behavioral core and non-core symptoms

The correlation between digestive enzyme deficiencies in autistic subjects was determined in subjects diagnosed with autism based on clinical (behavioral) symptoms. This correlation was also studied in subjects diagnosed with autism and a genetic co-morbidity. Following the initial discovery that autistic subjects exhibited self-imposed protein dietary restrictions, studies were conducted which indicated that abnormally low levels of fecal chymotrypsin (FCT) is useful as a biomarker for autism.

Fecal chymotrypsin may be assessed using conventional methods including, but not limited to those described herein, and commercially available kits (e.g., Monotest Chymotrypsin; Boehringer, Mannheim).

Infant feces are collected in a manner to keep them free from urine contamination and mixed with water to obtain a weight by weight (w/w) mixture (e.g., 1:4 w/w). This mixture is then mixed thoroughly to obtain a homogeneous suspension by homogenization or sonication. The feces are then diluted with a reaction buffer, described below, to obtain a fecal concentration which, when added to a protease substrate, hydrolyzes the substrate over a 5 to 60 minute period. Using such a method, for example, fecal chymotrypsin may be measured.

For the fecal chymotrypsin test, a stool sample is collected from a subject. Each stool sample can be analyzed using an enzymatic photo spectrometry analysis to determine the level of fecal chymotrypsin in the stool; in some cases the assay is performed at 30° C, *see*, *e.g*.: U.S. Pat. No. 6,660,831. Alternatively, other methods, such as the colorimetric method, use of substrates, use of assays, and/or any other suitable method may be used to measure the fecal chymotrypsin levels. The levels of fecal chymotrypsin in the samples of a subjects suspected of or diagnosed as having autism are compared to the levels of fecal chymotrypsin in subjects not suspected or diagnosed with autism to determine if the tested subjects exhibit lower fecal chymotrypsin values and to determine if a subjects would benefit from the administration of a composition as described herein.

In addition, the number of autistic subjects responding to pancreatic enzyme replacement was also determined, based on biomarker measurements and clinical symptoms. Changes in the gastrointestinal system as well as a change in the core symptoms of autism were examined.

Initial observations were based on observation of self-imposed dietary restriction by almost all subjects with autism. Multiple studies were then conducted to evaluate the ability of autistic subjects to digest protein. A study of the physiology of protein digestion led to an examination of the gastrointestinal system's cascade of digestive enzymes, especially those involved in protein degradation, such as chymotrypsin. As a measure of dysfunction, it was determined that fecal chymotrypsin (FCT) levels in subjects suffering from autism were abnormally low.

### Study 1

This initial study was an exploratory one to determine if a small cohort of subj ects with autism indeed would have abnormally low levels (<9.0) of fecal chymotrypsin (FCT).

All 9 subjects with autism evidenced an abnormally low FCT level of below 7 Units/gram (Normal ≥ 9.0). This observation in a small set of subjects led to further examination of the potential for a physiological link to autism heretofore undiscovered.

### Study 2

Study 2 was undertaken to determine if a larger cohort of subjects (26 subjects) with autism also experienced abnormally low FCT levels. Levels of fecal elastase-1, another pancreatic digestive enzyme present at low amounts in pancreatic insufficiency, were also determined. Again, the levels of FCT were abnormally low in 25 of the 26 subjects, falling at 8 U/g or below. One child had an FCT level of 9 U/g. On the other hand, all of the subjects had normal levels of fecal elastase-1.

### Study 3

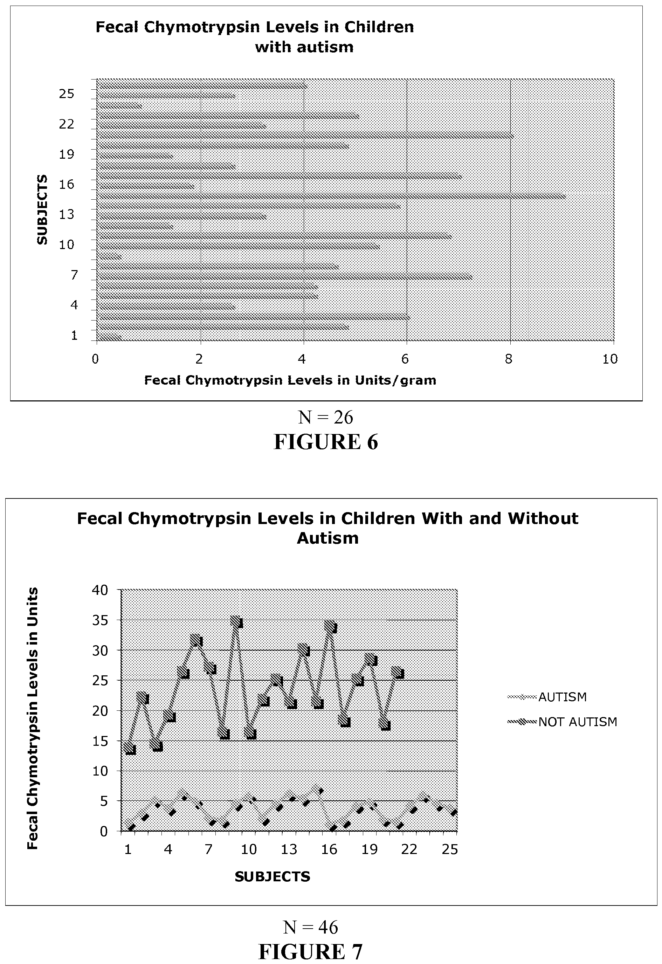

In Study 3, FCT levels were determined in 46 subjects aged 2 years to 14 years of age, 25 with autism and 21 without autism, The data demonstrated that subjects with autism had abnormally low FCT levels and subjects who did not have autism had normal FCT levels, of 12 U/g or higher. The results are summarized in Figure 7. The top line in Figure 7 shows the FCT levels in subjects who did not have autism, while the bottom line shows the FCT levels in subjects who did have autism.

### Study 4

In Study 4, FCT levels were determined for 463 subjects aged 2 years to 8 years of age, 266 diagnosed with autism and 197 diagnosed without autism, in a multi-office physician-conducted study. The data showed that the subjects with autism had abnormally low fecal chymotrypsin levels and subjects who did not have autism had normal levels of fecal chymotrypsin.

The data is summarized in the table below.

### Mean Fecal Chymotrypsin Levels in Subjects with and without Autism

| **N= 463** | **Subjects with Autism** | **Subjects not with Autism** |
|---|---|---|
| Total numbers of subjects | 266 | 197 |
| Mean FCT (U/g) | 4.4 | 23.2 |
| Total Subjects with Abnormal Levels of FCT | 203 | 3 |
| % (p<0.001) | 76.34% | 1.50% |
| Total Subjects with Normal Levels of FCT (p <0.01) | 63 | 194 |
| % | 23.68% | 98.50% |

Chymotrypsin is a pancreatic enzyme. Chymotrypsin is a serine protease and is unique in that it cleaves only essential amino acids during the digestive process. Specifically, chymotrypsin cleaves the peptide bond on the carboxyl side of aromatic amino acids. A lack of protein digestion as evidenced by abnormal FCT levels leaves the child with a dearth of amino acids available for new protein synthesis. Without sufficient levels of essential amino acids, new proteins required for various bodily functions cannot be synthesized. For example, a shortage or lack of proteins involved in neurological processes can then give rise to symptoms of autism.

Chymotrypsin cleaves specific amino acids which are not cleaved by the other protease. In specific it cleaves the essential amino acids: tryptophan, methionine, phenylalanine, and leucine, and the semi essential amino acid tyrosine. The two other major proteases do not cleave these essential amino acids and therefore the lack of chymotrypsin activity in the small intestine regardless of why it is low will leave a subject with a lack of these amino acids. Other very low by volume proteases carboxypeptidase A and B cleave minute amounts of some of these amino acids, but not sufficient quantities to make up the difference.

### Study 5

In Study 5, FCT levels were determined for 320 subjects aged 2 years to 18 years of age, 64 with autism, 64 with ADD. 64 with ADHD, 64 with known genetic conditions, and 64 normals (no known conditions). The data showed that the subjects with autism, ADD and ADHD exhibited abnormally low levels of FCT compared to the subjects with known genetic conditions and normal subjects. FCT data were gathered during a multi-physician office trial of age-matched subjects with multiple conditions. Figure 8 depicts FCT levels in separate groups of subjects aged 6 years to 18 years who have Autism, ADHD (Attention Deficit Hyperactivity Disorder), ADD (Attention Deficit Disorder), known genetic disorder also diagnosed with autism, or no known condition (normals).

The two upper lines in Figure 8 correspond to FCT levels in subjects without any known condition and subjects with known co-morbid conditions (genetic and others). The three bottom lines correspond to FCT levels in the subjects with autism, ADD and

### ADHD.

The Autism, ADD, and ADHD subjects had significantly lower levels FCT than subjects without any known condition, or subjects with a known genetic co-morbidity or traumatic condition (p <0.01).

### Study 6

In Study 6, 42 age-matched subjects, 25 with autism, and 17 without autism or other co-morbid condition, were examined using a stool test for the presence of multiple pathogens as well as markers of gastrointestinal dysfunction, including FCT levels. The subjects with autism had a larger number of stool pathogens present as well as abnormally low FCT levels.

This small pilot study was undertaken to examine the gastrointestinal flora of subjects with autism versus subjects without autism. Multiple markers of gastrointestinal health were examined to determine if there is an abnormal gastrointestinal presentation in these subjects.

Forty two (42) subjects aged matched 25 with autism and 17 without autism or other co-morbid condition were screened using a stool test for the presence of multiple pathogens as well as markers of Gastrointestinal dysfunction. Other GI pathogens or stool markers known to those of skill in the art can also be tested as a marker of GI dysfunction. The table below shows the incidence of presence of a GI pathogen or other stool marker.

| **Incidence of the Presence of Pathogens and other Stool Markers Representing Gastrointestinal Dysfunction** | | | | |
|---|---|---|---|---|
| | AUTISM | % TOTAL | NOT AUTISM | % TOTAL |
| LOW FCT | 25 | 100% | 0 | 0% |
| *C. difficile* antigen | 15 | 60% | 1 | 6% |
| Fecal Elastase <200 | 0 | 0% | 0 | 0% |
| *H. pylori* antigen | 17 | 67% | 0 | 0% |
| *E. histolytica* antigen | 8 | 32% | 0 | 0% |
| *Giardia* antigen | 9 | 36% | 1 | 6% |
| Yeast overgrowth | 4 | 16% | 0 | 0% |
| *Cryptosporidium* | 9 | 36% | 1 | 6% |
| | N=25 | | N= 17 | |

The presence of positive stool markers in the subjects with autism, including low levels of fecal chymotrypsin indicated additional gastrointestinal problems in subjects with autism.

### Example 11: Randomized Double Blind Placebo Controlled Trial of high Protease Enzyme Formulation in Subjects With Autism

Subjects to be treated (Formulation 1 or placebo) were diagnosed with autism. Subjects were administered Formulation 1 (comprising pancreatin) or a placebo as a powder taken three times a day with food.

The clinical trial was an interventional study with parallel assignment that was randomized. Primary outcome measures included evidence of changes in behavior scales and physical symptoms of autism at baseline, 14 days, 30 days, 60 days, and 90 days after treatment commenced.

Secondary Outcome Measures: other key measures of behavior and quality of life associated with autism were assessed at baseline, 14 days, 30 days, 60 days, and 90 days after treatment commencement.

One hundred eighty two subjects were enrolled in the study. The two arms of the study were as follows:

| **Arms** | **Assigned Interventions** |
|---|---|
| Active | Drug: FORMULATION 1 |
| Comparator: 1 | Single unit dose powder of active substance (Formulation 1) administered 3 times per day for 90 days |
| Formulation 1 | |
| Placebo | Drug: Placebo |
| Comparator: 2 | Single unit dose powder of non-active substance administered 3 times per day for 90 days |
| Placebo powder | |

### Eligibility

Subjects seeking treatment were administered questionnaires and tests to determine the nature and severity of autism.

| **Ages Eligible for Study:** | **3 years to 8 years of age** |
|---|---|
| Genders Eligible for Study: | Both |
| Accepts Healthy Volunteers: | No |

Inclusion criteria for subjects to enroll in the study required that the subject met the current Diagnostic and Statistical Manual for Mental Disorders (DSM-IV-TM) diagnostic criteria for autistic disorder (AD).

Exclusion criteria for subjects to be excluded from the study included the following parameters: **1.** Subject weighing less than (<) 11 kg (24.2 lbs.); **2.** Demonstrated previous allergy to porcine (pork) products; **3.** Previous history of severe head trauma or stroke, seizure within one year of entering study or uncontrolled systemic disease; **4.** Diagnosis of: HIV, cerebral palsy, endocrine disorder, pancreatic disease; **5.** Within 30 days of starting the study, certain supplementation, chelation or dietary restriction (a 30 day washout period would be required for inclusion); **6.** Use of any stimulant medication must be discontinued 5 days prior to entering the study; and **7.** Subject must have a stable dose of SSRI's for at least 30 days.

Locations: The clinical trials were conducted at 19 locations throughout the United States.

### Example 12: Clinical Trial of Formulation 1 in Subjects with Autism

Autism is currently a significant cause of disability in the pediatric population. Formulation 1 is based upon the observation that many subjects with autism do not digest protein. Formulation 1 is an enzyme composition that is designed as a powder taken three times a day. Formulation 1 is a composition as described as in Example 8, where the composition comprises a soybean oil coated pancreatin granule comprising at least 156 U.S.P. units per mg. The composition is administered in U.S.P. Units per 900 mg dose. It is formulated to be released in the small intestine to enhance protein digestion thus increasing the availability of essential amino acids. The purpose of this study is to determine efficacy of Formulation 1 in treating the core symptoms of autism.

Subjects to be treated (Formulation 1 or placebo) are diagnosed with autism. Subjects are administered Formulation 1 or a placebo.

The clinical trial is an interventional study with parallel assignment that was randomized. The endpoint classification assessed during the trial is to determine treatment efficacy of the subjects with Formulation 1.

Primary outcome measures to be assessed include evidence of changes in behavior scales associated with the core symptoms of autism at baseline, 4, 8, 12, 16, 20, 24, 36, 60, 72, 84, 96, 108, 120, 132, 144, 156, 168, and 180 weeks of treatment.

Secondary outcome measures to be assessed include other key measures of behavior and quality of life associated with autism at baseline, 4, 8,12,16, 20, 24, 36, 48, 60, 72, 84, 96, 108, 120, 132, 144, 156, 168, and 180 weeks of treatment.

One hundred seventy (170) subjects are enrolled in the study and treated according to the following arm:

| **Arms** | **Assigned Interventions** |
|---|---|
| Experimental: 1 | Drug: Formulation 1 |
| Formulation 1 | Single unit dose powder of active substance (Formulation 1) administered 3 times per day for 90 days |

### Eligibility

Subjects seeking treatment undergo a questionnaire and tests to determine the nature and severity of autism.

| **Ages Eligible for Study:** | **9 years to 12 years of age** |
|---|---|
| Genders Eligible for Study: | Both |
| Accepts Healthy Volunteers: | No |

Inclusion criteria for subjects to be included from the study included the following parameters**: 1.** Meets the current Diagnostic and Statistical Manual for Mental Disorders (DSM-IV-TR) diagnostic criteria for autistic disorder (AD). **2.** Ongoing 00102 Protocol requires completion of 00101 Protocol. **3.** Now recruiting subjects directly into 00102 Protocol.

Exclusion criteria for subjects to be excluded from the study included the following parameters: **1.** Ongoing study required subjects to be 3-8 years old weighing < 11 kg (24.2 lbs.), and achieving ages 9-12 years old weighing < 22 kg (48.4 lbs.). **2.** Newly recruited subjects must be between ages 9-12 years old weighing < 22 kgs (48.4 lbs.). **3.** Demonstrated previous allergy to porcine (pork) products. **4.** Previous history of severe head trauma or stroke, seizure within one year of entering study or uncontrolled systemic disease. **5.** Diagnosis of: HIV, cerebral palsy, endocrine disorder, pancreatic disease. **6.** Within 30 days of starting the study, certain supplementation, chelation or dietary restriction (a 30 day washout period would be required for inclusion). **7.** Any use of psychotropic medications, stimulants, or SSRI's must be discontinued for 30 days prior to entrance.

Locations: clinical trials will be conducted at 19 locations throughout the United States.

### Study 1A

Two hundred ninety eight (298) subjects were screened for autism by the DMS-IV criteria as well as by the SCQ and the ADI-R diagnostic tests. Once they were determined to have autism, they were tested for their fecal chymotrypsin levels.

Once they were determined to have autism by the above aforementioned tests, and found to have low FCT levels (<12.6 U/g of frozen stool) they were randomized and assigned to one of two parallel treatment groups: one administered the high protease enzyme composition (Formulation 1) as described herein ,or a placebo.

Ongoing study required subjects to be 3-8 years old weighing >11 kg (24.2 lbs.).

Formulation 1 or placebo was administered three times a day with food. Formulation 1 is described herein as a high protease enzyme composition (the medication).

One hundred eighty-two (182) subjects were randomized. The clinical trial is an interventional study with parallel assignment that was randomized and blinded. The endpoint classification assessed during the trial was undertaken to determine treatment efficacy of subjects with the high protease enzyme product described herein (the medication),versus subjects administered the placebo.

| **Group** | **FCT at Baseline** | **FCT at week 12** | **Change** |
|---|---|---|---|
| Formulation 1 | 7.46 | 10.94 | 3.47 |
| Placebo | 7.69 | 8.69 | 1.00 |
| P Value < | | | 0.0383 |

In this study, the changes from baseline between week 0 (baseline) and week 12 for fecal chymotrypsin levels were measured. Subjects administered the medication were supplemented with active chymotrypsin, and subjects who were administered placebo were not supplemented with chymotrypsin.

Figure 15 shows the changes in fecal chymotrypsin levels from baseline in subjects administered the medication versus subjects administered the placebo.

Chymotrypsin levels improved significantly over 12 weeks of replacement in the trial. The change of 1 demonstrated by the placebo is within the standard deviation of the test.

Subjects on placebo who did not receive chymotrypsin replacement did not improve significantly over the trial. A p value of <0.03 demonstrates significance over a 12 week period.

This demonstrated that the chymotrypsin was replaced in subjects who received the medication and that those who received placebo did not receive replacement.

### Study 2A

The same cohort of subjects as outlined in study 1A was used in Study 2A. Subjects were screened for autism. The medication or placebo was administered three times a day with food. The medication is described herein as a high protease enzyme (Formulation 1).

Stool changes in pH were measured in both subjects administered the medication and in subjects administered the placebo.

As an increase in pH (more alkaline) occurs it is pathagneumonic for an increase in protein digestion. As the maines present as a byproduct of the protein digestion are present it makes the stool more alkaline.

| **Group** | **FCT at baseline** | **FCT at week 12** | **Change** |
|---|---|---|---|
| Formulation 1 | 6.96 | 6.99 | 0.030 |
| Placebo | 6.93 | 6.78 | -0.16 |

The pH of those administered the medication increased to an average of 0.03 and the stool pH of subjects administered the placebo actually had a stool pH decrease by 0.16. The graphic depiction of the changes can be seen in Figure 16. The pH changes over the trial were increased in subjects who received enzyme replacement and subjects receiving the placebo lost pH change.

pH Levels at week 12 correlations with calcium, copper and vitamin C are provided in the following table. There were positive robust significant correlations between subjects administered Formulation 1 and calcium, copper and vitamin C intakes at week 12.

| | **Formulation 1 Correlation** |
|---|---|
| Calcium at Week 12 | 0.43 |
| Copper at Week 12 | 0.24 |
| Vitamin C at Week 12 | 0.36 |

### STUDY 3A

The same cohort of subjects as outlined in study 1A was used in Study 3A. The subjects were screened for autism. The medication or placebo was administered three times a day with food. The medication is described herein as a high protease enzyme composition (Formulation 1).

One hundred eight two (182) subjects were randomized. The clinical trial is an interventional study with parallel assignment that was randomized and blinded. The endpoint classification assessed during the trial was undertaken to determine treatment efficacy of subjects with the high protease enzyme composition described herein (Formulation 1) versus subjects administered the placebo.

Primary outcomes assessed were the heretofore mentioned subscales of the Aberrant Behavior Checklist. The total ABC was also assessed. Assessments were taken at time points of 12, 24, and 48 weeks. Weeks 0 to 12 were the medication versus the placebo, and weeks 24 and 48 were changes from baseline where all subjects are administered the medication.

The outcome of this study was the improvement from baseline on the 5 subscales of the ABC Aberrant Behavior Checklist observed in subjects treated with Formulation 1 compared to the placebo.

| | Formulation 1: Improvement in: | | |
|---|---|---|---|
| | 12 week (placebo adjusted) | 24 weeks | 48 weeks |
| Irritability | 9 | 27 | 34 |
| Lethargy | 11 | 36 | 48 |
| Hyperactivity | 11 | 25 | 27 |
| Stereotypy | 11 | 31 | 38 |
| Speech | 10 | 23 | 29 |
| Total ABC | 11 | 28 | 35 |

The table demonstrates the changes in total ABC score from baseline at 12, 24, and 48 weeks during the study.

Each time point is characterized by the percentage change from baseline. At the week 12 time point the percentage change is demonstrated as a placebo adjusted outcome, whereby the change on placebo is subtracted by the change on the assessment in subjects administered the medication.

For those skilled in the art, placebo adjusted changes from baseline are standard ways to assess change between the two groups: medication versus placebo. When there is a positive change, (sign is positive) the medication had a greater affect on the outcome change than the placebo. Should the change be negative the outcome is better in the placebo.

After 12 weeks, it is apparent that subjects administered the medication improve compared to those administered the placebo.

Improvement appears to continue through week 48. In subjects whose symptoms are never thought to change over the course of their lifetime, the ability to see this magnitude in change could not have been anticipated.

Of note, the subscales of social withdrawal/lethargy, and hyperactivity/non-compliance are known as reciprocal scales. When hyperactivity increases, social withdrawal and lethargy are decreased. The opposite is true as well. The fact that both subscales decreased is significant as the changes demonstrate that there is a non-sedating effect of the medication, and a non-sedating approach to the problem.

Further of note is the fact that all subscales have a large improvement over 12, 24 and 48 weeks. This is an unexpected improvement both in the fact that it is all subscales and also the fact that the total ABC demonstrates improvement.

### STUDY 4A

The PPVT and the EVT were administered to the subjects in the study. In the same cohort of subjects as outlined in study 1A.

The subjects were screened for autism. The medication or placebo was administered three times a day with food. The medication is described herein as a high protease enzyme (Formulation 1).

One hundred eighty two (182) subjects were randomized. The clinical trial is an interventional study with parallel assignment that was randomized and blinded. The endpoint classification assessed during the trial was undertaken to determine treatment efficacy of the subjects with the high protease enzyme composition described herein (Formulation 1), versus subjects administered the placebo.

In normally developing subjects the EVT and the PPVT should demonstrate similar growth. The growth scales seen over a 12 month period should keep pace with one another. In the case of neurologically or otherwise impaired subjects the scales will often not keep pace with one another.

Further the amount of growth (growth scores) demonstrated over a 12 month period should range from 8-10 points on both the EVT and the PPVT, depending upon age. In the ages 3-8 years range the growth score should be approximately 8.

| | PPVT Mean Changes | | |
|---|---|---|---|
| Group | Ceiling | Errors | Growth Score |
| Formulation 1 | 3.88 | -1.69 | 4.31 |
| Placebo | 5.60 | 1.83 | 3.20 |

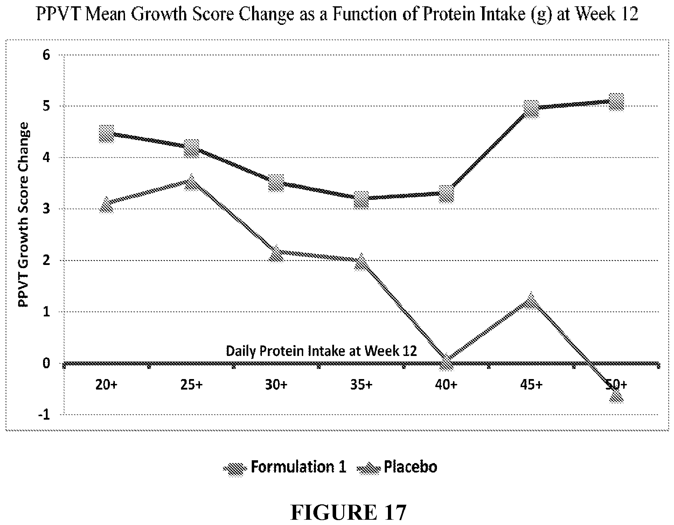

In these PPVT results the growth scores for subjects on the medication exceeded subjects on the placebo. Growth scores are adjusted for age and for standardization of scores across the entire study (See Figure 17).

Additionally, subjects on the medication made fewer errors; whereas subjects on the placebo made significantly more errors. This increase in error rate may signify an increase in hyperactivity or an increase in inattention, compared to subjects on the composition of Formulation 1.

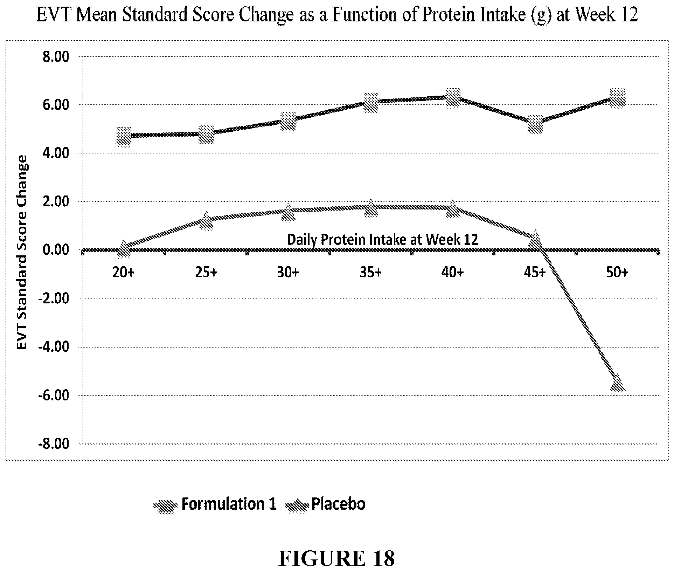

The EVT was administered to the same group of subjects as the PPVT. The EVT scores demonstrate a large difference between subjects administered the medication and subjects administered the placebo (See Figure 18).

There was also a reduction in the number of errors in subjects administered the medication compared to subjects administered the placebo.

The growth scores demonstrate a vast improvement in subjects administered the medication versus subjects administered the placebo.

Of note the growth scores seen in subjects administered the medication in 12 weeks are more than half of what would be expected in a normally developing subjects in 52 weeks. This increase in expressive language is a profound change in a subject with autism.

Also of note is the fact that there is a larger gain in growth scores in the EVT seen in subjects administered the medication versus subjects administered the medication in the PPVT. This is not generally seen in normally developing subjects.

| **EVT Mean Changes** | | | |
|---|---|---|---|
| **Group** | **Ceiling** | **Errors** | **Growth Score** |
| Formulation 1 | 6.73 | -0.24 | 5.84 |
| Placebo | 6.59 | +0.22 | 1.73 |

### STUDY 6A

The Block food screener was administered to subjects in the study. In the same cohort of subjects as outlined in study 1A were assessed in the present study. Subjects were screened for autism. The medication or placebo was administered three times a day with food. The medication is described herein as a high protease enzyme composition (Formulation 1).

One hundred eighty-two (182) subjects were randomized. The clinical trial is an interventional study with parallel assignment that was randomized and blinded. The endpoint classification assessed during the trial was undertaken to determine treatment efficacy of subjects with the high protease enzyme composition described herein (Formulation 1), versus subjects administered the placebo.

The food screener was administered at baseline, weeks 2, 4, 8, and 12.

The following are various correlations seen between the food intake and changes seen on the various scales.

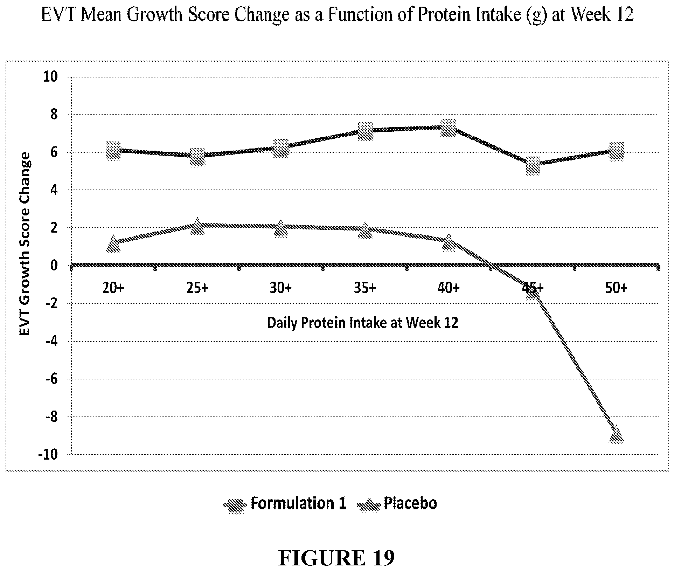

Figure 19 illustrates EVT mean standard score change as a function of protein intake (g) at week 12.

Secondary outcome measures to be assessed included other key measures of behavior and quality of life associated with autism at baseline, 4, 8, 12, 16, 20, 24, 36, 48, 60, 72, 84, 96, 108, 120, 132, 144, 156, 168, and 180 weeks of treatment.

ABC inappropriate speech percentage changes by week are provided in the following table:

| **Week** | **Formulation 1** | **Placebo** |
|---|---|---|
| 2 | -6% | -8% |
| 4 | -11% | -7% |
| 8 | -10% | -13% |
| 12 | -13% | -3% |

ABC subscale change as a function of subjects eating 50g+ daily protein at week 12 is provided in the following table:

| **ABC Subscale** | **Formulation 1** | **Placebo** | **p Value** |
|---|---|---|---|
| Irritability | -3.38 | 1.61 | .0381 |
| Lethargy | -5.70 | -1.44 | .0866 |
| Hyperactivity | -5.95 | -0.90 | .0392 |
| Stereotypy | -2.41 | 1.17 | .0055 |
| Total ABC | -18.11 | -0.12 | .0078 |

Conners Subscales Carbohydrate Change (g) >0 at week 12:

| | **Formulation 1** | **Placebo** |
|---|---|---|
| Hyperactivity | -3.07 | -1.30 |
| Conduct Disorder | -1.77 | -0.23 |
| Oppositional Defiant | -1.29 | -0.12 |
| Global Impression | -2.05 | -1.24 |
| Inattention | -3.55 | -1.74 |
| Hyperactivity | -5.27 | -1.99 |
| Learning Problems | -2.69 | -0.32 |
| Aggression | -2.19 | -0.47 |

When there is an increase in the amount of carbohydrates ingested >0g, there is a greater improvement in subjects administered the drug over subjects administered the placebo.

The following observations were made over the course of the trials:

Fecal Chymotrypsin Change Correlated with changes in vitamin K and lutein.

| | **Formulation 1 Correlation** |
|---|---|
| Vitamin K Change | 0.35 |
| Lutein Change | 0.39 |

Peabody Picture Vocabulary Test (PPVT)

| | PPVT Mean Changes | | |
|---|---|---|---|
| **Group** | **Ceiling** | **Errors** | **Growth Score** |
| Formulation 1 | 3.88 | -1.69 | 4.31 |
| Placebo | 5.60 | 1.83 | 3.20 |

Expressive Vocabulary Test (EVT):

| | EVT Mean Changes | | | |
|---|---|---|---|---|
| **Group** | **Ceiling** | **Errors** | **Standard Score** | **Growth Score** |
| Formulation 1 | 6.73 | -0.24 | 4.71 | 5.84 |
| Placebo | 6.59 | +0.22 | 0.62 | 1.73 |

While preferred embodiments have been shown and described herein, it will be obvious to those skilled in the art that such embodiments are provided by way of example only. Numerous variations, changes, and substitutions will now occur to those skilled in the art.

## Claims

1. A pharmaceutical composition comprising digestive enzymes and one or more excipients, for use in the treatment of vitamin K deficiency or bone fragility in a subject exhibiting Autism Spectrum Disorder (ASD), wherein the digestive enzymes comprise a protease, an amylase, and a lipase.

2. The pharmaceutical composition for use according to claim 1, wherein the subject having an ASD has malnutrition as a result of dietary restriction self-imposed by the subject.

3. The pharmaceutical composition for use of claim 1 or 2, wherein the composition is administered to the subject two or more times a day with food for at least 12 weeks.

4. The pharmaceutical composition for use according to any preceding claim, wherein the subject has an abnormal fecal chymotrypsin level before administration of the pharmaceutical composition.

5. The pharmaceutical composition for use according to claim 4, wherein the subject has a fecal chymotrypsin level of less than 13 U/g or less than 12.6 U/g as measured in a frozen stool sample before administration of the pharmaceutical composition; or wherein the subject has a fecal chymotrypsin level of less than 10 U/g or less than 9 U/g as measured in a fresh stool sample before administration of the pharmaceutical composition.

6. The pharmaceutical composition for use according to claim 4, wherein the subject has increased fecal chymotrypsin levels after administration of the pharmaceutical composition.

7. The pharmaceutical composition for use according to any preceding claim, wherein the subject exhibits a decrease in the number or severity of infections or a decrease in the number or severity of allergic incidents after administration of the pharmaceutical composition in comparison to before the subject was administered the pharmaceutical composition.

8. The pharmaceutical composition for use according to any preceding claim, wherein the ASD is autism.

9. The pharmaceutical composition for use according to any preceding claim, wherein the subject has increases in calcium after administration or after treatment with the pharmaceutical composition.

## Patentansprüche

1. Pharmazeutische Zusammensetzung, die Verdauungsenzyme und einen oder mehrere Hilfsstoffe umfasst, zur Verwendung bei der Behandlung von Vitamin-K-Mangel oder Knochenbrüchigkeit bei einem Subjekt, das Autismusspektrumsstörung (ASD) zeigt, wobei die Verdauungsenzyme eine Protease, eine Amylase und eine Lipase umfassen.

2. Pharmazeutische Zusammensetzung zur Verwendung nach Anspruch 1, wobei das Subjekt mit einer ASD Unterernährung als Ergebnis von Ernährungseinschränkung aufweist, die durch das Subjekt selbst auferlegt ist.

3. Pharmazeutische Zusammensetzung zur Verwendung nach Anspruch 1 oder 2, wobei die Zusammensetzung dem Subjekt zwei- oder mehrmals am Tag mit Nahrung für zumindest 12 Wochen verabreicht wird.

4. Pharmazeutische Zusammensetzung zur Verwendung nach einem vorhergehenden Anspruch, wobei das Subjekt einen abnormalen fäkalen Chymotrypsinspiegel vor Verabreichung der pharmazeutischen Zusammensetzung aufweist.

5. Pharmazeutische Zusammensetzung zur Verwendung nach Anspruch 4, wobei das Subjekt einen fäkalen Chymotrypsinspiegel von weniger als 13 U/g oder weniger als 12,6 U/g wie gemessen in einer gefrorenen Stuhlprobe vor Verabreichung der pharmazeutischen Zusammensetzung aufweist; oder wobei das Subjekt einen fäkalen Chymotrypsinspiegel von weniger als 10 U/g oder weniger als 9 U/g wie gemessen in einer frischen Stuhlprobe vor Verabreichung der pharmazeutischen Zusammensetzung aufweist.

6. Pharmazeutische Zusammensetzung zur Verwendung nach Anspruch 4, wobei das Subjekt erhöhte fäkale Chymotrypsinspiegel nach Verabreichung der pharmazeutischen Zusammensetzung aufweist.

7. Pharmazeutische Zusammensetzung zur Verwendung nach einem vorhergehenden Anspruch, wobei das Subjekt eine Verringerung der Anzahl oder Schwere von Infektionen oder eine Verringerung der Anzahl oder Schwere von allergischen Vorfällen nach Verabreichung der pharmazeutischen Zusammensetzung im Vergleich zu der Zeit zeigt, bevor dem Subjekt die pharmazeutische Zusammensetzung verabreicht wurde.

8. Pharmazeutische Zusammensetzung zur Verwendung nach einem vorhergehenden Anspruch, wobei die ASD Autismus ist.

9. Pharmazeutische Zusammensetzung zur Verwendung nach einem vorhergehenden Anspruch, wobei das Subjekt Erhöhungen an Calcium nach Verabreichung oder nach Behandlung mit der pharmazeutischen Zusammensetzung aufweist.

## Revendications

1. Composition pharmaceutique comprenant des enzymes digestives et un ou plusieurs excipients, destinée à être utilisée dans le traitement d'une carence en vitamine K ou d'une fragilité osseuse chez un sujet présentant un trouble du spectre autistique (TSA), lesdites enzymes digestives comprenant une protéase, une amylase et une lipase.

2. Composition pharmaceutique destinée à être utilisée selon la revendication 1, ledit sujet atteint d'un TSA souffrant de malnutrition en raison d'une restriction alimentaire auto-imposée par le sujet.

3. Composition pharmaceutique destinée à être utilisée selon la revendication 1 ou 2, ladite composition étant administrée au sujet deux fois par jour ou plus avec de la nourriture pendant au moins 12 semaines.

4. Composition pharmaceutique destinée à être utilisée selon l'une quelconque des revendications précédentes, ledit sujet présentant un taux de chymotrypsine fécale anormal avant l'administration de la composition pharmaceutique.

5. Composition pharmaceutique destinée à être utilisée selon la revendication 4, ledit sujet présentant un taux de chymotrypsine fécale inférieur à 13 U/g ou inférieur à 12,6 U/g tel que mesuré dans un échantillon de selles congelées avant l'administration de la composition pharmaceutique ; ou ledit sujet présentant un taux de chymotrypsine fécale inférieur à 10 U/g ou inférieur à 9 U/g tel que mesuré dans un échantillon de selles fraîches avant l'administration de la composition pharmaceutique.

6. Composition pharmaceutique destinée à être utilisée selon la revendication 4, ledit sujet présentant des taux de chymotrypsine fécale accrus après l'administration de la composition pharmaceutique.

7. Composition pharmaceutique destinée à être utilisée selon l'une quelconque des revendications précédentes, ledit sujet présentant une diminution du nombre ou de la gravité des infections ou une diminution du nombre ou de la gravité des incidents allergiques après l'administration de la composition pharmaceutique par rapport à avant que le sujet ne reçoive la composition pharmaceutique.

8. Composition pharmaceutique destinée à être utilisée selon l'une quelconque des revendications précédentes, ledit TSA étant l'autisme.

9. Composition pharmaceutique destinée à être utilisée selon l'une quelconque des revendications précédentes, ledit sujet présentant des augmentations de calcium après l'administration ou après le traitement avec la composition pharmaceutique.
